# EUROPEAN PATENT SPECIFICATION

(11) **EP 2 941 270 B1**
(45) Date of publication and mention of the grant of the patent: **10.06.2020**
(21) Application number: 14735320.5
(22) Date of filing: 02.01.2014
(51) Int. Cl.: A61K 39/395, A61K 48/00, A61P 29/00, A61K 31/7088, A61K 38/02, A61K 31/7105, C07K 16/22, C07K 16/28, C07K 14/475

(54) **METHODS FOR TREATING INFLAMMATION**
VERFAHREN ZUR BEHANDLUNG VON ENTZÜNDUNGEN
PROCÉDÉS POUR TRAITER L'INFLAMMATION

(30) Priority: 03.01.2013 US 201361748491 P
(43) Date of publication of application: 11.11.2015
(73) Proprietor: New York University, New York, NY 10016-5802 (US)
(72) Inventor: MOORE, Kathryn, J., Westfield, NJ 07090 (US); FISHER, Edward, A., Scarsdale, NY 10583 (US)
(74) Representative: Carpmaels & Ransford LLP
(86) International application number: PCT/US2014/010026
(87) International publication number: WO 2014/107484

(56) References cited:
- EP-A1- 2 385 121
- WO-A1-2014/063134
- WO-A2-02/24142
- WO-A2-2004/006898
- CA-A1- 2 489 780
- JP-A- 2010 111 631
- US-A- 6 017 714
- US-A1- 2003 232 419
- US-A1- 2006 019 896
- US-A1- 2011 263 483
- US-A1- 2012 020 929
- A. WANSCHEL ET AL: "Neuroimmune Guidance Cue Semaphorin 3E Is Expressed in Atherosclerotic Plaques and Regulates Macrophage Retention", ARTERIOSCLEROSIS, THROMBOSIS, AND VASCULAR BIOLOGY., vol. 33, no. 5, 21 February 2013 (2013-02-21), pages 886-893, XP055274032, US ISSN: 1079-5642, DOI: 10.1161/ATVBAHA.112.300941
- Anonymous: "Abstract 49: The Neuroimmune Guidance Molecule Semaphorin 3e Is Expressed in Macrophages of Advanced Atherosclerotic Plaques and Induces Macrophage Chemostasis", , 2012, XP055274030, Retrieved from the Internet: URL:http://atvb.ahajournals.org/content/32 /Suppl_1/A49.abstract [retrieved on 2016-05-20]
- SHIMIZU IPPEI ET AL: "Semaphorin-induced Inflammation Contributes to Insulin Resistance in Dietary Obesity", CIRCULATION, vol. 126, no. 21, Suppl. S, November 2012 (2012-11), page 9961, XP009190135, & AMERICAN-HEART-ASSOCIATION RESUSCITATION SCIENCE SYMPOSIUM; LOS ANGELES, CA, USA; NOVEMBER 03 -04, 2012 ISSN: 0009-7322
- IPPEI SHIMIZU ET AL: "Semaphorin3E-Induced Inflammation Contributes to Insulin Resistance in Dietary Obesity", CELL METABOLISM, vol. 18, no. 4, 1 October 2013 (2013-10-01), pages 491-504, XP55274253, United States ISSN: 1550-4131, DOI: 10.1016/j.cmet.2013.09.001
- ANN MARIE SCHMIDT ET AL: "The Semaphorin 3E/PlexinD1 Axis Regulates Macrophage Inflammation in Obesity", CELL METABOLISM, vol. 18, no. 4, 1 October 2013 (2013-10-01), pages 461-462, XP55274248, United States ISSN: 1550-4131, DOI: 10.1016/j.cmet.2013.09.011
- KATHRYN J MOORE ET AL: "Macrophages, atherosclerosis and the potential of netrin-1 as a novel target for future therapeutic intervention", FUTURE CARDIOLOGY, vol. 8, no. 3, 1 May 2012 (2012-05-01), pages 349-352, XP55304730, GB ISSN: 1479-6678, DOI: 10.2217/fca.12.30
- VAN GILS, J. M ET AL.: 'The neuroimmune guidance cue netrin-1 promotes atherosclerosis by inhibiting the emigration of macrophages from plaques' NATURE IMMUNOLOGY vol. 13, no. 2, 2012, pages 136 - 143, XP055251357

## Description

### FIELD OF THE INVENTION

The present invention relates to methods and compositions for treating and inhibiting inflammation and inflammatory diseases, such as, for instance chronic inflammation.

### BACKGROUND OF THE INVENTION

A combination of genetic and biochemical methods has led to the discovery of several important classes of axon guidance molecules and their receptors. Netrins are secreted molecules that can act to attract or repel axons by binding to their receptors, DCC and UNC5. Slits also known as Sli are secreted proteins that normally repel growth cones by engaging Robo (Roundabout) class receptors. Ephrins are cell surface molecules that activate Eph receptors on the surface of other cells. This interaction can be attractive or repulsive. In some cases, Ephrins can also act as receptors by transducing a signal into the expressing cell, while Ephs act as the ligands. Signaling into both the Ephrin- and Eph-bearing cells is called "bi-directional signaling." Semaphorins occur as many types and are primarily axonal repellents, and activate complexes of cell-surface receptors called Plexins and Neuropilins. Cell adhesion molecules (CAMs) are integral membrane proteins mediating adhesion between growing axons and eliciting intracellular signalling within the growth cone. CAMs are the major class of proteins mediating correct axonal navigation of axons growing on axons (fasciculation). There are two CAM subgroups: IgSF-CAMs (belonging to the immunoglobulin superfamily) and Cadherins (Ca-dependent CAMs).

In addition, many other classes of extracellular molecules are used by growth cones to navigate properly including developmental morphogens, such as BMPs, Wnts, Hedgehog, and FGFs, extracellular matrix and adhesion molecules such as laminin, tenascins, proteoglycans, N-CAM, and LI, growth factors like NGF, and neurotransmitters and modulators like GABA.

Growing axons rely on variety of guidance cues in deciding upon a growth pathway. The growth cones of extending axons process these cues in an intricate system of signal interpretation and integration, in order to insure appropriate guidance. Adhesive cues provide physical interaction with the substrate necessary for axon protrusion. These cues can be expressed on glial and neuronal cells the growing axon contacts or be part of the extracellular matrix. Examples are laminin or fibronectin, in the extracellular matrix, and cadherins or Ig-family cell-adhesion molecules, found on cell surfaces. Tropic cues act as attractants or repellents and cause changes in growth cone motility by acting on the cytoskeleton through intracellular signaling. For example, Netrin plays a role in guiding axons through the midline, acting as both an attractant and a repellent. While Semaphorin3A, helps axons grow from the olfactory epithelium to map different locations in the olfactory bulb. Modulatory cues influence the sensitivity of growth cones to certain guidance cues. For instance, neurotrophins can make axons less sensitive to the repellent action of Semaphorin3A.

Given the abundance of these different guidance cues it was previously believed that growth cones integrate various information by simply summing the gradient of cues, in different valences, at a given point in time, to making a decision on the direction of growth. However, studies in vertebrate nervous systems of ventral midline crossing axons, has shown that modulatory cues play a crucial part in tuning axon responses to other cues, suggesting that the process of axon guidance is nonlinear. For examples, commisurial axons are attracted by netrin and repelled by slit. However, as axons approach the midline, the repellent action of Slit is suppressed by Robo-3/Rig-1 receptor. Once the axons cross the midline, activation of Robo by Slit silences Netrin-mediated attraction, and the axons are repelled by Slit.

The netrin family is composed mostly of secreted proteins which serve as bifunctional signals: attracting some neurons while repelling others during the development of brain. Expressed in the midline of all animals possessing bilateral symmetry, they can act as long or short range signals during neurogenesis. In order to carry out their functions, netrins interact with specific receptors: DCC or UNC-5 depending on whether they are trying to attract or repel neurons respectively. There is a high degree of conservation in the secondary structure of netrins, which has several domains which are homologous with laminin at the amino terminal end. The C-terminal domain is where most of the variation is found between species and contains different amino acids which allow interaction with specific proteins in extracellular matrix or on cell surface. The differences in terms of structure and function have led to the identifications of several different types of netrins including netrin-1, netrin-3, and netrins-G.

Netrin-1 is found in the floor plate and neuroepithelial cells of the ventral region of the spinal cord, as well as other locations in the nervous system including the somatic mesoderm, pancreas and cardiac muscle. Its main role is in axonal guidance, neuronal migration and morphogenesis of different branching structures. Mice with mutations in the netrin-1 gene were observed to be lacking in forebrain and spinal cord commissural axons. Netrin-3 is different from other netrins. While expressed during development of the peripheral nervous system in the motor, sensory and sympathetic neurons, it is very limited in the central nervous system. Studies with netrin-3 have noticed a reduced ability to bind with DCC receptors when compared with netrin-1. This suggests that it mainly operates through other receptors. Netrins-G are secreted but remain bound to the extracellular surface of the cell membrane through Glycophosphatidylinositol (GPI). They are expressed predominantly in the central nervous system in places such as the thalamus and mitral cells of the olfactory bulb. (Park, et al., J Clin Invest, 2009, 119: 136-145) They do not bind to DCC or UNC-5 and instead bind to ligand NGL-1, which results in an intracellular transduction cascade. The two versions, netrins-G1 and netrins-G2, are found only in vertebrates. It is believed that they evolved independently of other netrins in order to facilitate the construction of the brain.

DCC and UNC-5 proteins carefully mediate netrin-1 responses. The UNC-5 protein is mainly involved in signaling repulsion. DCC, which is implicated in attraction, can also serve as a co-factor in repulsion signaling when far away from the source of netrin-1. DCC is highly expressed in the central nervous system and associated with the basal lamina of epithelia cells. In the absence of netrin-1, these receptors are known to induce apoptosis.

Semaphorins are a class of secreted and membrane proteins that act as axonal growth cone guidance molecules. They primarily act as short-range inhibitory signals and signal through multimeric receptor complexes. They are usually cues to deflect axons from inappropriate regions, especially important in neural system development. The major class of proteins that act as their receptors are called plexins.Every semaphorin is characterized by the expression of a specific region of about 500 amino acids called the sema domain.

The semaphorins are grouped into eight major classes based on structure and phylogentic tree analyses. The first seven are ordered by number, from class 1 to class 7. The eighth group is class V, where V stands for virus. Classes 1 and 2 are found in invertebrates only, whilst classes 3, 4, 6, and 7 are found in vertebrates only. Class 5 is found in both vertebrates and invertebrates, and class V is specific to viruses.

Classes 1 and 6 are considered to be homologues of each other. They are each membrane bound in invertebrates and vertebrates, respectively. The same applies to classes 2 and 3; they are both secreted proteins specific to their respective taxa. Each class of semaphorin has many subgroups of different molecules that share similar characteristics. For example, Class 3 Semaphorins range from Sema3a to Sema3g.In humans, the genes arehttp://en.wikipedia.org/wiki/SEMA3A SEMA3B, SEMA3C, SEMA3D, SEMA3E, SEMA3F, SEMA3G, SEMA4A, SEMA4B, SEMA4C ("SEMAF"), SEMA4D, SEMA4F, SEMA4G, SEMA5A, SEMA5B, SEMA6A, SEMA6B, SEMA6C, SEMA6D, and SEMA7A.

Different semaphorins use different types of receptors. Most semaphorins use receptors in the group of proteins known as plexins. Class 3 semaphorins signal through heterocomplexes of neuropilins, Class A Plexins, and cell adhesion molecules, and the makeup of these complexes likely provides specificity for binding and transducing signals from different Class 3 Semaphorins. Class 7 Semaphorin are thought to use integrins as their receptors. Semaphorins and their receptors may be involved in the sorting of pools of motor neurons and the modulation of pathfinding for afferent and efferent axons from and to these pools.

Semaphorins are very versatile. Their discovery was in regards to axon guidance. They not only guide axons in development, but also have major roles in immune function (classes 4, 6, and 7) and the development of bones. One of the most versatile semaphorin classes is number 3, and specifically Sema3a. Sema3a repels axons from the dorsal root ganglia, facial nerves, vagal nerves, olfactory-sensory, cortical nerves, hippocampal nerves and cerebellar nerves.

Atherosclerosis is a disease of chronic inflammation that is distinguished by the persistence of cholesterol-engorged macrophages in arterial plaques. Arterial inflammation is initiated by the subendothelial retention of plasma low density lipoprotein (LDL), and enhanced by oxidative modification of these lipoproteins, which triggers an influx of monocytes (Moore, et al., Cell, 2011, 145: 341-355). Unlike other inflammatory states, atherosclerotic inflammation does not readily resolve and cholesterol-laden macrophages persist in the arterial wall. These macrophage "foam cells" cause expansion of the plaque though recruitment of additional leukocytes and vascular smooth muscle cells, and contribute prominently to plaque instability through the secretion of extracellular matrix-degrading proteases and cytotoxic factors. Notably, atherosclerotic plaques that cause clinical events (ie. myocardial infarction and stroke) are characterized by a high macrophage content (Libby, et al., Nat Med, 2002, 8: 1257-1262).

While macrophage retention in the artery wall has long been recognized as a fundamental step in creating the chronic inflammatory milieu underlying atherosclerosis, the mechanisms regulating this process are not well understood. Resolution of acute inflammation typically involves emigration of monocyte-derived cells out of the inflamed site through nearby lymphatic vessels (Bellingan, et al., J Immunol 1996, 157: 2577-2585). This process appears to be impaired in atherosclerosis (Randolph, Curr Opin Lipidol, 2008, 19: 462-468) and has been attributed, in part, to the cholesterol loading of macrophages which shifts these cells to a more sessile phenotype (Nagao, et al., Arterioscler Thromb Vasc Biol, 2007, 27: 1596-1602; Pagler, et al. Circ Res, 2011, 108: 194-200; Park, et al., J Clin Invest, 2009, 119: 136-145; and Qin, et al., Arterioscler Thromb Vasc Biol, 2006, 26: 372-378). Notably, reducing plasma cholesterol and/or increasing levels of high-density lipoprotein (HDL) promotes plaque regression in mouse models of atherosclerosis, in a process characterized by lipid unloading and emigration of macrophages from lesions (Rong, et al., Circulation, 2001, 104: 2447-2452; Llodra, et al., Proc Natl Acad Sci U S A, 2004, 101: 11779-11784; Feig, et al., Circulation, 2011, 123: 989-998; Feig, et al., J Clin Invest, 2010, 120: 4415-4424; and Trogan, et al., Proc Natl Acad Sci USA, 2006, 103: 3781-3786).

Recent studies in transplant-based mouse models of atherosclerosis regression have shown that reducing plasma non-HDL cholesterol and/or increasing levels of high-density lipoprotein (HDL), the "good cholesterol", promotes a process characterized by lipid unloading and emigration of macrophages from lesions to regional and systemic lymph nodes (Rong, et al., Circulation, 2001, 104: 2447-2452; Llodra, et al., Proc Natl Acad Sci USA, 2004, 101: 11779-11784; Feig, et al., Circulation, 2011, 123: 989-998; Feig, et al., J Clin Invest, 2010, 120: 4415-4424; Trogan, et al., Proc Natl Acad Sci USA, 2006, 103: 3781-3786 and Feig, et al., Proc Natl Acad Sci USA, 2011, 108: 7166-7171). In addition, macrophage expression of the chemokine receptor CCR7 was shown to be essential for decreasing the macrophage content of atherosclerotic plaques (Feig, et al., J Clin Invest, 2010, 120: 4415-4424 and Trogan, et al., Proc Natl Acad Sci USA, 2006, 103: 3781-3786), implicating the CCR7-specific ligands CCL19 and CCL21 in promoting the egress of cell from the artery wall. These studies indicate that macrophage egress from the plaque is likely to be actively inhibited during hypercholesterolemia, although the regulatory signals that impair this process remain largely unknown.

A paradigm for inhibitory guidance cues exists in the developing nervous system, where axonal migration relies on the integration of both chemorepulsive and chemoattractive signals to steer the axonal growth cone. One such guidance molecule netrin-1, mediates both chemorepulsion and chemoattraction of axons navigating the spinal cord midline. This context-dependent response to netrin-1 is regulated by differential receptor expression by the target cell (Serafini, et al., Cell, 1994, 78: 409-424 and Kennedy, et al., Cell, 1994, 78: 425-435). For example, neurons expressing the Deleted in Colon Cancer (DCC) receptor or Neogenin are attracted by a diffusible gradient of netrin-1 secreted at the midline (Hong, et al., Cell, 1999, 97: 927-941 and Tessier-Lavigne, et al., Science, 1996, 274: 1123-1133). Conversely, co-expression of the UNC5b receptor with DCC converts netrin-1 attraction to repulsion, whereas expression of UNC5b alone mediates short-range repulsion (Cirulli, et al., Nat Rev Mal Cell Biol, 2997, 8: 296-306 and Keleman, et al., Neuron, 2001, 32: 605-617). A number of recent studies have uncovered instructional roles for netrin-1 and its receptors outside the nervous system in organogenesis (Srinivasan, et al., Dev Cell, 2003, 4: 371-382 and Salminen, et al., Development, 2007, 127: 13-22, angiogenesis (Nguyen, et al., Proc Natl Acad Sci USA, 2006, 103: 6530-6535 and Wilson, et al. Science, 2006, 313: 640-644) and tumorigenesis (Arakawa, Nat Rev Cancer, 2004, 4: 978-987 and Fitamant, et al., Proc Natl Acad Sci USA, 2008, 105: 4850-4855), suggesting that netrin-1 regulates cell migration in a broader context.

Netrin-1 expression is modulated during acute inflammation due to *Staphylococcus aureus* infection (Ly, et al., Proc Natl Acad Sci USA, 2005, 102: 14729-14734). These studies established that netrin-1 inhibited migration of monocytes, neutrophils, and lymphocytes, via its receptor UNC5b. Recent studies in models of hypoxia and reperfusion injury have extended these findings to show that expression of netrin-1 by epithelial cells also attenuates leukocyte accumulation (Ly, et al., Proc Natl Acad Sci USA, 2005, 102: 14729-14734, Mirakaj, et al., Am J Respir Crit Care Med, 2010, 181: 815-824; Rosenberger, et al., Nat Immunol, 2009, 10: 195-202 and Wang, et al., Am J Physiol Renal Physiol, 2008, 294: F739-747).
Kathryn J Moore et al., Future Cardiology, 2012, 8(3): 349-352 relates to the local delivery of systems to block the actions of netrin-1 or Unc5B in plaques.
US 2006/019896 A1 describes methods and compositions for modulating proliferation, differentiation, migration and adhesion of cardiovascular cell types.
CA 2489780 A1 describes compositions and methods for modulating angiogenesis mediated by netrin-1 or the netrin-1 receptor.

### SUMMARY OF THE INVENTION

The invention provides a pharmaceutical composition comprising an antagonist anti-netrin-1 antibody or an antagonist anti-unc5b antibody effective to inhibit or reduce the biological activity of netrin-1 or its receptor unc5b, for use in a method for inhibiting, slowing or preventing atherosclerosis characterized by inflammation or for use in a method for treating a disease characterised by inflammation selected from the group consisting of atherosclerosis and obesity, wherein a therapeutically effective amount of the agent is to be administered.

The invention relates to the application and use of modulators of axonal guidance, including antagonists or inhibitors of axonal guidance proteins, to inhibit inflammation and treat inflammatory diseases such as, for instance, chronic inflammation including atherosclerosis, arthritis, diabetes and tuberculosis.

In a first instance, the disclosure relates to a method for inhibiting, reducing or slowing inflammation or inflammatory diseases by inhibiting, inhibiting the biological activity of or antagonizing an axonal guidance protein. The method may feature administering to a subject a therapeutically effective amount of an agent effective to inhibit or reduce the biological activity of an axonal guidance protein or a receptor of the axonal guidance protein, or an analog, derivative or combination thereof. The inflammatory disease may be, for instance, atherosclerosis, rheumatoid arthritis, tuberculosis, diabetes, autoimmune syndromes, or obesity where macrophage accumulation in adipose tissue is known to promote insulin resistance.

In a second instance, the disclosure relates to a method for inhibiting or reducing sequestration or collecting or localization of macrophages by inhibiting, inhibiting the biological activity of or antagonizing an axonal guidance protein. The sequestration or collecting or localization of macrophages may be in, for instance, an atherosclerotic plaque or adipose tissue. The method may feature administering to a subject a therapeutically effective amount of an agent effective to inhibit or reduce the biological activity of an axonal guidance protein or a receptor of the axonal guidance protein, or an analog, derivative or combination thereof.

In a third instance, the disclosure relates to a method for treating a disease caused all or in part by or characterized by inflammation such as, for instance, chronic inflammation. The method features inhibiting, inhibiting the biological activity of or antagonizing an axonal guidance protein. The method may feature administering to a subject a therapeutically effective amount of an agent effective to inhibit or reduce the biological activity of an axonal guidance protein or a receptor of the axonal guidance protein, or an analog, derivative or combination thereof.

The disease may be, for instance, one of atherosclerosis, rheumatoid arthritis, tuberculosis, diabetes, autoimmune syndromes, and obesity where macrophage accumulation in adipose tissue is known to promote insulin resistance.

In a fourth instance, the disclosure relates to a method for inhibiting, slowing, reversing or preventing atherosclerosis by inhibiting, inhibiting the biological activity of or antagonizing an axonal guidance protein. The method may feature administering to a subject a therapeutically effective amount of an agent effective to inhibit or reduce the biological activity of an axonal guidance protein or a receptor of the axonal guidance protein, or an analog, derivative or combination thereof. The method may feature reducing, inhibiting or reversing inflammation, reducing, inhibiting or reversing accumulation of macrophages, such as, for instance foam cells, reducing, inhibiting or reversing infiltration of smooth muscle cells, or reducing, inhibiting or reversing necrosis, in an atherosclerotic plaque.

In a fifth instance, the disclosure relates to a method for increasing insulin sensitivity, decreasing or inhibiting resistance to insulin, or treating diabetes, by inhibiting, inhibiting the biological activity of or antagonizing an axonal guidance protein. The method may feature administering to a subject a therapeutically effective amount of an agent effective to inhibit or reduce the biological activity of an axonal guidance protein or a receptor of the axonal guidance protein, or an analog, derivative or combination thereof.

In a sixth instance, the disclosure relates to a method for screening for or identifying an agent useful for any one or more of a) inhibiting, reducing or slowing inflammation or inflammatory diseases, b) inhibiting or reducing sequestration or collecting or localization of macrophages, c) treating a disease caused all or in part by or characterized by inflammation such as, for instance, chronic inflammation, including, for instance, atherosclerosis, rheumatoid arthritis, tuberculosis, diabetes, autoimmune syndromes, and obesity, d) inhibiting, slowing, reversing or preventing atherosclerosis, or e) increasing insulin sensitivity, decreasing or inhibiting resistance to insulin, or treating diabetes. The method for screening for or identifying an agent useful for any one or more of these purposes a) through e) features identifying an agent capable of or useful for inhibiting, inhibiting the biological activity of or antagonizing an axonal guidance protein.

For each of these above instances, the axonal guidance protein may be, for instance, one of sema3E, plexin, and netrin-1. Also, for each of these above aspects the agent effective to inhibit or reduce the biological activity of an axonal guidance protein or a receptor of the axonal guidance protein, such as for instance netrin-1 or its receptor unc5b or semaphorin3E or its receptor plexinD1, may be, for instance, an antibody, a peptide, a nucleic acid, for instance, an interfering RNA such as siRNA, or a small molecule.

In some instances the agent effective to inhibit or reduce the biological activity of an axonal guidance protein or a receptor of the axonal guidance protein, or an analog, derivative or combination thereof, may be administered in combination with one or more drugs useful in inhibiting inflammation. Such other compounds may be, for instance, anti-inflammatory compounds, bisphosphonates or growth factors. The methods may be practiced *in vivo* or *in vitro.* In some instances the agent effective to inhibit or reduce the biological activity of an axonal guidance protein or a receptor of the axonal guidance protein, or an analog, derivative or combination thereof, may effectively inhibit or reduce the biological activity by 10%, 20%, 30%, 40%, 50%, 60%, 70%, 80%, 90%, or 95% or more relative to the activity of the protein or the amount of the protein expressed or present in a cell or tissue prior to the time the agent is administered.

The present invention relates to a pharmaceutical composition comprising an agent effective to inhibit or reduce the biological activity of an axonal guidance protein or a receptor of the axonal guidance protein, or an analog, derivative or combination thereof alone or in combination with one or more second compounds or agents effective for inhibiting inflammation.

The agent effective to inhibit or reduce the biological activity of an axonal guidance protein or a receptor of the axonal guidance protein and the one or more second compounds or agents may be formulated and administered alone or together. The pharmaceutical composition(s) comprising the agent effective to inhibit or reduce the biological activity of an axonal guidance protein or a receptor of the axonal guidance protein and the one or more second compounds or agents may be administered concurrently or sequentially.

The pharmaceutical compositions may be delivered topically, orally or parenterally. They may be delivered via the intravenous route, the intramuscular route, or the subcutaneous route. They may be delivered as an immediate release formulation or as a slow or sustained release formulation. Likewise, they may be delivered as suppositories, enemas or via aerosol.

In some instances, the pharmaceutical composition comprising an agent effective to inhibit or reduce the biological activity of an axonal guidance protein or a receptor of the axonal guidance protein may also contain one or more drugs such as anti-inflammatory agents. Also, the agent effective to inhibit or reduce the biological activity of an axonal guidance protein or a receptor of the axonal guidance protein, such as for instance netrin-1 or its receptor unc5b may be, for instance, an antibody, a peptide, a nucleic acid, for instance, an interfering RNA such as siRNA, or a small molecule.

Other objects and advantages will become apparent to those skilled in the art from a review of the following description which proceeds with reference to the following illustrative figures.

### BRIEF DESCRIPTION OF THE FIGURES

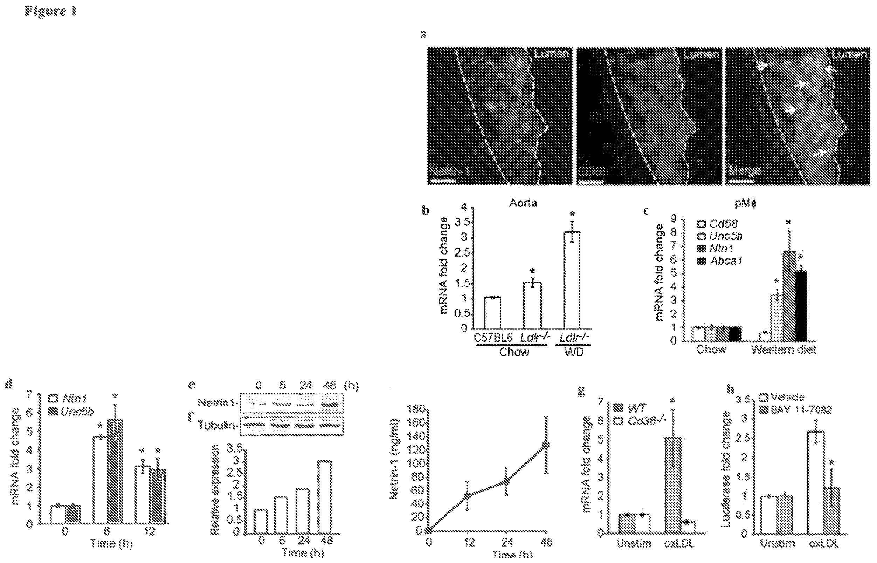

Figure 1 Abundant expression of netrin-land UNC5b by macrophage foam cells in atherosclerotic lesions. (a) Immunofluorescence staining of netrin-1 (green) and CD68 (red) and their colocalization (yellow; arrows) in aortic sinus atherosclerotic plaques of an *Ldlr -*/- mouse fed a Western diet. Dashed lines indicate lesion borders. Scale bars, 50 µm. (b) Quantitative PCR analysis *of Ntnl* mRNA isolated from the aortic arches *Ldlr -*/- mice fed a chow or Western diet (WD); results are presented relative to those of chow-fed *Ldlr* + / + (C57BL / 6) mice, set as 1. (c) Quantitative PCR analysis of mRNA for *Cd68* , *Unc5b*, *Ntnl* and *Abca1* (control gene) in peritoneal macrophages (pM Φ) isolated from *Ldlr* - / - mice fed a Western diet; results are presented relative to those of chow-fed *Ldlr -* / - mice, set as 1. (d) Quantitative PCR analysis *of Ntnl* mRNA and *Unc5b* mRNA in peritoneal macrophages treated with oxLDL (50 µg / ml), presented relative to mRNA amounts in untreated cells, set as 1. (e, f) Immunoblot analysis of netrin-1 in lysates (e) and enzyme-linked immunosorbent assay of netrin-1 in conditioned medium (f) of the cells in d ; netrin-1 expression in e (bottom) is presented relative to that of tubulin (loading control). (g) Quantitative PCR analysis *of Ntnl* mRNA in wild-type (WT) and *Cd36 -*/- peritoneal macrophages stimulated for 6 h with oxLDL (50 µg / ml), presented relative to mRNA in unstimulated cells (Unstim), set as 1. (h) *NTN1* promoter -luciferase reporter activity in HEK293 human embryonic kidney cells treated with oxLDL in the presence (BAY) or absence (Veh) of the NF- □B inhibitor BAY 11-7082 (20 µM), presented relative to reporter activity in unstimulated cells, set as 1. * *P* < 0.05 (Student ' s *t* - test). Data are representative of two experiments (*n* = 4 mice; a) or are from one experiment representative of three independent experiments (b - h ; mean +/-s.d. of triplicate samples).

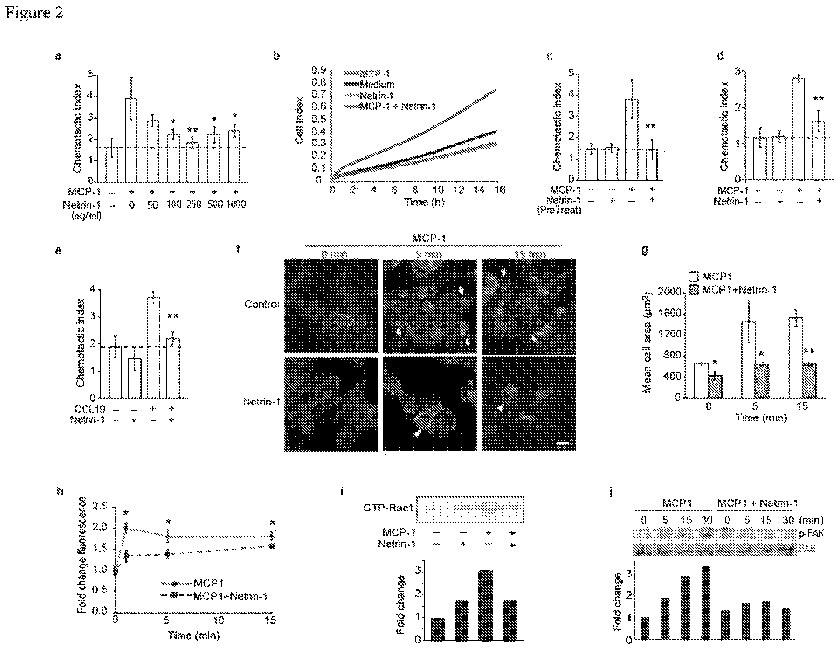

Figure 2 demonstrates that netrin-1 inhibits the migration of macrophages toward CCL2 and CCL19 via UNC5b. (a) Migration of RAW264.7 cells toward CCL2 (100 ng / ml) in the presence or absence of increasing concentrations of recombinant netrin-1 placed in the lower compartment of a Boyden chamber. (b) Migration of RAW264.7 cells toward medium or toward netrin-1 (250 ng / ml) or CCL2 (100 ng / ml) alone or together. (c) Migration of RAW264.7 cells pretreated (PT) with netrin-1 (250 ng / ml) and exposed to CCL2 (100 ng / ml). (d, e) Migration of mouse peritoneal macrophages toward CCL2 (100 ng / ml; d) or CCL19 (500 ng / ml; e) in the presence (+) or absence (-) of netrin-1 (250 ng / ml). (f) Microscopy of peritoneal macrophages treated with CCL2 (100 ng / ml) with or without (control) netrin-1 (250 ng / ml) and stained with phalloidin (to detect polymerized actin). Arrows indicate membrane ruffles in control cells and rounded morphology in netrin-1-treated cells. Scale bar, 10 µm. (g) Cell surface area of the peritoneal macrophages in f. (h) Quantification of actin polymerization by flow cytometry analysis of phallodin staining, presented relative to results at time 0, set as 1. (i) Activated (GTP-) Rac1 in peritoneal macrophages treated for 5 min with CCL2 (100 ng / ml) with or without netrin-1 (250 ng / ml), presented relative to results of untreated cells, set as 1. (j) Immunoblot analysis of phosphorylated (p-) and total FAK in peritoneal macrophages incubated with CCL2 (100 ng / ml) with or without pretreatment with netrin-1 (250 ng / ml); results are presented relative to results at time 0, set as 1. * *P* < 0.05 and * * *P* < 0.01 (Student ' s *t* -test). Data are from one experiment representative of three independent experiments (mean +/-s.d. of triplicate samples in a - e , g - j).

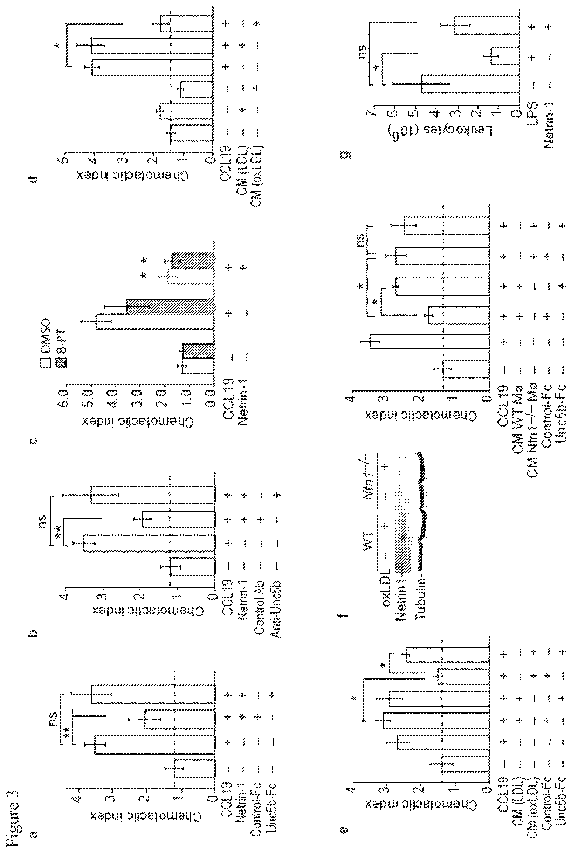

Figure 3 demonstrates that foam cell secreted netrin-1 blocks macrophage migration. (a-c) Migration of pMø to CCL19 (500 ng/ml) with/without netrin-1 (250 ng/ml) in the presence of (a) recombinant UNC5b-Fc or IgG control, (b) anti-UNC5b or isotype-matched control antibody, or (c) an inhibitor of the A2B adenosine receptor (10 uM 8-PT). (d-e) Migration of pMø to CCL19 (500 ng/ml) in (d) the presence/absence of conditioned medium (CM) from macrophages treated with LDL or oxLDL (50 µg/ml; 48 h) and (e) with/without recombinant UNC5b-Fc or IgG control. (f) Migration of pMø to CCL19 (500 ng/ml) as in (e) except using conditioned medium (CM) from WT or *Ntn1*^{*-*/*-*} macrophages treated with oxLDL. Inset: immunoblot of netrin-1 in lysates of WT and *Ntn1*^{*-*/*-*} pMø. Data are the mean ± s.d. of triplicate samples in a single experiment and are representative of an experimental n=3. *P<0.01. (g) Netrin-1 blocks the emigration of leukocytes in a model of mouse peritonitis. C57BL6 mice with established thioglycollate-induced peritonitis were pre-treated with netrin-1 (500 ng, ip; 1 h) or PBS, and then injected with LPS (400 ng, ip) which induces efflux of leukocytes from the peritoneum to the lymphatics. The number of leukocytes in the peritoneum was determined after 4 h. Data are the mean ± s.e.m, n=6. *P<0.05.

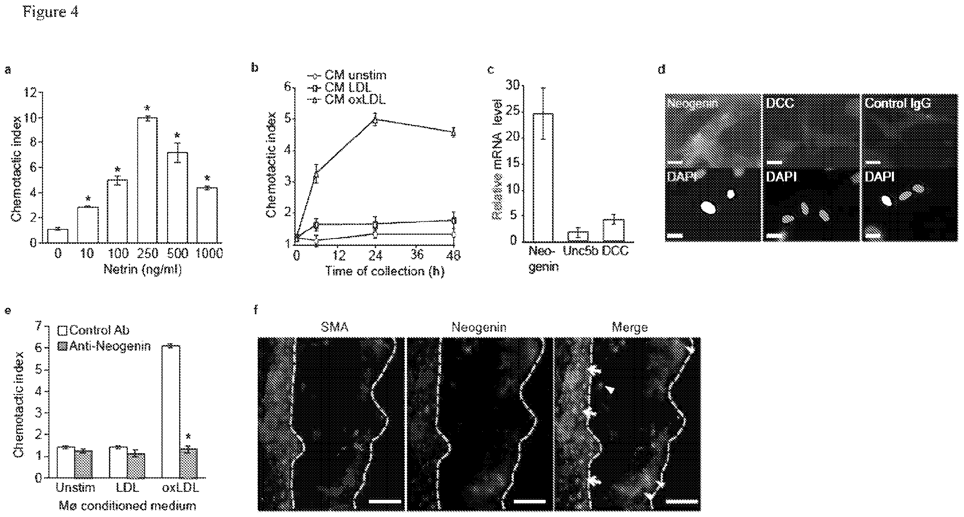

Figure 4 demonstrates that netrin-1 acts as a chemoattractant for smooth muscle cells via the receptor Neogenin. (a) Migration of human CaSMC in the presence of recombinant netrin-1. (b) Migration of CaSMC in the presence of conditioned media from macrophages treated for the indicated times with LDL or oxLDL (50 µg/ml). (c) qPCR analysis of the netrin-1 receptors *Neogenin, Unc5b* and *Dcc* in CaSMC. (d) Immunofluorescent staining for neogenin, DCC or isotype matched control antibody in CaSMC. Cells were co-stained with DAPI nuclear stain. Scale bar, 10 µm. (e) Migration of CaSMC pre-treated with anti-neogenin or isotype control antibody prior to exposure to conditioned media from macrophages treated as indicated for 24 h. (f) Immunofluorescent staining of alpha smooth muscle actin (SMA, green), neogenin (red) and DAPI (blue) in atherosclerotic plaques of *Ldlr*^{*-*/*-*} mice fed a WD. Co-localization of SMA and neogenin (yellow in the merged image) was detected in the media (arrows) and in SMC that had invaded the intima (arrowheads). Scale bar, 50 µm. (a-c, e) Data are the mean ± s.d. of triplicate samples in a single experiment and are representative of an experimental n of 3. *P<0.05.

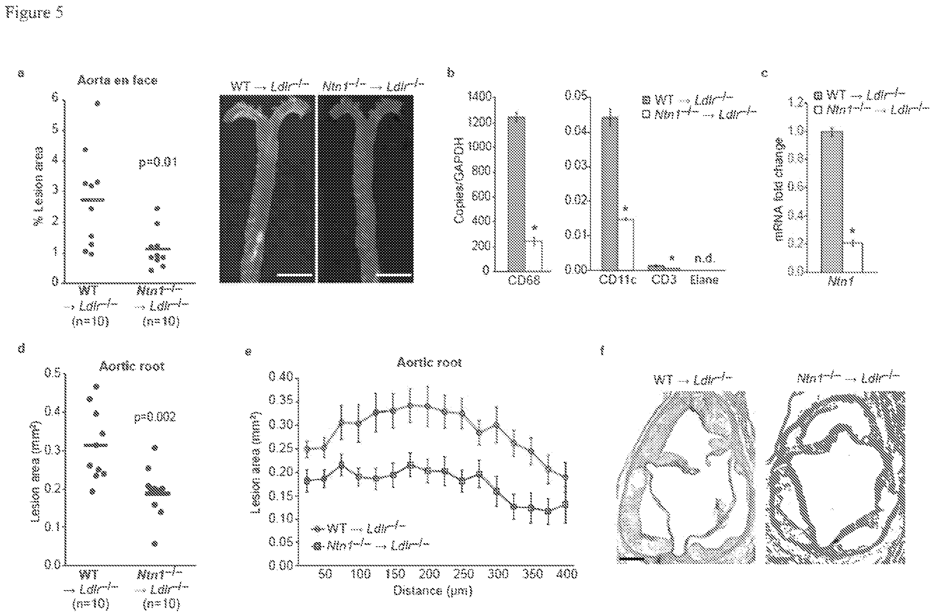

Figure 5 demonstrates that targeted deletion of netrin-1 in immune cells reduces atherosclerosis burden. (a) Quantification and representative photographs of atherosclerosis in the aorta *en face* of *WT→Ldlr*^{*-*/*-*} and *Ntn1*^{*-*/}*⁻ → Ldlr*^{*-*/*-*} chimeric mice fed a WD for 12 weeks. Scale bar, 5 mm. (b-c) qPCR analysis of *Cd68* (macrophage), *Cd3* (T cell), *Cd11c* (dendritic cell), *Elane* (neutrophil) and *Ntnl* mRNA in the aortic arches of *WT→Ldlr*^{*-*/*-*} and *Ntn1*^{*-*/}*⁻ →Ldlr*^{*-*/-} chimeric mice. (d-e) Lesion area of atherosclerotic plaques of the aortic root of *WT →Ldlr*^{*-*/*-*} and *Ntn1*^{*-*/}*⁻ →Ldlr*^{-/-} mice expressed as the mean (d) of individual mice and (e) of each genotype across the 400 µm of the aortic root. (f) Representative photographs of hematoxylin and eosin stained aortic sinus lesions of *WT→Ldlr*^{*-*/*-*} and *Ntn1*^{*-*/}*⁻ →Ldlr*^{-/-} mice. Scale bar, 200 µm. Data in b and c are the mean ± s.d. of triplicate samples in a single experiment and are representative of an experimental n=3. *P<0.01.

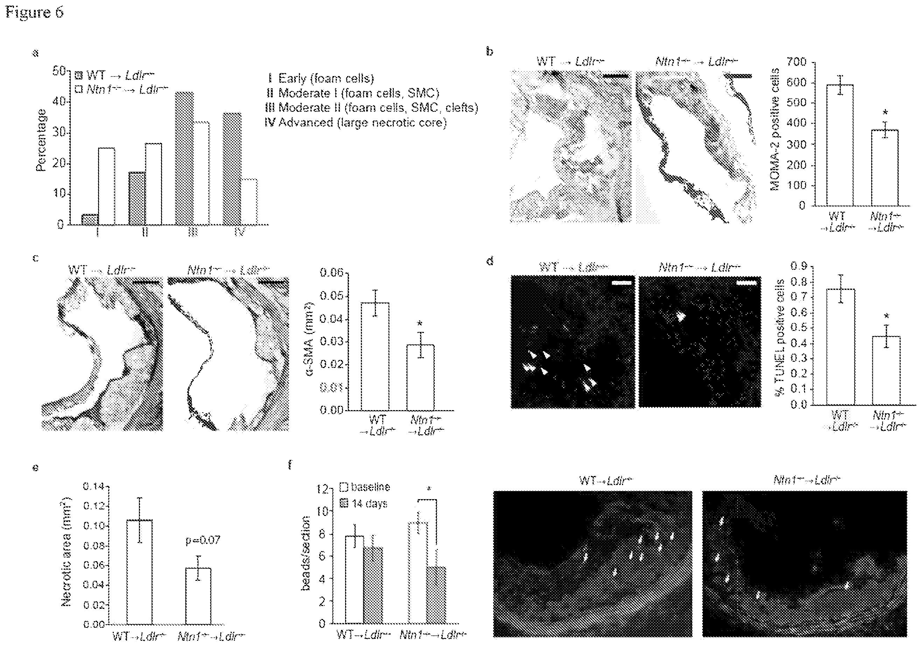

Figure 6 demonstrates that targeted deletion of netrin-1 in immune cells reduces plaque complexity and promotes macrophage emigration. (a) Classification of aortic sinus plaques of *WT→Ldlr*^{*-*/*-*} and *Ntn1*^{*-*/}*⁻ →Ldlr*^{-/-} mice (n=10) according to the Stary method. (b,c) Immunohistochemical staining of aortic sinus plaques for (b) macrophages (MOMA-2) and (c) SMC (α-smooth muscle actin). Scale bar, 100 µm. Staining quantified using IP Lab Spectrum software is presented at right. Data are the mean ± s.e.m, n=6-9. *P<0.05. (d) Immunofluorescent staining of apoptotic cells (green) in aortic sinus plaques. TUNEL positive nuclei are indicated by arrowheads TUNEL and quantification is presented at right. Scale bar, 50 µm. Data are the mean ± s.e.m, n=9. *P<0.05. (e) Quantification of necrotic areas of aortic sinus plaques. Data are the mean ± s.e.m, n=10. (f) In vivo analysis of macrophage recruitment and retention in atherosclerotic plaques of *WT→Ldlr*^{-/-} and *Ntn1*^{-/-} *→Ldlr*^{-/-} mice using a monocyte bead-tracking model. The mean number of bead-labeled macrophages per plaque (± s.e.m) was assessed after 3 days (baseline) and 14 days later (n=3-4/group). Fewer bead-labeled macrophages were present in plaques from *Ntn1*^{-/-}*→Ldlr*^{-/-} mice after 14 days, indicating emigration of the cells. *P<0.05. Representative image of plaques stained for CD68 (red) and cell nuclei (blue). Arrows indicate the presence of the cells containing fluorescent beads (green) within the lesion.

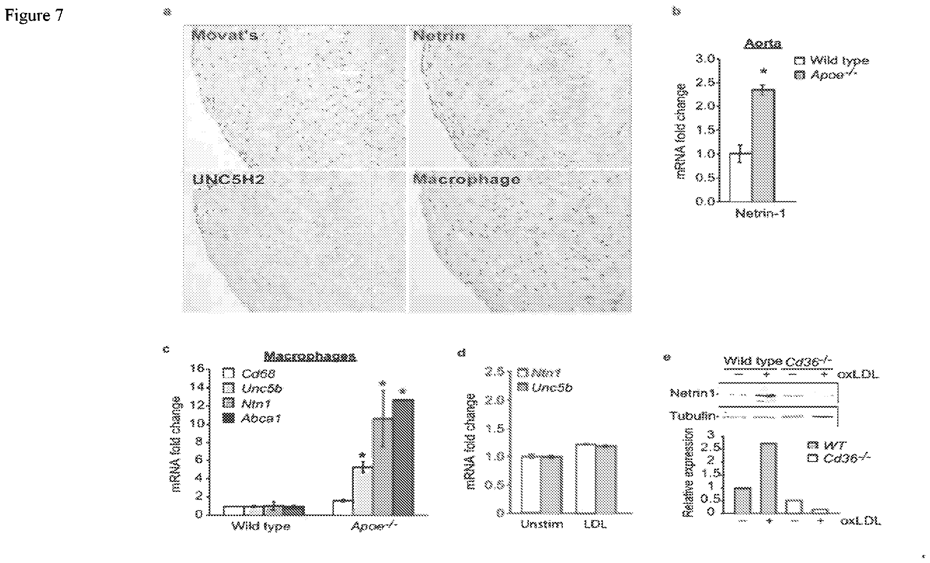

Figure 7 demonstrates that netrin-1 and its receptor UNC5b are expressed in human and Apoe-/- mouse atherosclerotic plaques. (a) Immunohistochemical staining for netrin-1, UNC5H2 (UNC5b) and macrophages (HAM56) in serial sections of a human atherosclerotic plaque from the left anterior descending coronary artery. Movat's pentachrome stain was used to show histological features of the plaque (original Mag x 200). (**b**) qPCR analysis *of Ntnl* mRNA isolated from the aortic arch of C57BL6 or *Apoe-*/*-* mice fed a chow diet for 6 months. (**c**) qPCR analysis *of Ntnl* and *Unc5b* mRNA in peritoneal macrophages isolated from C57BL6 or *Apoe-*/*-* mice fed chow for 6 months. Expression of the macrophage marker *Cd68* and the cholesterol responsive gene *Abca1* were measured as controls. (**d**) qPCR analysis *of Ntnl* and *Unc5b* mRNA in peritoneal macrophages treated with 50 µg/ml LDL (6 h). (**e**) Western blot of netrin-1 protein in wild type (WT) or Cd36-/- macrophages stimulated with oxLDL (50 µg/ml, 48 h). Data are mean ± s.d. of triplicate samples in a single experiment and are representative of an experimental n=3. *P<0.05.

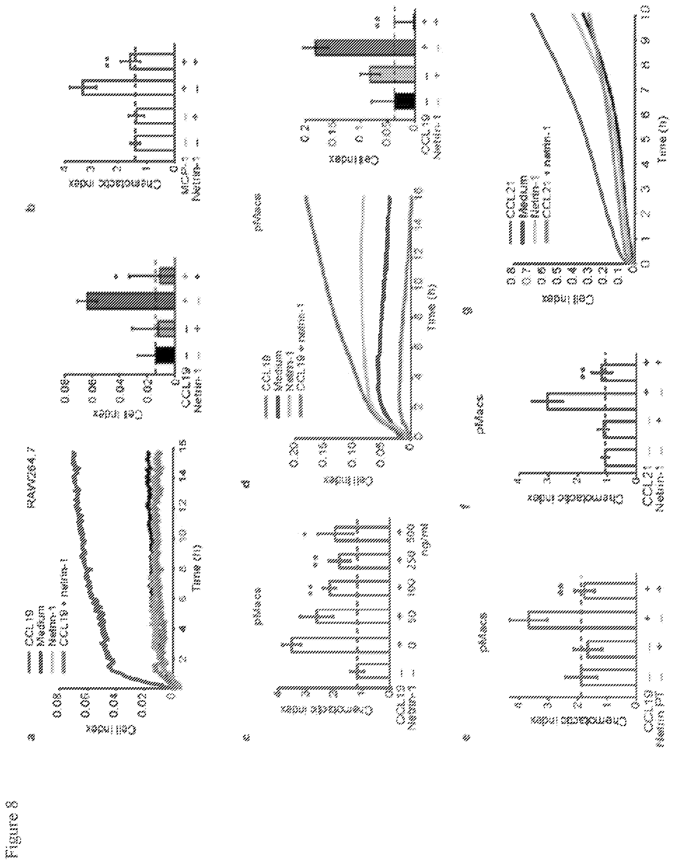

Figure 8 demonstrates that netrin-1 inhibits macrophage migration to MCP-1 and CCL19. (a) Measurement of RAW264.7 cell migration to 250 ng/ml netrin-1, 500 ng/ml CCL19, or both, using the Real-time Cell Invasion and Migration xCelligence system. Graph at right shows the mean cell index ±s.d. of triplicate samples at the 16 h endpoint. (b) Migration of RAW264.7 cells to 250 ng/ml netrin-1, 100 ng/ml MCP-1, or both as measured by Boyden chamber assay. (c) Migration of peritoneal macrophages to CCL19 (500 ng/ml) in the presence of increasing concentrations of recombinant netrin-1. (d) Real time measurement of peritoneal macrophage migration to 250 ng/ml netrin-1, 500 ng/ml CCL19, or both. Graph at right shows the mean cell index ±s.d. of triplicate samples at the 16 h endpoint. (e) Migration of peritoneal macrophages pretreated (PT) with 250 ng/ml netrin-1 for 1 hour and washed 3x prior to exposure to CCL19 (500 ng/ml). (f) Migration of mouse peritoneal macrophages to 250 ng/ml netrin-1, 500 ng/ml CCL21, or both as measured by Boyden chamber assay. (g) Real time measurement of macrophage migration to 250 ng/ml netrin-1, 500 ng/ml CCL21, or both. (a-f) Data are mean ±s.d. of triplicate samples in a single experiment and are representative of an experimental n of ≥3. *P<0.05, **P<0.01.

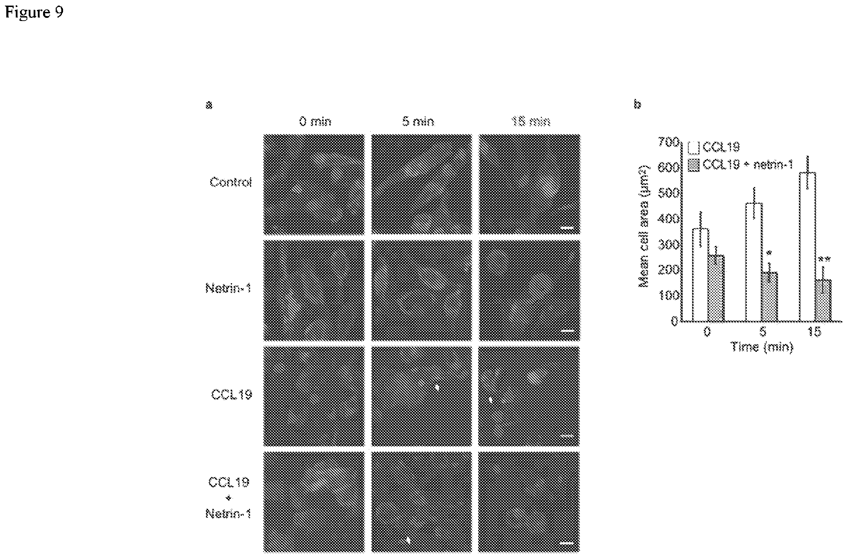

Figure 9 demonstrates that mouse peritoneal macrophages were incubated with 500 ng/ml CCL19 with/without 250 ng/ml netrin-1 pretreatment. After the indicated times the cells were fixed, permeabilized and stained with phalloidin to detect polymerized actin. (**a**) Cell morphology was analyzed with fluorescence microscopy and arrows indicate membrane ruffles. Scale bar, 10 µm. (**b**) mean surface area of cells in (a). Data are the mean of 5 high power fields ±s.d. *P<0.05.

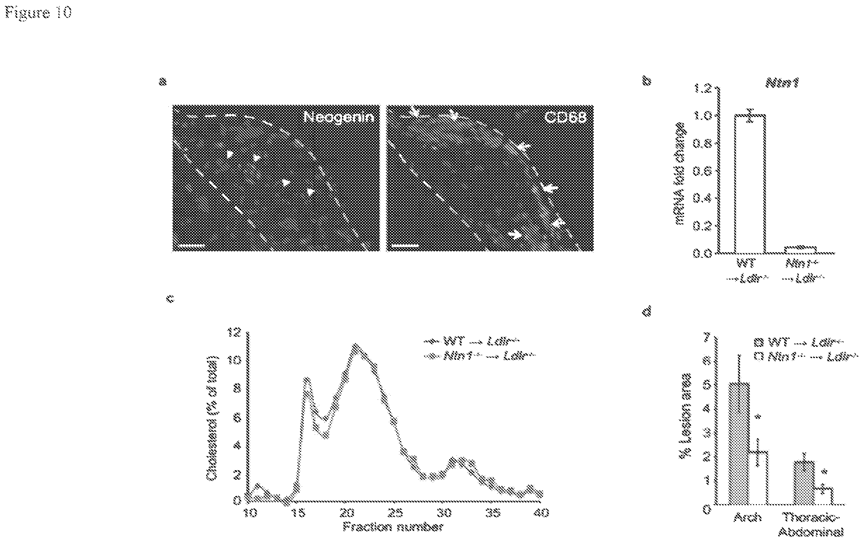

Figure 10 shows (a) Immunofluorescent staining of neogenin (left panel, green) or CD68 (right panel, green) and DAPI (blue) in serial sections *of* atherosclerotic plaques of *Ldlr-*/*-* mice fed a Western diet. Arrowheads indicate neogenin staining in lesion areas distinct from CD68+ regions, indicated by arrows. Scale bar, 50 µm. (**b**) qPCR analysis *of Ntnl* mRNA in peritoneal macrophages of *WT→Ldlr-*/*-* and *Ntn1-*/*- →Ldlr-*/*-* chimeric mice confirming reconstitution of hematopoietic cells to the donor genotype. (**c**) FPLC fractionation of pooled plasma from *WT →Ldlr-*/*-* and *Ntn1-*/*-* →*Ldlr*-/-chimeric mice (n=3/group) fed a Western diet for 12 weeks. (**d**) Regional analysis of lesion distribution in the en face aorta of *WT →Ldlr-*/*-* and *Ntn1-*/*- →Ldlr-*/- chimeric mice fed a Western diet for 12 weeks (n=10/group). Data are expressed as mean % lesion area ±s.e.m. *P<0.05.

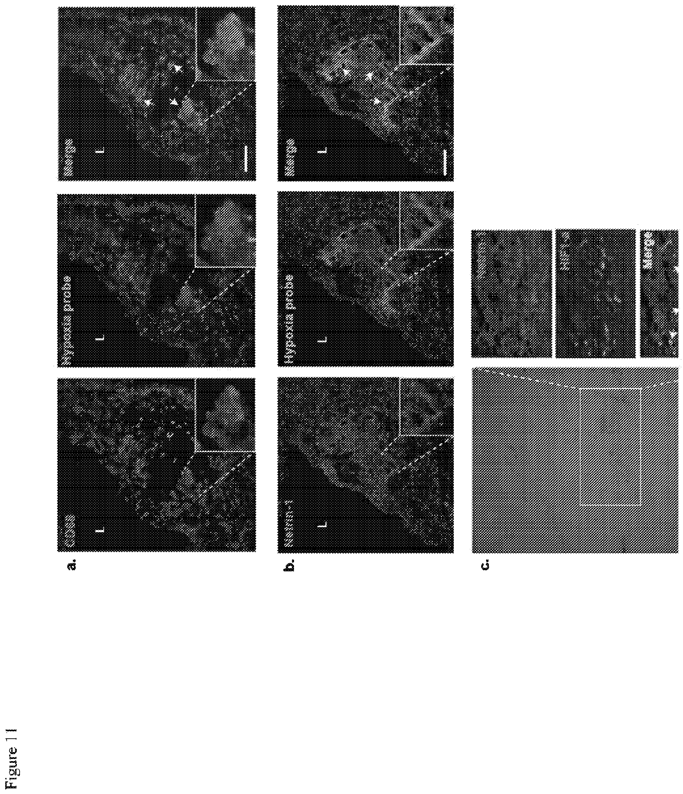

Figure 11 demonstrates that hypoxia correlates with Ntn1 expression in mouse and human atherosclerotic plaques. Immunofluorescence staining of (a) macrophage CD68 (red) or (b) netrin-1 (red) in aortic root plaques from *Ldlr*^{-/-} mice fed a Western diet for 12 weeks and injected with a hypoxia probe (green) that detects hypoxic regions. Areas of co-localization are shown in yellow in the merged image (arrows). Scale bar, 100 µm. The inset is a 10x zoom of the area of interest. Images are representative of n ≥ 3 mice. (c) Immunofluorescence staining of human carotid plaques for Netrin-1 (red) and HIF-1α (green). DAPI nuclear stain is in blue. Areas of co-localization are shown in yellow in the merged image (arrows). Scale bar 500µm. L indicates the lumenal side.

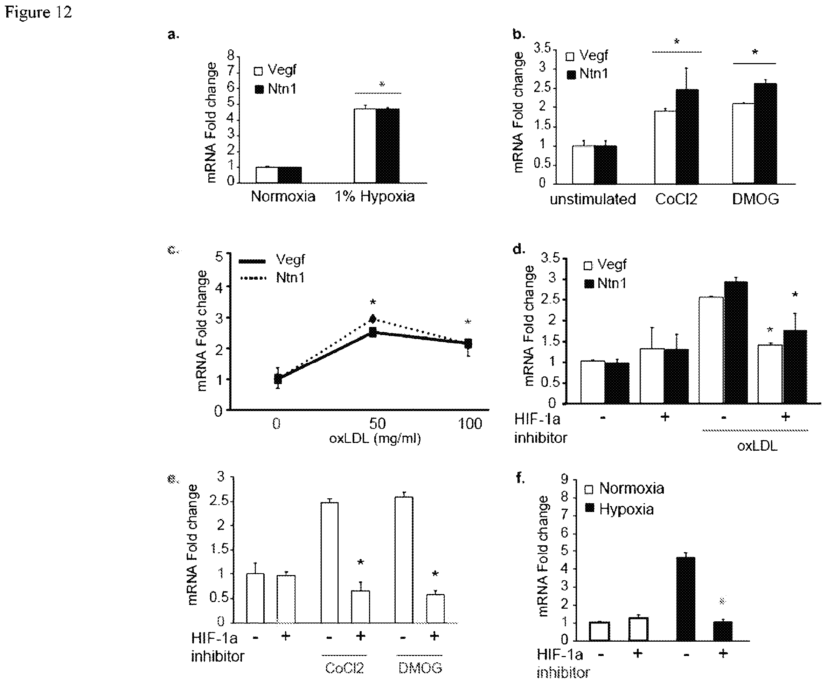

Figure 12 demonstrates that mediators of hypoxic stress induce *Ntnl* in macrophages. (a) qPCR analysis of *Ntn1* and *Vegf mRNA* in BMDMs (a) exposed to 1% hypoxia or stimulated with (b) DMOG (1 mmol/L) and CoCl₂ (0.1 mmol/L) for 24 hours. (c-d) qPCR analysis of *Ntn1* and *Vegf mRNA* expression in peritoneal macrophages treated with oxLDL with or without HIF-1 α inhibitor (100 µmol/L). (e-f) qPCR analysis *of Ntnl* and *Vegf* mRNA expression in BMDMs exposed to or stimulated with DMOG (1 mmol/L) or CoCl₂ (0.1 mmol/L) or 1% hypoxia for 24 hours with or without HIF-1 α inhibitor (100 µmol/L). Data are mean ± s.d. of triplicate samples in a single experiment and are representative of an experimental n=3. *P<0.05.

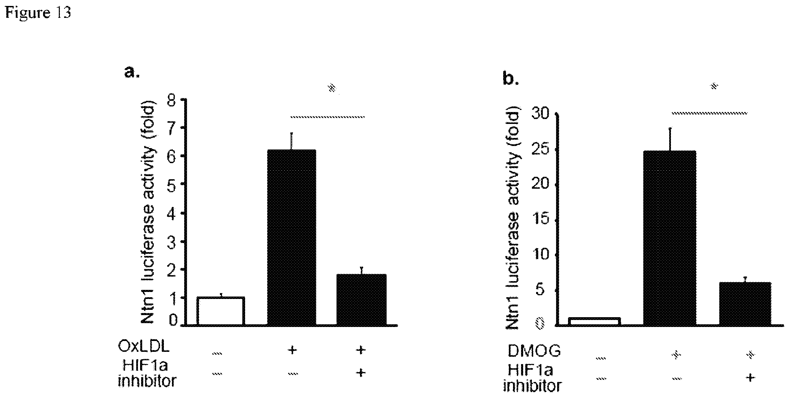

Figure 13 demonstrates HIF-1α activates the Ntn1 promoter. (a) Netrin-1 promoter-luciferase reporter activity in HEK293 cells treated with (a) oxLDL (50µg/ml) or (b) DMOG (1 mmol/L) in the presence/absence of an HIF-1α inhibitor (100µmol/L). Data are mean ± s.d. of triplicate samples in a single experiment and are representative of an experimental n=4. *P<0.05.

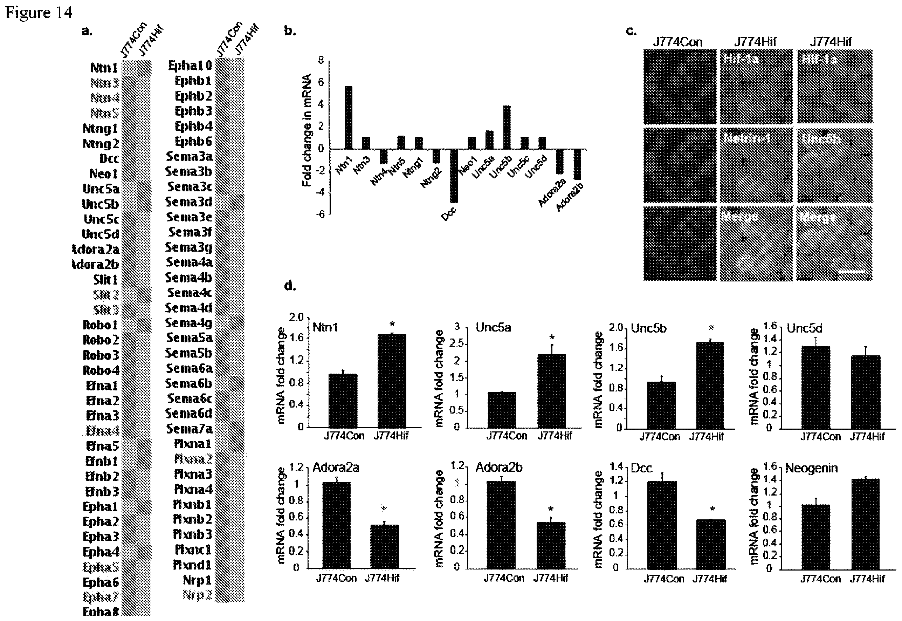

Figure 14 demonstrates that netrin-1 and unc5b are regulated by a common mechanism during hypoxia. (a-b) mRNA profiling of J774 macrophages overexpressing a stable form of HIF-1α (J774Hif) versus J774 control (J774Con) cells using a custom qRT-PCR array for the neuronal guidance cue family members (n=3) expressed as (a) a heat map or (b) relative fold change in mRNA expression. (c) Immunofluorescence staining of J774Con and J774Hif cells for HIF-1α netrin-1 and Unc5b. Scale bar = 50 µm. (d) qPCR validation of mRNA expression of *Ntnl* and its receptors in J774Hif compared to J774con macrophages. Data are mean ± s.d of triplicate samples in a single experiment and are representative of an experimental n=4. *P<0.05.

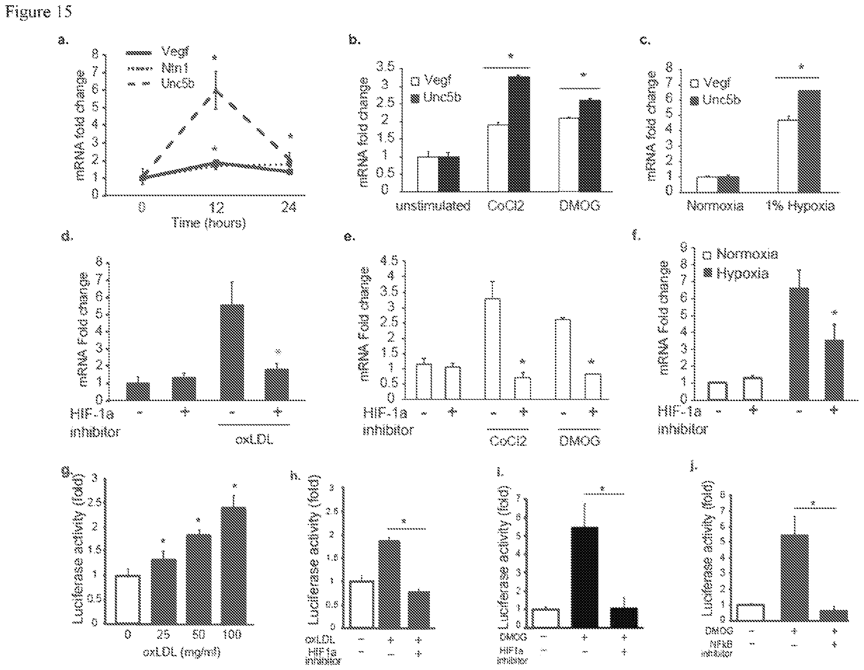

Figure 15 demonstrates that mediators of hypoxic stress induce *Unc5b* in macrophages. qPCR analysis of *Unc5b, Ntn1* or *Vegf* mRNA in peritoneal macrophages treated with (a) 50µg/ml oxLDL. (b) DMOG (1 mmol/L) and CoCl₂ (0.1 mmol/L) or (c) 1% oxygen, and (d-f) with or without HIF-1α inhibitor (100 µmol/L). (g) Unc5b promoter-luciferase reporter activity in HEK293 cells treated with the indicated concentration of oxLDL. (h-j) Unc5b promoter-luciferase reporter activity in HEK293 cells treated with (h) oxLDL (50µg/ml) or (i-j) DMOG (1 mmol/L) in the presence/absence of a (h-i) HIF-1α (100µmol/L) or (j) NFkB inhibitor (BAY11-7082). Data are mean ± s.d. of triplicate samples in a single experiment and are representative of an experimental n=3. *P<0.05.

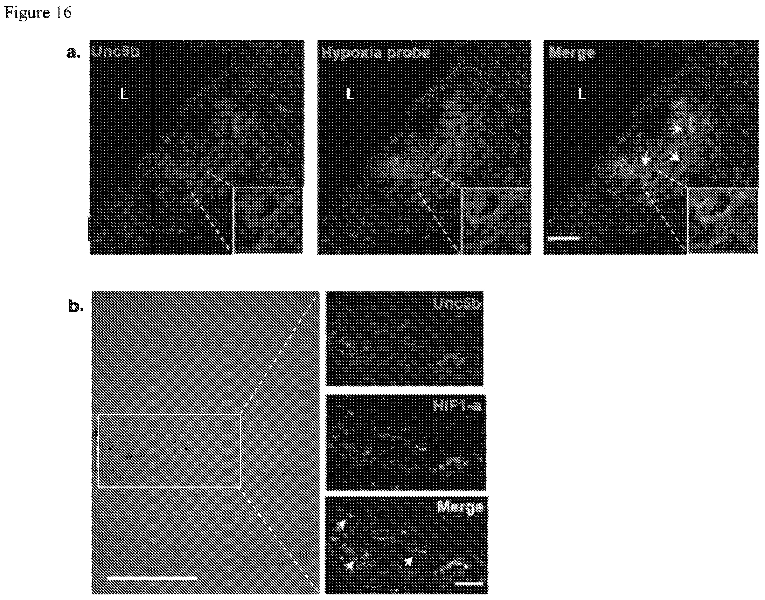

Figure 16 demonstrates that Unc5b is expressed in hypoxic-rich regions in mouse and human atherosclerotic lesions. Immunofluorescence staining of (a) Unc5b (red) in aortic root plaques from *Ldlr*^{-/-} mice fed a Western diet for 12 weeks and injected with a hypoxia probe (green) to detect hypoxic regions. Areas of co-localization are shown in yellow in the merged image (arrows). Scale bar, 100 µm. The inset is a 10x zoom of the area of interest. L indicates the lumenal side. Images are representative of n ≥ 3 mice. (b) Immunofluorescence staining of human carotid plaques for Unc5b (red) and HIF-1α (green). DAPI nuclear stain is in blue. Areas of co-localization are shown in yellow in the merged image (arrows). Scale bar 500µm.

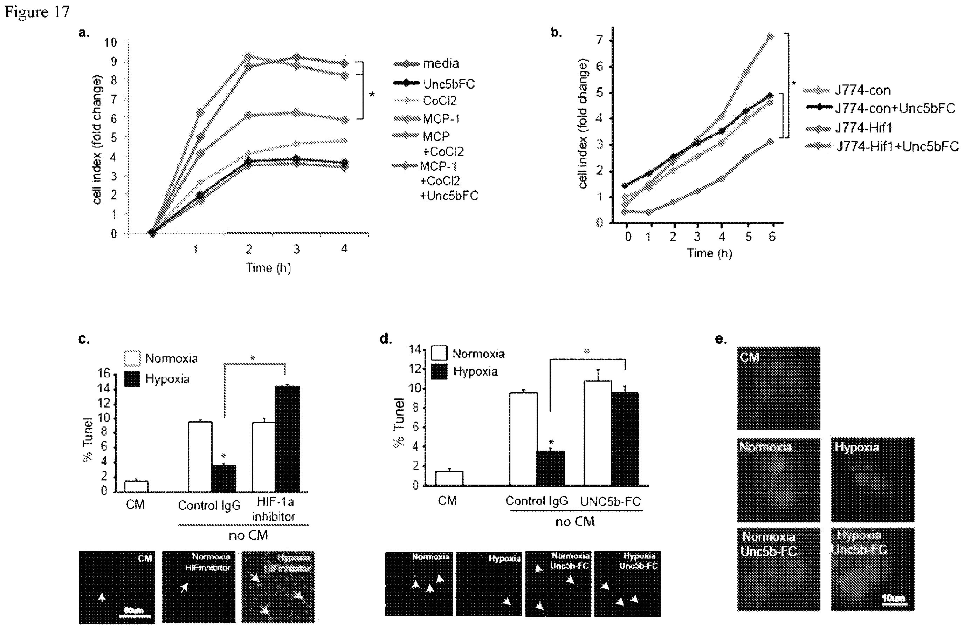

Figure 17 demonstrates that hypoxia-induced netrin-1 inhibits the migration and promotes the survival of macrophages. (a) Real-time migration of BMDMs to media alone, MCP-1 (100 nmol/L) in the presence or absence of CoCl2 (0.1 nmol/L) or Unc5bFC. (b) Real-time migration of J774con or J774Hif macrophages in the presence or absence of Unc5b-Fc to block the effects of netrin-1. (c-d) Apoptosis measured by Tunel assay of BMDMs cultured in L929 conditional media (CM) or deprived of growth factor (- CM) under normoxic or hypoxic (1% oxygen) conditions. Cells were incubated with (c) a HIF-1α inhibitor (100µM) (d) control IgG or Unc5b-FC. Data are the mean ± s.d of triplicate samples in a single experiment and are representative of an experimental n=3. *P<0.05. Representative images of Tunel staining are shown below. Arrows indicate Tunel positive cells. (e) BMDM apoptosis as measured by active caspase-3 staining of BMDMs cultured in L929 conditional media (CM) or deprived of growth factor and cultured under normoxic or hypoxic (1% oxygen) conditions in the presence or absence of Unc5b-FC.

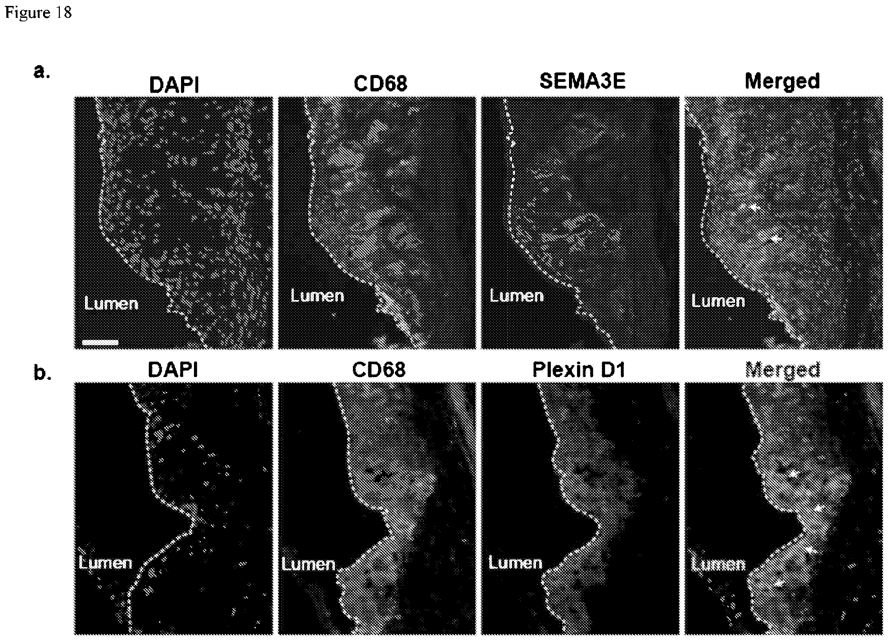

Figure 18 demonstrates that Sema3E and its receptor PlexinD1 are expressed by macrophages in advanced atherosclerotic plaques. Immunofluorescent staining of CD68 (green), DNA (DAPI, blue) and (a) Sema3E (red) or (b) PlexinD1 (red) in aortic root atherosclerotic plaques of *Apoe*^{-/-} mice fed a Western diet for 12 weeks. Areas of co-localization are shown in yellow in the merged image (arrows). Scale bar, 50 µm. Images are representative of n ≥ 3 mice.

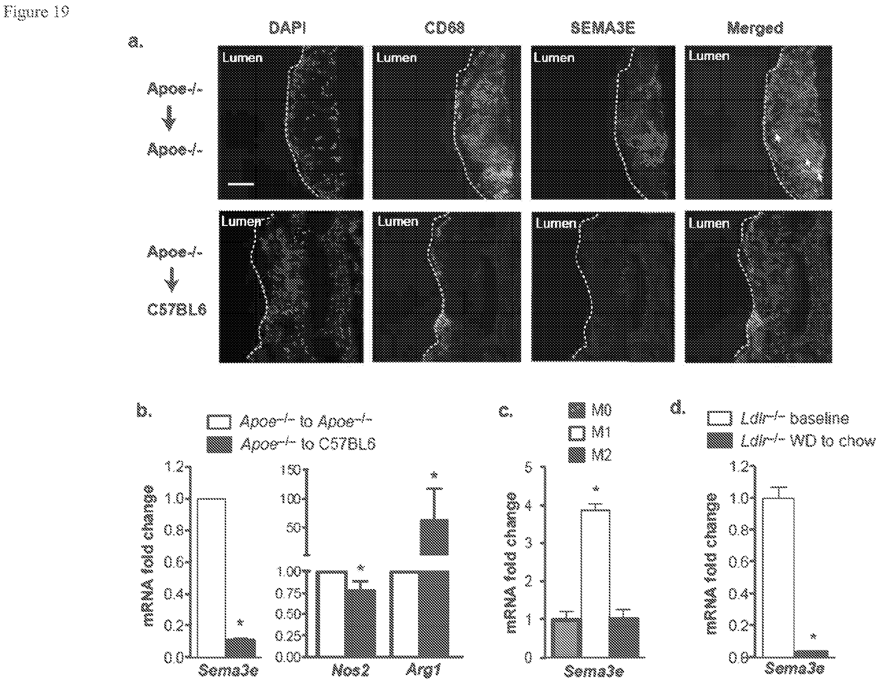

Figure 19 demonstrates that Sema3E expression by lesional macrophages is downregulated during atherosclerosis regression. (a) Immunofluorescent staining of CD68 (green) and Sema3E (red) in aortic arch plaques transplanted into a progressive (*Apoe*^{-/-} recipient) or regressive (C57BL6 recipient) environment. Areas of co-localization (yellow) are shown in the merged image. Images are representative of n ≥ 3 mice. (b) qPCR analysis of mRNA from laser captured CD68+ cells in aortic arch plaques from mice in (a) (n=4 mice/group). (c) qPCR analysis of *Sema3e* mRNA in BMDMs polarized towards M1, M2 or unpolarized (M0). (d) qPCR analysis of *Sema3e* mRNA from laser captured CD68+ cells in aortic root plaques from *Ldlr*^{-/-} mice fed a western diet for 14 weeks (baseline) and then switched to a chow diet for 4 weeks (regression conditions) (n=5 mice/group). Data in b-d are the mean of triplicate samples ± SD. *P<0.05

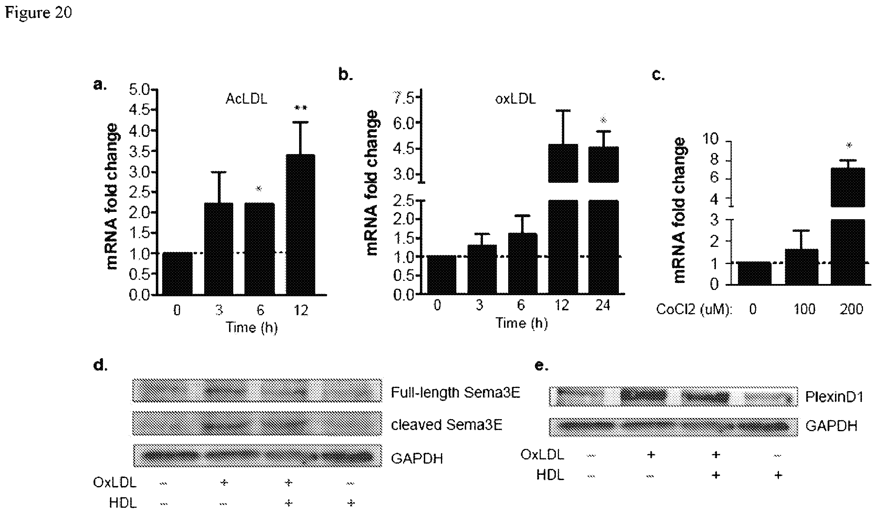

Figure 20 demonstrates that Sema3E expression is up-regulated by physiological drivers of plaque inflammation. qPCR analysis of *Sema3e* mRNA in BMDMs treated with (a) 50 µg/ml of AcLDL (*P<0.01 and **P<0.05) or (b) oxLDL (*P<0.05) for the indicated times, or (c) CoCl₂ for 24 h (P*<0.01). (d) Western blot of full-length and cleaved Sema 3E in supernatants from BMDMs stimulated with 50 µg/ml oxLDL, 10 µg/ml HDL, or both for 24 h. (e) Western blot of PlexinD1 in cell lysates of BMDMs stimulated with 50 µg/ml oxLDL, 10 µg/ml HDL, or both for 24 h. Data in a-c are the mean of triplicate samples ± SD.

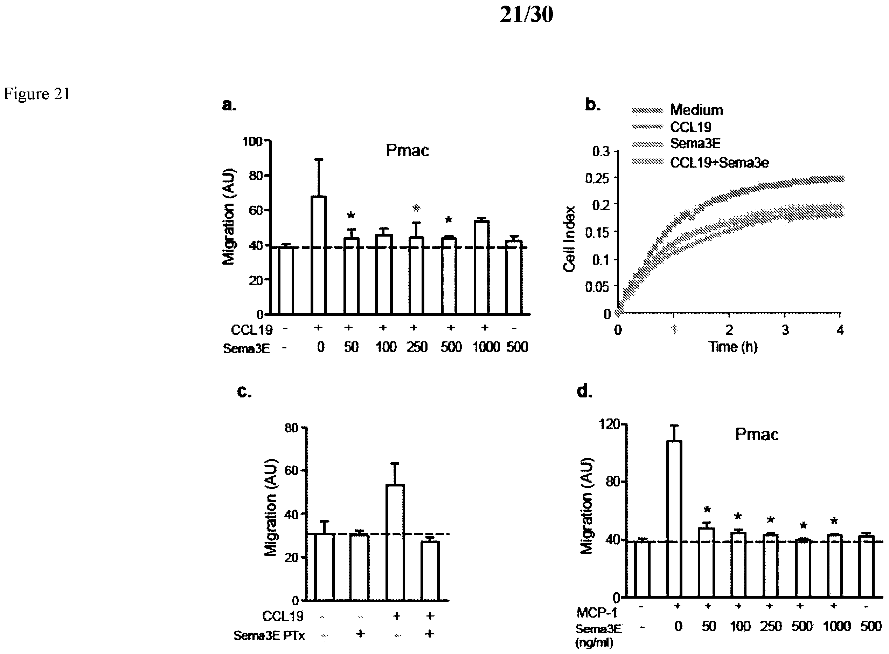

Figure 21 demonstrates that Sema3E inhibits the migration of macrophages. (a) Transwell migration of peritoneal macrophages to CCL19 (500 ng/ml) with or without recombinant Sema3E at the concentrations indicated. (b) Real-time migration (xCelligence) of peritoneal macrophages to CCL19 with or without 250 ng/ml Sema3E. (c) Transwell migration of peritoneal macrophages pre-treated with 250 ng/ml Sema3E for 45 minutes, washed and then exposed to 500 ng/ml CCL19. (d) Transwell migration of peritoneal macrophages to 100 ng/ml CCL2 with or without recombinant Sema3E at the concentrations indicated. Data are the mean ± s.d. of triplicate samples in a single experiment and are representative of an experimental n=3. *P<0.05.

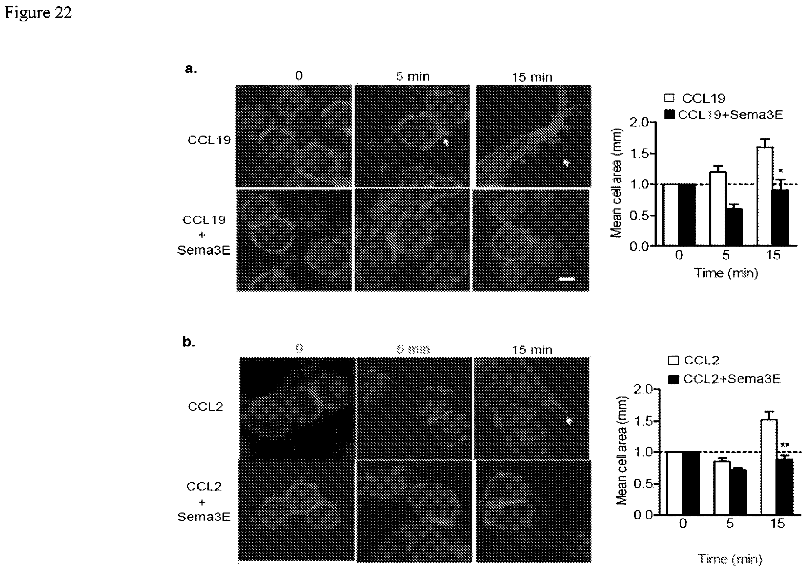

Figure 22 demonstrates that Sema3E affects the organization of the actin cytoskeleton. Peritoneal macrophages stained with Phalloidin to detect polymerized actin after treatment with (a) 500 ng/ml CCL19 or (b) 100 ng/ml CCL2 in the presence or absence of recombinant Sema3E (250 ng/mL). Arrows indicate membrane ruffles, scale bar 10 µm. Mean cell surface area of macrophages is graphed at right.

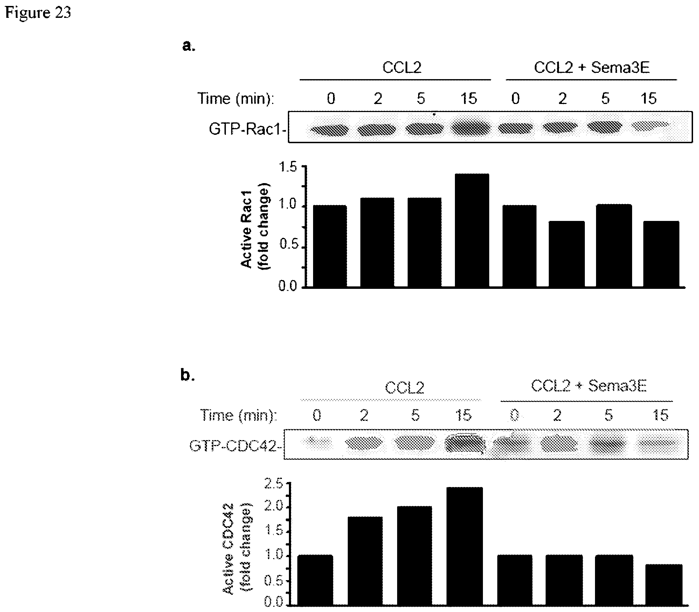

Figure 23 demonstrates that Sema3E impairs Rho GTPAse signaling. Immunoblot of activated (a) Rac1 and (b) CDC42 in macrophages treated with 100 ng/ml CCL2 with and without 250 ng/ml Sema3E. Densitometry quantification of blots is shown graphically.

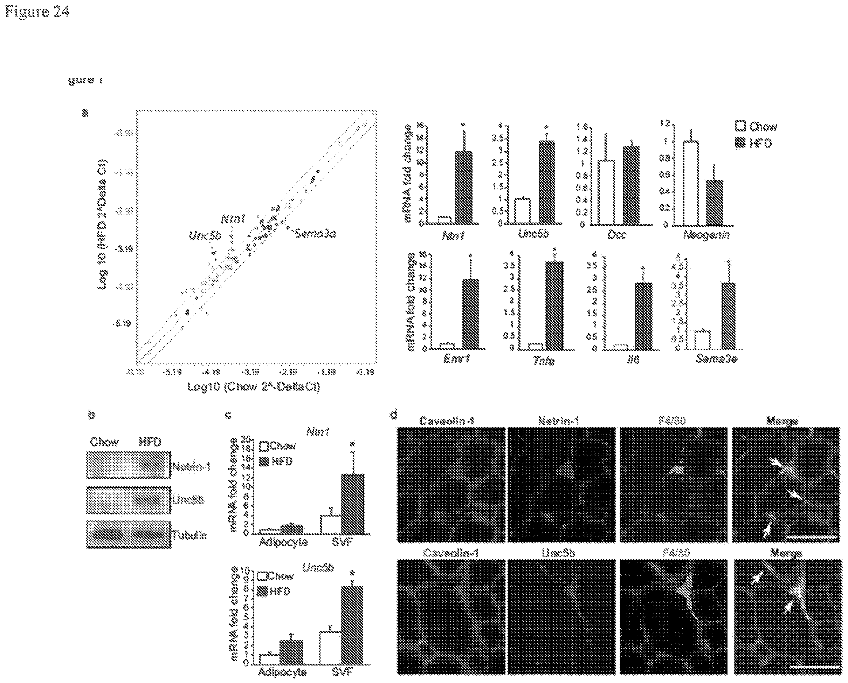

Figure 24 demonstrates that netrin-1 and Unc5b are upregulated in obese-adipose tissue. (a) Scatter plot of neuronal guidance cue expression in visceral adipose tissue from mice fed a HFD or chow for 20 weeks: upregulated genes are indicated in red; downregulated genes in green (n=3 micelgroup). Genes that were differentially expressed were confirmed by qRT-PCR of mRNA from WAT of mice fed chow (n=5 mice) or HFD (n=6 mice) (right panel). (b) Western blot of netrin-1 and Unc5b in WAT from mice fed chow or HFD. Tubulin is shown as an internal control. (c) Relative mRNA expression of Ntnl and Unc5b in the adipocyte and SVF fraction isolated from WAT of mice fed chow (n=4 mice) or HFD (n=6 mice). (d) Immunofluorescence staining for netrin-1 (red, upper) or Unc5b (red, lower), the macrophage marker F4180 (green) and caveolin-I (blue) in WAT of HFD-fed mice. Co-localization of netrin-1 or Unc5b with F4180 is seen in yellow in the merged image (arrows). Scale bar = 100 pm. Data in a and c are mean k s.e.m. **P* < 0.05.

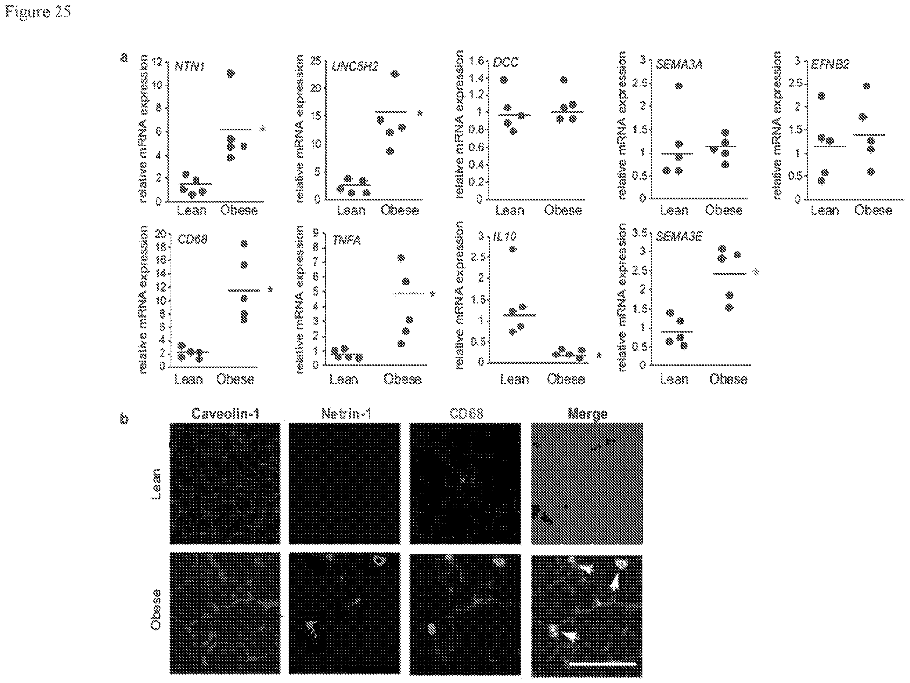

Figure 25 demonstrates that netrin -1 is expressed by adipose tissue macrophages in obese human WAT. (a) qRT-PCR analysis of mRNA isolated from WAT of lean (n=5) or obese (n=5) human subjects. Data are the mean k s.e.m. **P* < 0.05. (b) Immunofluorescence staining of netrin-1 (red), caveolin-1 (blue), and CD68 (green) in visceral adipose of lean and obese individuals. Co-localization of netrin-1 and CD68 is seen in yellow in the merged image (arrows). Scale bar=100 pm.

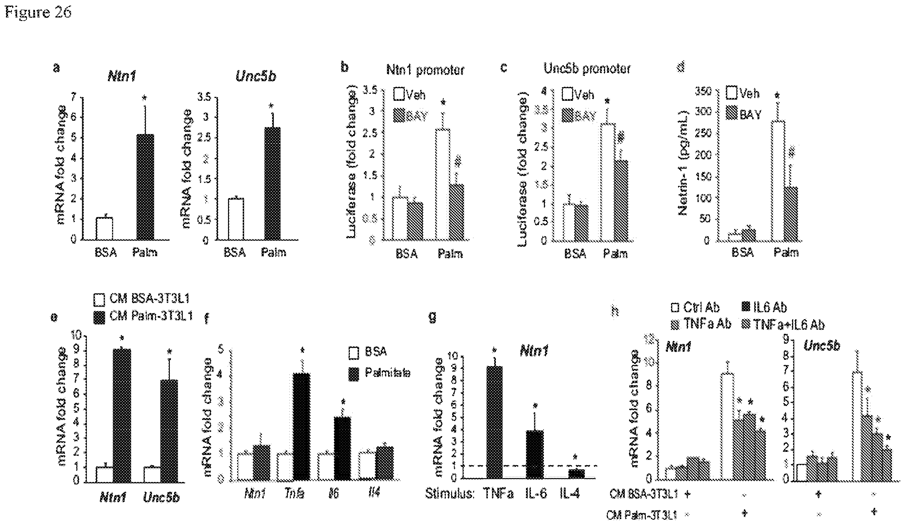

Figure 26 demonstrates that palmitate upregulates *Ntnl* and *Unc5b* expression in macrophages. (a) qRTPCR analysis of mRNA for Ntnl and Unc5b in BMDM treated with 250 pM BSA or palmitate for 24 h. (b-c) NTNI and Unc5b promoter-luciferase reporter activity in HEK293T cells treated with 250 pM BSA or palmitate in the presence or absence (Vehicle) of the NFkB inhibitor BAY1 1-7082 (10 pmollL). (d) netrin-1 ELISA of conditioned media from BMDM treated with 250 pM BSA or palmitate for 24 h in the presence or absence of BAY1 1-7082. (e) qRT-PCR of Ntnl and Unc5b mRNA in BMDM treated with conditioned media (CM) from 3T3L1 adipocytes exposed to BSA or palmitate (250 pM) for 24 h. (f) qRT-PCR of mRNA for the indicated genes in 3T3L1 adipocytes treated with BSA or palmitate (250pM) for 24 h. (g) qRT-PCR of Ntnl mRNA in BMDM treated with TNFa (10 nglml), IL-6 (40 nglml) or IL-4 (10 nglml) for 24 h. (h) Ntnl and Unc5b mRNA in BMDM treated with conditioned media (CM) from 3T3L1 adipocytes exposed to BSA or palmitate (250 pM) in the presence or absence of anti-TNFa, anti-IL-6 or both. Data are the mean + s.d. of triplicate samples. Data presented are from a single experiment and are representative of three independent experiments. **P* < 0.05.

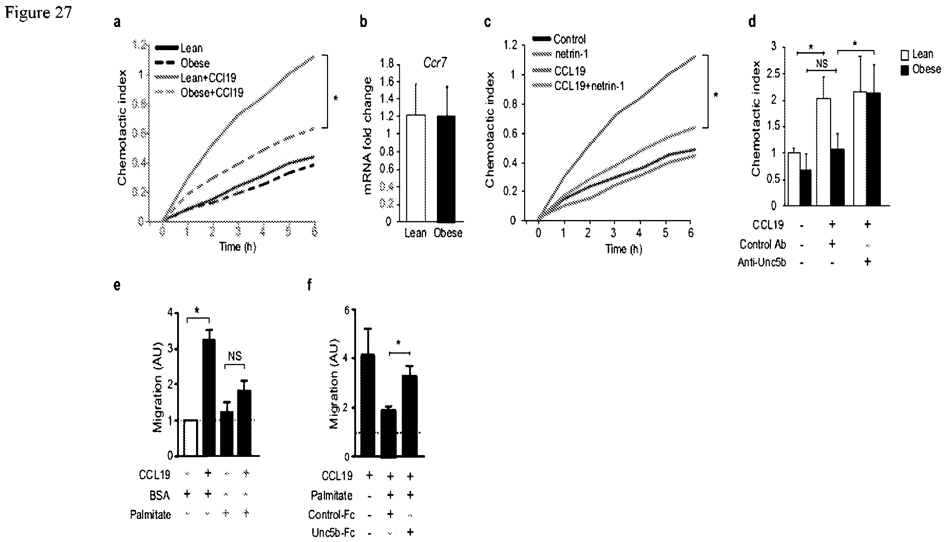

Figure 27 demonstrates that netrin-1 blocks chemokine-induced migration of ATM. (a) Migration of ATM isolated from WAT of lean (chow-fed) or obese (HFD-fed) mice and exposed to CCL19 (500 nglml). (b) qRT-PCR analysis of Ccr7 mRNA in ATMs isolated from lean or obese WAT. (c) Migration of ATM isolated from lean mice and exposed to either CL19, netrin-1 (250 nglml) or both. (d) Migration of ATM isolated from WAT of lean or obese mice and exposed to CCL19 in the presence or absence of UNC5b blocking antibody or isotype matched control antibody (Control Ab). (e-f) Migration of BMDM pretreated with 250 IJM BSA or palmitate to CCL19 (e) in the presence or absence of Unc5b-Fc or isotype control (Control-Fc) (f). Data are the mean + s.d. of triplicate samples (b,d-f) from a single experiment and are representative of three independent experiments. *P<0.05.

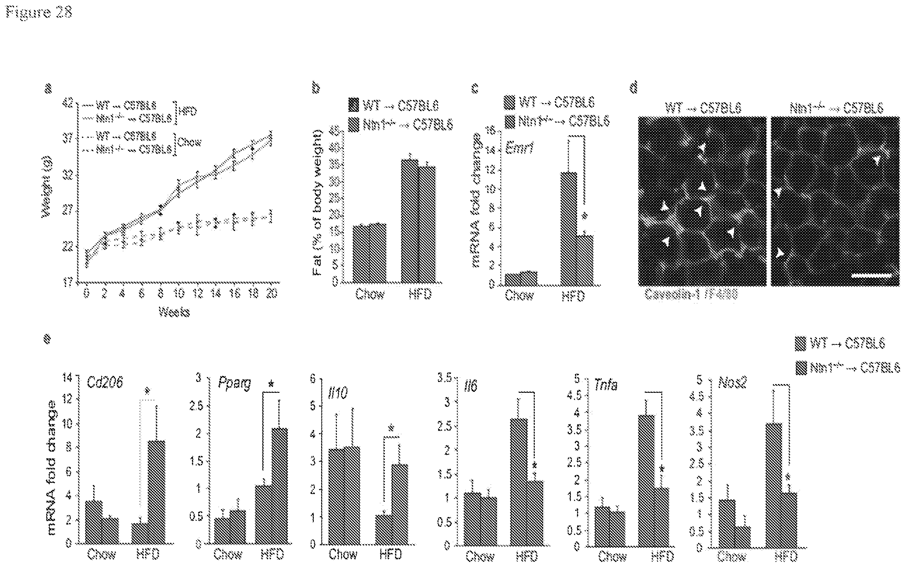

Figure 28 demonstrates that netrin -1 promotes the accumulation of MI macrophages in adipose. C57BL6 mice transplanted with WT or Ntnl-'- bone marrow were fed chow (n=7/group) or HFD (n=9/group) for 20 weeks and body weight (a) and fat mass (b) were measured by dual-energy X-ray absorptiometry (DEXA). (c) qRT-PCR analysis of Emrl (F4180) mRNA in visceral dipose tissue of mice of the indicated genotype fed a chow (n=4) or western diet (n=9). (d) immunofluorescence staining for F4180 in WAT of representative mice from (c). Scale bar = 100 pm. (e) qRT-PCR analysis of mRNA for MI (Nos2 Tnfa, 116) and M2 (Cd206, ILIO, Pparg) markers in macrophages isolated from the WAT of mice of the indicated genotype fed chow (n=7) or HFD (n=9).

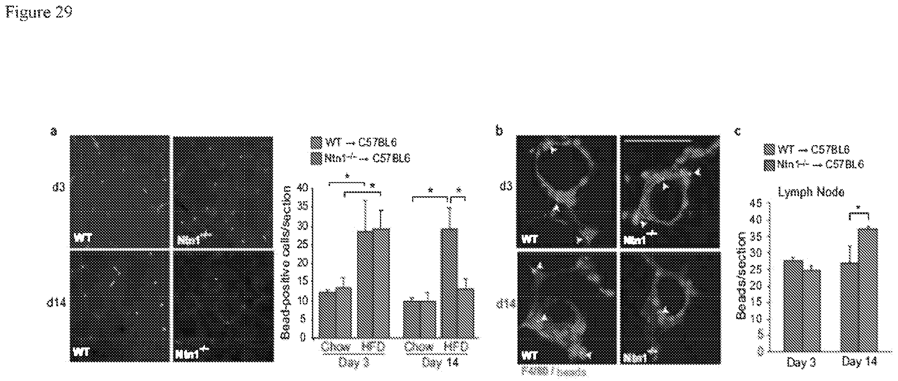

Figure 29 demonstrates that netrin-1 promotes macrophage retention in adipose. (a) Analysis of macrophage recruitment and retention in visceral adipose tissue of mice fed a chow or HFD using a fluorescent bead-tracking model. Left: Representative images of fluorescent bead-labeled cells in WAT of HFD-fed mice at day 3 and 14. Right: Mean number of bead-labeled macrophages in adipose sections 3 days (recruitment) and 14 days (retention) after monocyte labeling (n=5 mice/group). (b) F4/80 staining of adipose tissue from HFD-fed mice 3 and 14 days post-labeling showing green fluorescent beads within macrophages in crown-like structures. (c) Mean number of beads in mesenteric lymph nodes 3 and 14 days after monocyte labeling (n=5 mice/group). Data in a-c are the mean ± s.e.m. *P<0.05.

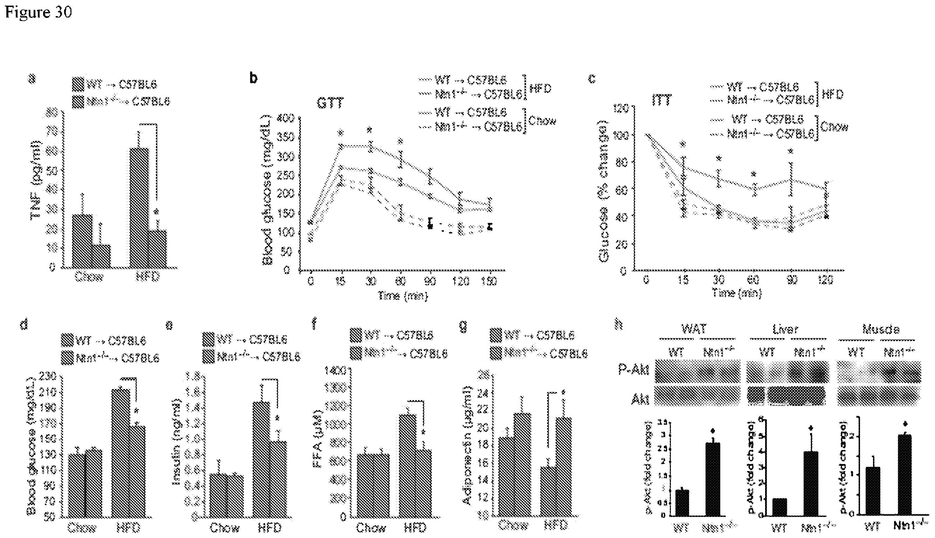

Figure 30 demonstrates that netrin -1 expression by ATM promotes metabolic dysfunction. (a) Serum TNFa levels in C57BL6 mice transplanted with WT or Ntnl-'- bone marrow and fed chow (n=5) or HFD (n=6) for 20 wk. (b) Glucose tolerance test (GTT) and (c) Insulin tolerance test (ITT) of mice of the indicated genotype fed a chow (n=7) or HFD (n=9). (d) Blood glucose levels, and plasma levels of (e) insulin, (f) free fatty acid (FFA), and (g) adiponectin of mice of the indicated genotype fed a chow (n=7) or HFD (n=9). (h) Western blot of phosphoAKT (Ser 473) and total AKT in visceral adipose tissue, liver and muscle. Quantification of p-Aktltotal Akt is shown below (n=4 micelgroup). Data in a-g are the mean k s.e.m. *P<0.05.

### DETAILED DESCRIPTION OF THE INVENTION

Netrin-1 is an axonal guidance protein which acts as a chemorepulsant. Prior studies have indicated that netrin-1 is produced by activated macrophages and may play a role in the pathogenesis of atherosclerotic plaque, among other inflammatory lesions.

The present invention is based in part upon that discovery that it is possible to inhibit inflammation and treat inflammatory diseases by use of agents that either neutralize or block netrin-1 or sema3E (antibodies, peptides, siRNA, etc) or agents which block their respective receptors (unc5b and plexinD1). This approach to therapy is useful for the treatment of atherosclerosis, obesity, diabetes, inflammatory diseases of bone, etc.

Before the present methods and treatment methodology are described, it is to be understood that this invention is not limited to particular methods and experimental conditions described, as such methods and conditions may vary. It is also to be understood that the terminology used herein is for purposes of describing particular instances only, and is not intended to be limiting, since the scope of the present invention will be limited only by the appended claims.

As used in this specification and the appended claims, the singular forms "a", "an", and "the" include plural references unless the context clearly dictates otherwise. Thus, for example, references to "the method" includes one or more methods, and/or steps of the type described herein and/or which will become apparent to those persons skilled in the art upon reading this disclosure and so forth in their entirety.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described.

In accordance with the present invention there may be employed conventional molecular biology, microbiology, and recombinant DNA techniques within the skill of the art. Such techniques are explained fully in the literature. See, e.g., Sambrook et al, "Molecular Cloning: A Laboratory Manual" (1989); "Current Protocols in Molecular Biology" Volumes I-III [Ausubel, R. M., ed. (1994)]; "Cell Biology: A Laboratory Handbook" Volumes I-III [J.E. Celis, ed. (1994))]; "Current Protocols in Immunology" Volumes I-III [Coligan, J.E., ed. (1994)]; "Oligonucleotide Synthesis" (M. J. Gait ed. 1984); "Nucleic Acid Hybridization" [B. D. Hames & S. J. Higgins eds. (1985)]; "Transcription And Translation" [B. D. Hames & S. J. Higgins, eds. (1984)]; "Animal Cell Culture" [R. I. Freshney, ed. (1986)]; "Immobilized Cells And Enzymes" [IRL Press, (1986)]; B. Perbal, "A Practical Guide To Molecular Cloning" (1984).

### Definitions

The terms used herein have the meanings recognized and known to those of skill in the art, however, for convenience and completeness, particular terms and their meanings are set forth below.

"Agent" refers to all materials that may be used to prepare pharmaceutical and diagnostic compositions, or that may be compounds such as small synthetic or naturally derived organic compounds, nucleic acids, polypeptides, antibodies, fragments, isoforms, variants, or other materials that may be used independently for such purposes, all in accordance with the present disclosure.

By "agonist" is meant a substance that binds to a specific receptor and triggers a response in a cell. It mimics the action of an endogenous ligand (such as hormone or neurotransmitter) that binds to the same receptor. A "full agonist" binds (has affinity for) and activates a receptor, displaying full efficacy at that receptor. One example of a drug that acts as a full agonist is isoproterenol which mimics the action of acetylcholine at β adrenoreceptors. A "partial agonist" (such as buspirone, aripiprazole, buprenorphine, or norclozapine) also binds and activates a given receptor, but has only partial efficacy at the receptor relative to a full agonist. A "partial agonist" may also be considered a ligand that displays both agonistic and antagonistic effects - when both a full agonist and partial agonist are present, the partial agonist actually acts as a competitive antagonist, competing with the full agonist for receptor occupancy and producing a net decrease in the receptor activation observed with the full agonist alone. A "co-agonist" works with other co-agonists to produce the desired effect together. An antagonist blocks a receptor from activation by agonists. Receptors can be activated or inactivated either by endogenous (such as hormones and neurotransmitters) or exogenous (such as drugs) agonists and antagonists, resulting in stimulating or inhibiting a biological response. A ligand can concurrently behave as agonist and antagonist at the same receptor, depending on effector pathways.

The potency of an agonist is usually defined by its EC₅₀ value. This can be calculated for a given agonist by determining the concentration of agonist needed to elicit half of the maximum biological response of the agonist. Elucidating an EC₅₀ value is useful for comparing the potency of drugs with similar efficacies producing physiologically similar effects. The lower the EC₅₀, the greater the potency of the agonist and the lower the concentration of drug that is required to elicit a maximum biological response.

"Antagonist" refers to an agent that down-regulates (e.g., suppresses or inhibits) at least one bioactivity of a protein. An "antagonist" or an agent that "antagonizes" may be a compound which inhibits or decreases the interaction between a protein and another molecule, e.g., a target peptide or enzyme substrate. An antagonist may also be a compound that down-regulates expression of a gene or which reduces the amount of expressed protein present. Methods for assessing the ability of an agent to "antagonize" or "inhibit" an axon guidance protein or a receptor thereof are known to those skilled in the art.

"Analog" as used herein, refers to a chemical compound, a nucleotide, a protein, or a polypeptide that possesses similar or identical activity or function(s) as the chemical compounds, nucleotides, proteins or polypeptides having the desired activity and therapeutic effect of the present disclosure (e.g. to treat, inhibit or prevent inflammation), but need not necessarily comprise a compound that is similar or identical to those compounds of the preferred instance, or possess a structure that is similar or identical to the agents disclosed herein.

"Derivative" refers to the chemical modification of molecules, either synthetic organic molecules or proteins, nucleic acids, or any class of small molecules such as fatty acids, or other small molecules that are prepared either synthetically or isolated from a natural source, such as a plant, that retain at least one function of the active parent molecule, but may be structurally different. Chemical modifications may include, for example, replacement of hydrogen by an alkyl, acyl, or amino group. It may also refer to chemically similar compounds which have been chemically altered to increase bioavailability, absorption, or to decrease toxicity. A derivative polypeptide is one modified by glycosylation, pegylation, or any similar process that retains at least one biological or immunological function of the polypeptide from which it was derived.

A "small molecule" refers to a molecule that has a molecular weight of less than 3 kilodaltons (kDa), preferably less than about 1.5 kilodaltons, more preferably less than about 1 kilodalton. Small molecules may be nucleic acids, peptides, polypeptides, peptidomimetics, carbohydrates, lipids or other organic (carbon-containing) or inorganic molecules. As those skilled in the art will appreciate, based on the present description, extensive libraries of chemical and/or biological mixtures, often fungal, bacterial, or algal extracts, may be screened with any of the assays of the disclosure to identify compounds that modulate a bioactivity. A "small organic molecule" is normally an organic compound (or organic compound complexed with an inorganic compound (e.g., metal)) that has a molecular weight of less than 3 kilodaltons, and preferably less than 1.5 kilodaltons, and more preferably less than about 1 kDa.

"Diagnosis" or "screening" refers to diagnosis, prognosis, monitoring, characterizing, selecting patients, including participants in clinical trials, and identifying patients at risk for or having a particular disorder or clinical event or those most likely to respond to a particular therapeutic treatment, or for assessing or monitoring a patient's response to a particular therapeutic treatment.

The concept of "combination therapy" is well exploited in current medical practice. Treatment of a pathology by combining two or more agents that target the same pathogen or biochemical pathway sometimes results in greater efficacy and diminished side effects relative to the use of the therapeutically relevant dose of each agent alone. In some cases, the efficacy of the drug combination is additive (the efficacy of the combination is approximately equal to the sum of the effects of each drug alone), but in other cases the effect can be synergistic (the efficacy of the combination is greater than the sum of the effects of each drug given alone). As used herein, the term "combination therapy" means the two compounds can be delivered in a simultaneous manner, e.g. concurrently, or one of the compounds may be administered first, followed by the second agent, e.g. sequentially. The desired result can be either a subjective relief of one or more symptoms or an objectively identifiable improvement in the recipient of the dosage.

"Modulation" or "modulates" or "modulating" refers to up regulation (i.e., activation or stimulation), down regulation (i.e., inhibition or suppression) of a response, or the two in combination or apart. As used herein, an inflammation "modulator" or "modulating" compound or agent is a compound or agent that modulates at least one biological marker or biological activity characteristic of inflammation.

As used herein, the term "candidate compound" or "test compound" or "agent" or "test agent" refers to any compound or molecule that is to be tested. As used herein, the terms, which are used interchangeably, refer to biological or chemical compounds such as simple or complex organic or inorganic molecules, peptides, proteins, oligonucleotides, polynucleotides, carbohydrates, or lipoproteins. A vast array of compounds can be synthesized, for example oligomers, such as oligopeptides and oligonucleotides, and synthetic organic compounds based on various core structures, and these are also included in the terms noted above. In addition, various natural sources can provide compounds for screening, such as plant or animal extracts, and the like. Compounds can be tested singly or in combination with one another. Agents or candidate compounds can be randomly selected or rationally selected or designed. As used herein, an agent or candidate compound is said to be "randomly selected" when the agent is chosen randomly without considering the specific interaction between the agent and the target compound or site. As used herein, an agent is said to be "rationally selected or designed", when the agent is chosen on a nonrandom basis which takes into account the specific interaction between the agent and the target site and/or the conformation in connection with the agent's action.

"Treatment" or "treating" refers to therapy, prevention and prophylaxis and particularly refers to administering medicine or performing medical procedures on a patient, for either prophylaxis (prevention) or to cure or reduce the extent of or likelihood of occurrence of the infirmity or malady or condition or event.

"Subject" or "patient" refers to a mammal, preferably a human, in need of treatment for a condition, disorder or disease.

An "amount sufficient to inhibit inflammation" refers to the amount of the agent sufficient to block or inhibit the activity of cells involved in or biological markers indicative of an inflammatory response.

In a specific instance, the term "about" means within 20%, preferably within 10%, and more preferably within 5% or even within 1%.

An "effective amount" or a "therapeutically effective amount" is an amount sufficient to decrease or prevent the symptoms associated with the conditions disclosed herein, including atherosclerosis, obesity, diabetes, inflammation, edema, fever, etc. For example, an "effective amount" for therapeutic uses is the amount of the composition comprising an active compound herein required to provide reversal or inhibition of inflammation, etc. Such effective amounts may be determined using routine optimization techniques and are dependent on the particular condition to be treated, the condition of the subject, the route of administration, the formulation, and the judgment of the practitioner and other factors evident to those skilled in the art. The dosage required for the pharmaceutical compositions for use in a method of the invention is that which reduces an inflammatory response or chronic inflammation. The "effective amount" or "therapeutically effective amount" may range from about 1 mg/Kg to about 200 mg/Kg *in vivo,* or more preferentially from about 10 mg/Kg to about 100 mg/Kg, and most preferentially from about 25 mg/Kg to about 50 mg/Kg *in vivo.*

The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the federal or a state government or listed in the U.S. Pharmacopeia or other generally recognized pharmacopeia for use in animals, and more particularly in humans. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin.

Binding compounds can also be characterized by their effect on the activity of the target molecule. Thus, a "low activity" compound has an inhibitory concentration (IC₅₀) (for inhibitors or antagonists) or effective concentration (EC₅₀) (applicable to agonists) of greater than 1 µM under standard conditions. By "very low activity" is meant an IC₅₀ or EC₅₀ of above 100 µM under standard conditions. By "extremely low activity" is meant an IC₅₀ or EC₅₀ of above 1 mM under standard conditions. By "moderate activity" is meant an IC₅₀ or EC₅₀ of 200 nM to 1 µM under standard conditions. By "moderately high activity" is meant an IC₅₀ or EC₅₀ of 1 nM to 200 nM. By "high activity" is meant an IC₅₀ or EC₅₀ of below 1 nM under standard conditions. The IC₅₀ (or EC₅₀) is defined as the concentration of compound at which 50% of the activity of the target molecule (e.g., enzyme or other protein) activity being measured is lost (or gained) relative to activity when no compound is present. Activity can be measured using methods known to those of ordinary skill in the art, e.g., by measuring any detectable product or signal produced by occurrence of an enzymatic reaction, or other activity by a protein being measured.

An individual "at risk" may or may not have detectable disease, and may or may not have displayed detectable disease prior to the treatment methods described herein. "At risk" denotes that an individual who is determined to be more likely to develop a symptom based on conventional risk assessment methods or has one or more risk factors that correlate with development of inflammation including chronic inflammation.

"Prophylactic" or "therapeutic" treatment refers to administration to the host of one or more of the subject compositions. If it is administered prior to clinical manifestation of the unwanted condition (e.g., disease or other unwanted state of the host animal) then the treatment is prophylactic, i.e., it protects the host against developing the unwanted condition, whereas if administered after manifestation of the unwanted condition, the treatment is therapeutic (i.e., it is intended to diminish, ameliorate or maintain the existing unwanted condition or side effects therefrom).

### General Description

### Atherosclerosis

Atherosclerosis is a disease of chronic inflammation that is distinguished by the persistence of cholesterol-engorged macrophages in arterial plaques leading to disease progression and complication (Ross, American Heart Journal, 1999, 138:S419-420). These cells are the central mediators of the establishment, progression and ultimately, instability, of atherosclerotic plaques. Unlike in other tissues, macrophages that accumulate in plaques appear to have a diminished capacity to migrate (Nagao et al., Arterioscler Thromb Vasc Biol., 2007, 27:1596-1602; Park, et al., J Clin Invest., 2009, 119:136-145; Qin et al., Arterioscler Thromb Vasc Biol., 2006, 26:372-378; and Randolph, Curr Opin Lipidol., 2008,19:462-468), and go from being chemotactic to chemostatic, thereby contributing to a failure to resolve the inflammatory process in arteries set in motion by the retention of atherogenic lipoproteins. Although the regulatory signals that impair this process remain largely unknown, netrin-1, a laminin-like molecule normally expressed during embryonic development to guide the movement of neurons, is secreted by macrophage foam cells and inhibits the emigration of these cells from atherosclerotic plaques (van Gils, et al., Nature Immunology, 2012, 13:136-143). In the developing nervous system, netrin-1 can act as either a positive or negative regulator of migration depending on receptor expression by the target cell, and can also serve as a cell survival cue. (Cirulli, et al., Nat Rev Mol Cell Biol., 2007, 8:296-306) Netrin-1 differentially regulates cellular constituents of atheroma: netrin-1 blocks the migration of macrophages expressing the Unc5b receptor to chemokines (CCL2, CCL19) implicated in the egress of macrophages from plaques, while promoting chemoattraction of smooth muscle cells expressing the Neogenin receptor (van Gils, et al., Nature Immunology, 2012, 13:136-143). These combined effects of netrin-1 - macrophage retention and smooth muscle cell recruitment - likely increase plaque size. Notably, targeted deletion of netrin-1 in myeloid cells markedly reduced atherosclerosis burden in *Ldlr*^{-/-} mice and promoted macrophage emigration from plaques. These findings linked netrin-1 in macrophages to the promotion of chronic inflammation, however the mechanisms regulating expression of this guidance cue in atherosclerotic plaques are poorly understood.

The progression of atherosclerotic lesions is closely associated with microenvironmental changes within lipid- and macrophage-rich areas, particularly shifts in oxygen supply, which can lead to hypoxia (Vink, et al., Atherosclerosis, 2007, 195:e69-75). As plaques grow bigger, the oxygen supply is decreased due to both diffusional limitations and the higher demand for oxygen by highly metabolically active cells within the plaque, leading to regions of hypoxia in both human and mouse atheroma. Furthermore, hypoxia has become recognized as a primary impetus of inflammation, and as inflamed lesions often become hypoxic, this further amplifies the inflammatory milieu of the plaque. A recent study reported that netrin-1 is induced by ambient hypoxia in mucosal surfaces of the intestine, where it protects the tissue from transient ischemia by attenuating neutrophil transepithelial migration (Rosenberger, et al., Nature Immunology, 2009,10:195-202). In this setting, epithelial expression of netrin-1 was regulated by the hypoxia-inducible transcription factor (HIF), is a well-known sensor and mediator of hypoxic responses. HIF is a heterodimeric protein consists of 2 subunits: a constitutively expressed HIF-1β subunit, and a HIF-1α or HIF-2α subunit, whose protein levels are regulated by oxygen concentration (Semenza, Cancer, 2003;3:721-732). HIF-1α and -2α are rapidly degraded by the ubiquitin/proteasome pathway in the presence of oxygen, but under hypoxic conditions these proteins translocate to the nucleus, dimerize with HIF-1β, and induce transcription of genes that coordinate cellular adaptions to hypoxia.

In atherosclerosis, HIF-1α is detected in macrophage-rich and hypoxic regions of human and mouse plaques (Sluimer, et al., Journal of the American College of Cardiology, 2008, 51:1258-1265 and Parathath, et al., Circulation Research, 2011, 109:1141-1152). HIF-1α has been reported to regulate a number of gene programs relevant to atherosclerosis, including angiogenesis (Celletti, et al., Nature Medicine,. 2001, 7:425-429), glucose metabolism (Semenza, Cancer, 2003;3:721-732), apoptosis, nitric oxide metabolism, and the inflammatory response (Vink, et al., Atherosclerosis, 2007, 195:e69-75). Furthermore, HIF-1α in the arterial wall may be stabilized under normoxic conditions by inflammation (Celletti, et al., Nature Medicine,. 2001, 7:425-429 and Hellwig-Burgel, et al., Blood, 1999, 94:1561-1567) particularly by inflammatory lipids that accumulate in plaques such as oxidized low density lipoprotein (oxLDL) (Qin et al., Arterioscler Thromb Vasc Biol., 2006, 26:372-378 and Bostrom, et al., Arterioscler Thromb Vasc Biol., 2006, 26:1871-1876). Hypoxic stress may promote netrin-1 expression in macrophages in atherosclerotic plaques, thereby increasing macrophage retention and chronic inflammation. Netrin-1 co-localizes with regions of hypoxia and HIF-1α accumulation in the arterial wall, most prominently in macrophages. *In vitro,* netrin-1 and its receptor Unc5b are induced in macrophages by hypoxic stressors, including oxLDL, and these responses are dependent on HIF-1α. Overexpression of an active form of HIF-1α reduces macrophage migration, and this is reversed by blocking netrin-1, providing a mechanism for macrophage chemostasis during hypoxia. Finally, netrin-1 also promotes the survival of macrophages under hypoxic conditions, which combined with its effects on macrophage retention, would be expected to fuel chronic inflammation in the plaque.

### SEMA

The persistence of myeloid-derived cells in the artery wall is characteristic of advanced atherosclerotic plaques, and these inflammatory cells contribute not only to atherosclerosis progression, but also plaque instability through the secretion of extracellular matrix-degrading proteases and cytotoxic factors. Not surprisingly, then, there has been a major effort to understand macrophage dynamics in the atherosclerotic plaque. Studies examining monocyte recruitment in atherosclerosis have revealed a number of different chemokines and receptors involved in monocyte homing to the arterial wall (Ley, et al., Arterioscler Thromb Vasc Biol., 2011, 31:1506-1516). Perturbations in blood flow at areas of arterial branching, as well as both foreign and endogenous insults (including the retention of atherogenic lipoproteins) to the endothelium, are thought to be responsible for the upregulation of adhesion molecules and chemokines leading to monocyte recruitment and influx into the subendothelial layer of the aortic wall. As atherosclerosis is a chronic, progressive disease, it is thought that the accumulation of monocyte-derived macrophages compounds over time and is a major contributor to the persistence of the disease state (Ye, et al., Am J Pathol., 2011, 178:413-422).

As part of the resolution phase of acute inflammation, activated macrophages normally emigrate from the site of localized inflammation to the draining lymphatics (Bellingan, et al., J Immunol., 1996, 157:2577-2585). However, unlike other acute inflammatory states, atherosclerotic inflammation does not readily resolve and cholesterol-laden macrophages (foam cells) persist in the arterial wall. Macrophages in atherosclerotic plaques appear to be impaired in their ability to emigrate to draining lymph nodes (Llodra, et al., Proc Natl Acad Sci USA, 2004, 101:11779-11784 and Trogan, et al., Proc Natl Acad Sci U S A, 2006, 103:3781-3786). Trapped in an inflammatory milieu, some macrophages die locally, either by apoptosis or secondary necrosis, and the retention of activated macrophages further aggravates plaque inflammation, which can lead to instability and rupture (Ley, et al., Arterioscler Thromb Vasc Biol., 2011, 31:1506-1516). Yet, the mechanisms by which macrophages are retained in the plaque are poorly understood. It has been shown that emigration of macrophages from atherosclerotic lesions can occur under conditions of plaque regression induced either by reducing plasma cholesterol (e.g. (Llodra, et al., Proc Natl Acad Sci U S A, 2004, 101:11779-11784 and Trogan, et al., Proc Natl Acad Sci USA, 2006, 103:3781-3786)) or by increasing levels of high-density lipoprotein (HDL) (eg. (Feig, et al., Proc Natl Acad Sci U S A, 2011, 108:7166 - 7171 and Rayner, et al., J Clin Invest., 2011, 121:2921-2931). Notably, macrophage emigration was largely dependent in a transplant model of atherosclerosis regression on the expression of the chemokine receptor CCR7 (Trogan, et al., Proc Natl Acad Sci U S A, 2006, 103:3781-3786 and Feig, et al., J Clin Invest., 2010, 120:4415-4424), implicating the CCR7-specific ligands CCL19 and CCL21 in promoting the egress of cells from the artery wall in this model.

In addition to chemokine pathways serving to regulate the emigration of macrophages from plaques, there are also factors that are likely to actively inhibit macrophage chemotaxis. A source of potential candidates has come from the realization that the vascular and nervous systems of vertebrates share common features both in their anatomy and in the molecular factors that regulate their development (Melani, et al., Annu Rev Cell Dev Biol., 2010, 26:639-665). Indeed, the role of neuronal guidance molecules in the vasculature has been a recent focus of investigation, and a growing body of evidence demonstrates the participation of classical neuronal guidance molecules in the development of the vascular system (Melani, et al., Annu Rev Cell Dev Biol., 2010, 26:639-665). In addition, emerging evidence is revealing roles for neuronal guidance molecules in the immune system where they appear to exert diverse effects on leukocyte migration, adhesion and inflammatory responses (Perala, et al., Differentiation, 2012, 83:77-91, Sakurai, et al., Mol Cell Biol., 2010, 30:3086-3098 and Cirulli, et al., Nat Rev Mol Cell Biol., 2007, 8:296-306). Notably, the involvement of netrin-1, a neuronal guidance molecule, in promoting chronic inflammation in atherosclerosis by retaining macrophages in plaque was recently reported (van Gils, et al., Nat Immunol., 2012, 13:136-143).

As noted above, the development of a mouse model of atherosclerosis regression involving the transplant of an atherosclerotic aortic arch into a normolipidemic donor has permitted the study of the egress of monocyte-derived cells, primarily macrophages, from aortic arch plaques (Llodra, et al., Proc Natl Acad Sci U S A, 2004, 101:11779-11784; Feig, et al., Proc Natl Acad Sci U S A, 2011, 108:7166 - 7171; Chereshnev, et al., J Surg Res., 2003, 111:171-176 and Feig, et al., PLoS One, 2012, 7:e39790). In addition, the study of gene expression changes specifically in macrophages from atherosclerotic plaques has become possible with the advent of laser capture microdissection (Trogan, et al., Proc Natl Acad Sci USA. 2002;99:2234-2239). Using these tools, the transcriptome changes in progressing and regressing atherosclerotic plaques (Feig, et al., PLoS One, 2012, 7:e39790) were profiled. Of the genes significantly changed during regression of atherosclerotic plaques, Semaphorin 3E (*Sema3e*) was among the most highly downregulated (- 6 fold) compared to that in macrophages in progressing plaque. The Semaphorins are a large family of neuronal guidance cues that have been described as having roles in vascular development and neuroimmune signaling (Nakamura, et al., J Neurobiol., 2000, 44:219-229; Raper, Curr Opin Neurobiol., 2000, 10:88-94; and Steinbach, et al., Exp Cell Res., 2002, 279:52-61). In particular, the class 3 Semaphorins, of which Sema3E is a member, are highly conserved secreted and matrix-associated proteins that can signal through various transmembrane receptors in the Plexin or Neuropilin family, mediating both repulsive and attractive signaling that appears to be cell type and context specific (Puschel, Nat Neurosci., 1999, 2:777-778). Given the finding that netrin-1 expression in atheroma macrophages promotes chronic inflammation, the expression and function of Sema3E in atherosclerosis was investigated to determine whether it had similar effects.

The data clearly show that Sema3E is expressed in macrophages of advanced atherosclerotic plaques in mice, and in vitro potently inhibits migration of macrophages to chemokines implicated in the recruitment of inflammatory macrophages to the draining lymph nodes (e.g., CCL2 and CCL19). Sema3E expression in macrophages *in vitro* is up-regulated by physiological drivers of plaque inflammation, such as oxidized low density lipoprotein (oxLDL) and hypoxia, and reduced under conditions that promote cholesterol efflux. Furthermore, Sema3E is highly expressed in inflammatory M1, but not anti-inflammatory M2 macrophages, and consistent with this is the reduction in expression of Sema3E in regressing atherosclerotic plaques, in which there is also a shift in macrophage phenotype from a predominantly M1 to M2 phenotype. Together, these data demonstrate a role for Sema3E in the retention of macrophages in atherosclerosis and highlight the expanding functions of neuroimmune guidance cues in regulating the persistence of inflammation in atherosclerotic plaques.

### Obesity

There is a clear association between the accumulation of adipose tissue macrophages and insulin resistance in the obese state, yet the factors that sustain chronic inflammation in obesity are not well defined. The present data demonstrate a key role for the neuroimmune guidance cue netrin-1 and its receptor Unc5b in the retention of macrophages in the adipose tissue during high-fat diet feeding. The netrin-1/Unc5b axis is highly upregulated in ATMs of obese mice and humans compared to lean controls. Notably, exposing ATMs from lean mice to netrin-1 or treating macrophages with palmitate to induce netrin-1 secretion impairs their migration to CCL19, a chemokine that directs the emigration of inflammatory macrophages and DCs from tissues to the lymph nodei. ATM isolated from obese mice also exhibit impaired migration to CCLI 9, which is restored by blocking the netrin-1 receptor Unc5b, suggesting that netrin-1 induces ATM chemostasis. Indeed, the selective deletion of hematopoietic netrin-1 in mice fed a HFD reduces ATM accumulation, adipose and systemic inflammation, and insulin resistance. Collectively, these findings suggest that local secretion of netrin-1 by ATM promotes their retention in the adipose tissue, inciting chronic inflammation and metabolic dysfunction.

To understand how netrin-1 promotes ATM accumulation in obesity, a fluorescent bead-based macrophage labeling technique that has been used to monitor the migration of macrophages into and out of atherosclerotic plaques was adapted. (Tacke, et al., Journal of Clinical Investigation, 2007; 117: 185-194; Feig, et al., J Clin Invest, 2010; 120: 4415-4424). *In vivo* macrophage tracking demonstrated that monocyte recruitment is equivalently increased in HFD-fed mice with *WT* and *Ntnl*^{-/-} bone marrow compared to chow-fed mice of each genotype, however the residence time of monocyte-derived macrophages in the adipose tissue was reduced in *Ntnl*^{-/-} chimeric mice. Whereas the bead-labeled ATM persists for 14 days without diminishing in number in the adipose tissue of WT HFD-fed mice, there is a marked decline in bead-labeled ATM in *Ntnl*^{-/-} chimeric mice. Notably, this decline is accompanied by an increase in beads in the mesenteric lymph nodes of *Ntnl*^{-/-} chimeric mice, indicating emigration of ATM from the adipose to the lymph nodes in the absence of netrin-1. These results demonstrate that netrin-1 acts downstream of monocyte chemoattractant signals that induce ATM recruitment, such as CCL2, to promote the local retention of these cells in the adipose tissue during obesity. Indeed, targeted deletion of netrin-1 in hematopoietic cells induces a phenotype similar to that observed in Ccr2^{-/-} mice (Weisberg, et al., J Clin Invest, 2006; 116; 115-124): protection from HFD diet-induced adipose inflammation, and improved systemic glucose homeostasis and insulin sensitivity.

Studies in humans and animal models have shown that caloric restriction, or a switch from a high-fat to a low-fat diet, reduces adipose inflammation (Kovacikova, et al., Int J Obes (Lond), 2011; 35: 91-98; Wang, et al., Atherosclerosis, 2011; 219: 100-108; Jung, et al, Am J Physiol Endocrinol Metab, 2013; 304: E964-976). The signals controlling resolution of inflammation in the adipose tissue remain poorly defined, but are likely to involve reduced recruitment, local macrophage death and efferocytosis, as well as egress of macrophages from the inflammatory site. In other tissues, including the intestine (Diehl, et al., Nature, 2013; 494: 116-120) and atherosclerotic plaques (Feig, et al., J Clin Invest, 2010; 120: 4415-4424; Trogan, et al., Proc Natl Acad Sci USA, 2006; 103: 3781-3786), the chemokine receptor CCR7 has been shown to promote the trafficking of macrophages to the lymph nodes. ATM isolated from obese, but not lean mice, exhibit impaired migration to the CCR7 ligand CCL19, despite equivalent expression of CCR7. Notably, the responsiveness to this chemokine could be restored in obese ATM by inhibiting the netrin-1 receptor Unc5b, indicating that netrin-1 is responsible for this chemostasis. Consistent with this, previous studies have shown that netrin-1 inhibits Rac-mediated re-organization of the actin cyctoskeleton and cell spreading, key steps required for cell migration (van Gils, et al., Nat Immunol, 2012; 13: 136-143). Although the signaling pathways regulating netrin-1 expression in ATM of obese mice and humans *in vivo* require further experimentation, these data demonstrate that palmitate directly induces *Ntnl* and *Unc5b* expression by macrophages, and also upregulates expression of TNFα and IL-6 by neighboring adipocytes, which can in turn induce macrophage *Ntnl* and *Unc5b* expression. These findings are in agreement with previous reports that increased FFA concentrations promote macrophage accumulation in the adipose tissue (Kosteli, et al., J Clin Invest, 2010; 120: 3466-3479). Collectively, these studies demonstrate that netrin-1 secreted by ATM in the setting of obesity acts as a "stop" signal to promote the accrual of these immune cells in the adipose tissue, and the establishment of chronic inflammation.

Collectively, these data demonstrate a model in which netrin-1 secreted by ATM in the setting of obesity acts as a "stop" signal to promote the accrual of these immune cells in the adipose tissue. Macrophage number, as a percent of total cells in the adipose tissue, expands from approximately 10% in lean individuals to more than 50% in advanced obesity. Yet, the underlying cause and functions of these adipose tissue macrophages remain poorly understood. A number of hypotheses regarding the triggers of adipose tissue macrophage accumulation have been put forth, including an increase in adipocyte death in the expanding adipose tissue (Cinti, et al., Journal of lipid research 2005; 46:2347-2355), the release of saturated fatty acids that activate innate immune signaling pathways (Shi et al., The Journal of clinical investigation 2006; 116:3015-3025; Suganami et al., Arteriosclerosis, thrombosis, and vascular biology 2007; 27:84-91), and most recently, the need for macrophages to buffer the increased concentration of lipids released from obese adipocytes in order to protect other tissues from potentially toxic levels of lipid49. These signals, either alone or combined, appear to lead to sustained accumulation of ATM and the expression of inflammatory molecules that impair the metabolic function of the adipose tissue. These data implicate the induction of netrin-1 and Unc5b expression in ATM by adipocyte derived factors, such as saturated fatty acids, in this retention of ATM. Netrin-1 may be turned on as a signal to locally trap ATMs during obesity to support the adipose tissue in buffering increased concentrations of lipids, however, its sustained expression leads to chronic inflammation. Indeed, deletion of hematopoietic expression of netrin-1 in mice decreases ATM accumulation, and local and systemic cytokine expression during HFD-feeding. Moreover, the increased ATM emigration from the adipose tissue in these mice indicates that netrin-1 contributes to the failure to reestablish tissue homeostasis in obesity.

It is notable that netrin-1 is selectively upregulated in the adipose tissue, but not serum, of obese mice and humans. In fact, there is a modest but significant reduction in circulating levels of netrin-1 in obese human patients compared to lean controls, and a similar trend in HFD-fed mice. Although the implications of reduced serum netrin-1 levels in obese individuals are unknown, recent studies suggest that netrin-1 in the circulation may inhibit inflammation by reducing leukocyte recruitment into tissues. Netrin-1 is expressed on vascular endothelium and gut epithelium where its expression is regulated to promote or inhibit leukocyte transmigration into tissues (Rosenberger, et al., Nat Immunol, 2009; 10: 195-202, Wang, et al., Am J Pathol, 2009; 175: 1010-1018; Mirakaj, et al., Am J Respir Crit Care Med, 2010; 181: 815-824; Ly, et al., Proc Natl Acad Sci USA, 2005; 102: 14729-14734) under various conditions. Reduced circulating levels of netrin-1 have been associated with neutrophil infiltration of the kidney during ischemia-reperfusion injury (Tadagavadi, et al., J Immunol, 2010; 1185: 3750-3758; Ranganathan, et al., Kidney Int, 2013; 83: 1087-1098). Furthermore, lentiviral-overexpression of netrin-1 on endothelial cells protects Ldlr^{-/-} mice from atherosclerosis (Khan, et al., Gene Ther, 2010), presumably by reducing leukocyte recruitment into the artery wall. However, netrin-1 has also been shown to be abundantly expressed by macrophage foam cells in atherosclerotic plaques, where its expression promotes the accumulation of macrophages and disease progression (van Gils, et al., Nat Immunol, 2012; 13: 136-143), consistent with findings in obese adipose tissue. Thus, therapeutic targeting of netrin-1 to reduce chronic inflammation in obesity and atherosclerosis might be optimized by macrophage-targeted delivery of netrin-1 or Unc5b inhibitors.

Current strategies aimed at targeting adipose inflammation have so far yielded variable results: blocking TNFα signaling in obese individuals with T2D produced suboptimal outcomes (Dominguez, et al., J Vasc Res, 2005; 43: 517-525), yet clinical trials of IL-1 inhibition appear more promising (Larsen, et al., N Engl J Med, 2007; 356: 1517-1526). However, these therapies directed at blocking cytokines that result from chronic inflammation are likely to be less effective than treatments targeting the source of this problem, i.e. the sustained accumulation of macrophages and other immune cells in adipose tissue. The present data suggests that targeting local factors that promote the retention of macrophages, such as the netrin-1/Unc5b axis, may reverse the local and systemic mediators of inflammation that drive metabolic dysfunction.

### Selecting compounds

Selecting the compounds that interact with or bind to a protein, peptide or receptor or otherwise antagonize or agonize or stimulate the receptor may be performed in multiple ways. The compounds may first be chosen based on their structural and functional characteristics, using one of a number of approaches known in the art. For instance, homology modeling can be used to screen small molecule libraries in order to determine which molecules are candidates to interact with the receptor thereby selecting plausible targets. The compounds to be screened can include both natural and synthetic ligands. Furthermore, any desired compound may be examined for its ability to interact with or bind to the receptor.

Binding to or interaction with an axonal guidance protein or a receptor for the same may be determined by performing an assay such as, for example, a binding assay between a desired compound and a receptor. In one aspect, this is done by contacting said compound to a protein, peptide or receptor and determining its dissociation rate. Numerous possibilities for performing binding assays are well known in the art. The indication of a compound's ability to bind to a protein, peptide or receptor is determined, e.g., by a dissociation rate, and the correlation of binding activity and dissociation rates is well established in the art. For example, the assay may be performed by radio-labeling a reference compound, or other suitable radioactive marker, and incubating it with the cell bearing a receptor. Test compounds are then added to these reactions in increasing concentrations. After optimal incubation, the reference compound and receptor complexes are separated, e.g., with chromatography columns, and evaluated for bound ¹²⁵I-labeled peptide with a gamma (γ) counter. The amount of the test compound necessary to inhibit 50% of the reference compound's binding is determined. These values are then normalized to the concentration of unlabeled reference compound's binding (relative inhibitory concentration (RIC) ⁻¹=concentrationₜₑₛₜ/concentration_{reference}). A small RIC⁻¹ value indicates strong relative binding, whereas a large RIC⁻¹ value indicates weak relative binding. *See,* for example, Latek et al., Proc. Natl. Acad. Sci. USA, 2000, 97(21): 11460-11465. A receptor agonist mimic may be computationally evaluated and designed by means of a series of steps in which chemical groups or fragments are screened and selected for their ability to associate with the individual binding pockets or interface surfaces of the protein. One skilled in the art may employ one of several methods to screen chemical groups or fragments for their ability to associate with the receptor. This process may begin by visual inspection of, for example, the protein/protein interfaces or the binding site on a computer screen based on the available crystal complex coordinates of the receptor, including a protein known to interact with selected fragments or chemical groups may then be positioned in a variety of orientations, or docked, at an individual surface of the receptor that participates in a protein/protein interface or in the binding pocket. Docking may be accomplished using software such as QUANTA and SYBYL, followed by energy minimization and molecular dynamics with standard molecular mechanics forcefields, such as CHARMM and AMBER (AMBER, version 4.0 (Kollman, University of California at San Francisco, copyright, 1994); QUANTA/CHARMM (Molecular Simulations, Inc., Burlington, Mass., copyright, 1994)). Specialized computer programs may also assist in the process of selecting fragments or chemical groups. These include: GRID (Goodford, J. Med. Chem., 1985, 28:849-857), available from Oxford University, Oxford, UK; MCSS (Miranker, et al., Proteins: Structure, Function and Genetics, 1991, 11:29-34), available from Molecular Simulations, Burlington, Mass.; AUTODOCK (Goodsell, et al., Proteins: Structure, Function, and Genetics, 1990, 8:195-202), available from Scripps Research Institute, La Jolla, Calif; and DOCK (Kuntz et al., J. Mol. Biol., 1982, 161:269-288), available from University of California, San Francisco, Calif. Once suitable chemical groups or fragments that bind to an adenosine receptor have been selected, they can be assembled into a single compound or agonist. Assembly may proceed by visual inspection of the relationship of the fragments to each other in the three-dimensional image displayed on a computer screen in relation to the structure coordinates thereof. This would be followed by manual model building using software such as QUANTA or SYBYL. Useful programs to aid one of skill in the art in connecting the individual chemical groups or fragments include: CAVEAT (Bartlett et al., 1989, 'CAVEAT: A Program to Facilitate the Structure-Derived Design of Biologically Active Molecules'. In Molecular Recognition in Chemical and Biological Problems', Special Pub., Royal Chem. Soc. 78:182-196), available from the University of California, Berkeley, Calif; 3D Database systems such as MACCS-3D (MDL Information Systems, San Leandro, Calif.). This area is reviewed in Martin, J. Med. Chem., 1992, 35:2145-2154); and HOOK (available from Molecular Simulations, Burlington, Mass.). Compounds may be designed as a whole or 'de novo' using either an empty binding site or the surface of a protein that participates in protein/protein interactions or optionally including some portion(s) of a known activator(s). These methods include: LUDI (Bohm, J. Comp. Aid. Molec. Design 1992, 6:61-78), available from Molecular Simulations, Inc., San Diego, Calif; LEGEND (Nishibata et al., Tetrahedron, 1991, 47:8985), available from Molecular Simulations, Burlington, Mass.; and LeapFrog (available from Tripos, Inc., St. Louis, Mo.). Other molecular modeling techniques may also be employed in accordance with this invention. See, e.g., Cohen et al., J. Med. Chem., 1990, 33:883-894. See also, Navia, et al., Current Opinions in Structural Biology, 1992, 2:202-210.

Once a compound has been designed by the above methods, the efficiency with which that compound may bind to or interact with the receptor protein may be tested and optimized by computational evaluation. Agonists or antagonists may interact with the receptor in more than one conformation that is similar in overall binding energy. In those cases, the deformation energy of binding is taken to be the difference between the energy of the free compound and the average energy of the conformations observed when the inhibitor binds to the receptor protein.

A compound selected for binding to the receptor may be further computationally optimized so that in its bound state it would preferably lack repulsive electrostatic interaction with the target protein. Such non-complementary electrostatic interactions include repulsive charge-charge, dipole-dipole and charge-dipole interactions. Specifically, the sum of all electrostatic interactions between the compound and the receptor protein when the mimic is bound to it preferably make a neutral or favorable contribution to the enthalpy of binding. Specific computer software is available in the art to evaluate compound deformation energy and electrostatic interaction. Examples of programs designed for such uses include: Gaussian 92, revision C (Frisch, Gaussian, Inc., Pittsburgh, Pa. copyright 1992); AMBER, version 4.0 (Kollman, University of California at San Francisco, copyright 1994); QUANTA/CHARMM (Molecular Simulations, Inc., Burlington, Mass., copyright 1994); and Insight II/Discover (Biosym Technologies Inc., San Diego, Calif., copyright 1994). These programs may be implemented, for instance, using a computer workstation, as are well-known in the art. Other hardware systems and software packages will be known to those skilled in the art.

Once a receptor modulating compound has been optimally designed, for example as described above, substitutions may then be made in some of its atoms or chemical groups in order to improve or modify its binding properties, or its pharmaceutical properties such as stability or toxicity. Generally, initial substitutions are conservative, i.e., the replacement group will have approximately the same size, shape, hydrophobicity and charge as the original group. Substitutions known in the art to alter conformation should be avoided. Such altered chemical compounds may then be analyzed for efficiency of binding to the receptor by the same computer methods described in detail above.

### Candidate Compounds and Agents

Examples of agents, candidate compounds or test compounds include, but are not limited to, nucleic acids (e.g., DNA and RNA), carbohydrates, lipids, proteins, peptides, peptidomimetics, small molecules and other drugs. In one preferred instance, agents can be obtained using any of the numerous suitable approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam, Anticancer Drug Des., 1997, 12:145; U.S. Patent No. 5,738,996 and U.S. Patent No. 5,807,683).

Phage display libraries may be used to screen potential ligands or receptor modulators. Their usefulness lies in the ability to screen, for example, a library displaying a large number of different compounds. For use of phage display libraries in a screening process, see, for instance, Kay et al., Methods, 2001, 240-246. An exemplary scheme for using phage display libraries to identify compounds that bind or interact with a receptor may be described as follows: initially, an aliquot of the library is introduced into microtiter plate wells that have previously been coated with target protein. After incubation (e.g., 2 hours), the nonbinding phage are washed away, and the bound phage are recovered by denaturing or destroying the target with exposure to harsh conditions such as, for instance pH 2, but leaving the phage intact. After transferring the phage to another tube, the conditions are neutralized, followed by infection of bacteria with the phage and production of more phage particles. The amplified phage is then rescreened to complete one cycle of affinity selection. After three or more rounds of screening, the phage is plated out such that there are individual plaques that can be further analyzed. For example, the conformation of binding activity of affinity-purified phage for the receptor may be obtained by performing ELISAs. One skilled in the art can easily perform these experiments. In one instance, a receptor molecule used for any of the assays may be a recombinant receptor protein, or a fusion protein, an analog, derivative, or mimic thereof.

Examples of methods for the synthesis of molecular libraries can be found in the art, for example in: DeWitt et al., Proc. Natl. Acad Sci. USA, 1993, 90:6909; Erb et al., Proc. Natl. Acad Sci. USA, 1994, 91:11422; Zuckermann et al., J Med Chem., 1994, 37:2678; Cho et al., Science, 1993, 261:1303; Carrell et al., Angew. Chem. Int. Ed. Engl., 1994, 33:2059; Carell et al., Angew. Chem. Int. Ed. Engl., 1994, 33:2061; and Gallop et al., J. Med. Chem., 1994, 37:1233.

Libraries of compounds may be presented, e.g., in solution (Houghten, Bio/Techniques, 1992, 13:412-421), or on beads (Lam, Nature, 1991, 354:82-84), chips (Fodor, Nature, 1993, 364:555-556), bacteria (U.S. Pat. No. 5,223,409), spores (U.S. Pat. Nos. 5,571,698; 5,403,484; and 5,223,409), plasmids (Cull et al., Proc. Natl. Acad. Sci. USA 1992, 89:1865-1869) or phage (Scott, et al., Science, 1990, 249:386-390; Devlin, Science, 1990, 249:404-406; Cwirla et al., Proc. Natl. Acad. Sci. USA, 1990, 87:6378-6382; and Felici, J. Mol. Biol., 1991, 222:301-310).

The methods of screening compounds may also include the specific identification or characterization of such compounds, whose effect on inflammation was determined by the methods described above. If the identity of the compound is known from the start of the experiment, no additional assays are needed to determine its identity. However, if the screening for compounds that modulate the protein, peptide or receptor is done with a library of compounds, it may be necessary to perform additional tests to positively identify a compound that satisfies all required conditions of the screening process. There are multiple ways to determine the identity of the compound. One process involves mass spectrometry, for which various methods are available and known to the skilled artisan (e.g. the neogenesis website). Neogenesis' ALIS (automated ligand identification system) spectral search engine and data analysis software allow for a highly specific identification of a ligand structure based on the exact mass of the ligand. One skilled in the art can also readily perform mass spectrometry experiments to determine the identity of the compound.

Antibodies, including polyclonal and monoclonal antibodies, for instance anti-receptor antibodies and neutralizing antibodies may be useful as compounds to inhibit or antagonize an axon guidance protein. These antibodies may be available from such vendors as Upstate Biologicals, Santa Cruz, or they made be prepared using standard procedures for preparation of polyclonal or monoclonal antibodies known to those skilled in the art. Also, antibodies including both polyclonal and monoclonal antibodies, and drugs that modulate the activity of the receptor and/or its subunits may possess certain diagnostic applications and may for example, be utilized for the purpose of detecting and/or measuring conditions such as inflammation. The receptor or its subunits may be used to produce both polyclonal and monoclonal antibodies to themselves in a variety of cellular media, by known techniques such as the hybridoma technique utilizing, for example, fused mouse spleen lymphocytes and myeloma cells. Likewise, small molecules that mimic or act as agonists for the activities of the A_{2A} receptor may be discovered or synthesized, and may be used in diagnostic and/or therapeutic protocols.

### Therapeutic and Prophylactic Compositions and Their Use

Candidates for therapy with the agents identified by the methods described herein are patients either suffering from inflammation, obesity, diabetes, etc. or at risk for doing so.

The invention provides pharmaceutical compositions for use in methods of treatment featuring administering to a subject an effective amount of a pharmaceutical composition of the invention. The compound is preferably substantially purified (e.g., substantially free from substances that limit its effect or produce undesired side-effects).
The subject is preferably an animal, including but not limited to animals such as monkeys, cows, pigs, horses, chickens, cats, dogs, etc., and is preferably a mammal, and most preferably human. In one specific embodiment, a non-human mammal is the subject. In another specific embodiment, a human mammal is the subject. Accordingly, the agents identified by the methods described herein may be formulated as pharmaceutical compositions to be used for prophylaxis or therapeutic use to treat these patients.

Various delivery systems are known and can be used to administer a pharmaceutical composition for use in a method of the invention, e.g., encapsulation in liposomes, microparticles, or microcapsules. Methods of introduction can be enteral or parenteral and include but are not limited to intradermal, intramuscular, intraperitoneal, intravenous, subcutaneous, intranasal, epidural, topical and oral routes. The compounds may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may be desirable to introduce the pharmaceutical compositions for use in a method of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent. In a specific instance, it may be desirable to administer the pharmaceutical compositions for use in a method of the invention locally to the area in need of treatment.

Such compositions comprise a therapeutically effective amount of an agent, and a pharmaceutically acceptable carrier. In a particular instance, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.
W. Martin. Such compositions will contain a therapeutically effective amount of the compound, preferably in purified form, together with a suitable amount of carrier so as to provide the form for proper administration to the subject. The formulation should suit the mode of administration.

In a preferred embodiment, the pharmaceutical composition for use is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

In another embodiment, the pharmaceutical composition for use can be delivered in a vesicle, in particular a liposome (Langer, Science, 1990, 249:1527-1533; Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327)

In yet another embodiment, the pharmaceutical composition for use can be delivered in a controlled or sustained release system. In one embodiment, a pump may be used (see Langer, *supra;* Sefton CRC Crit. Ref Biomed Eng. 1987, 14:201; Buchwald et al., Surgery, 1980, 88:507; Saudek et al., N Engl. J Med 1989, 321:574). In another embodiment, polymeric materials can be used (See, Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger et al., Macromol. Sci. Rev. Macromol. Chem. 1983, 23:61; Levy et al., Science, 1985, 228:190; During et al. Ann. Neurol., 1989, 25:351; Howard et al., J Neurosurg., 1989, 71:105). In yet another embodiment, a controlled release system can be placed in proximity of the therapeutic target, thus requiring only a fraction of the systemic dose (see, e.g., Goodson, in Medical Applications of Controlled Release (1984) supra, vol. 2, pp. 115-138). Other suitable controlled release systems are discussed in the review by Langer, Science, 1990, 249:1527-1533.

The present invention further contemplates therapeutic pharmaceutical compositions for use in practicing the therapeutic methods of this invention. A subject therapeutic pharmaceutical composition includes, in admixture, a pharmaceutically acceptable excipient (carrier).

The present compounds or agents that modulate the axonal guidance protein or its receptor themselves can be used as the sole active agents, or can be used in combination with one or more other active ingredients. In particular, combination therapy using antagonists with one or more other agents is contemplated. These agents are known in the art, and can be selected from anti-inflammatory compounds, bisophosphonates, soluble RANK, for instance.

When contemplating combination therapy with an agent that inhibits or antagonizes an axon guidance protein and one or more of the above-noted agents, it is important to assess clinical safety by methods known to those skilled in the art. Appropriate dose titration may be necessary when certain groups of compounds are contemplated for use together.

The pharmaceutical compositions for use in a method of the invention may be combined for administration with or embedded in polymeric carrier(s), biodegradable or biomimetic matrices or in a scaffold. The carrier, matrix or scaffold may be of any material that will allow the composition to be incorporated and expressed and will be compatible with the addition of cells or in the presence of cells. Preferably, the carrier matrix or scaffold is predominantly non-immunogenic and is biodegradable. Examples of biodegradable materials include, but are not limited to, polyglycolic acid (PGA), polylactic acid (PLA), hyaluronic acid, catgut suture material, gelatin, cellulose, nitrocellulose, collagen, albumin, fibrin, alginate, cotton, or other naturally-occurring biodegradable materials. It may be preferable to sterilize the matrix or scaffold material prior to administration or implantation, e.g., by treating it with ethylene oxide or by gamma irradiation or irradiation with an electron beam. In addition, a number of other materials may be used to form the scaffold or framework structure, including but not limited to: nylon (polyamides), dacron (polyesters), polystyrene, polypropylene, polyacrylates, polyvinyl compounds (e.g., polyvinylchloride), polycarbonate (PVC), polytetrafluorethylene (PTFE, teflon), thermanox (TPX), polymers of hydroxy acids such as polylactic acid (PLA), polyglycolic acid (PGA), and polylactic acid-glycolic acid (PLGA), polyorthoesters, polyanhydrides, polyphosphazenes, and a variety of polyhydroxyalkanoates, and combinations thereof. Matrices suitable include a polymeric mesh or sponge and a polymeric hydrogel. In the preferred instance, the matrix is biodegradable over a time period of less than a year, more preferably less than six months, most preferably over two to ten weeks. The polymer composition, as well as method of manufacture, can be used to determine the rate of degradation. For example, mixing increasing amounts of polylactic acid with polyglycolic acid decreases the degradation time. Meshes of polyglycolic acid that can be used can be obtained commercially, for instance, from surgical supply companies (e.g., Ethicon, N.J.). A hydrogel is defined as a substance formed when an organic polymer (natural or synthetic) is cross-linked via covalent, ionic, or hydrogen bonds to create a three- dimensional open-lattice structure which entraps water molecules to form a gel. In general, these polymers are at least partially soluble in aqueous solutions, such as water, buffered salt solutions, or aqueous alcohol solutions that have charged side groups, or a monovalent ionic salt thereof.

For use in treating animal subjects, the pharmaceutical compositions of the invention can be formulated as pharmaceutical or veterinary compositions. Depending on the subject to be treated, the mode of administration, and the type of treatment desired, e.g., prevention, prophylaxis, therapy; the compositions are formulated in ways consonant with these parameters. A summary of such techniques is found in Remington's Pharmaceutical Sciences, latest edition, Mack Publishing Co., Easton, Pa.

The preparation of therapeutic compositions containing small organic molecules polypeptides, analogs or active fragments as active ingredients is well understood in the art. The pharmaceutical compositions for use in a method of the present invention may be administered parenterally, orally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Formulations may be prepared in a manner suitable for systemic administration or for topical or local administration. Systemic formulations include, but are not limited to those designed for injection (e.g., intramuscular, intravenous or subcutaneous injection) or may be prepared for transdermal, transmucosal, nasal, or oral administration. Such pharmaceutical compositions may be prepared as injectables, either as liquid solutions or suspensions, however, solid forms suitable for solution in, or suspension in, liquid prior to injection can also be prepared. The preparation can also be emulsified. The active therapeutic ingredient is often mixed with excipients which are pharmaceutically acceptable and compatible with the active ingredient. Suitable excipients are, for example, water, saline, dextrose, glycerol, ethanol, or the like and combinations thereof. The formulation will generally include a diluent as well as, in some cases, adjuvants, buffers, preservatives and the like. In addition, if desired, the composition can contain minor amounts of auxiliary substances such as wetting or emulsifying agents, pH buffering agents which enhance the effectiveness of the active ingredient.

A small organic molecule/compound, a polypeptide, an analog or active fragment thereof can be formulated into the therapeutic composition as neutralized pharmaceutically acceptable salt forms. Pharmaceutically acceptable salts include the acid addition salts (formed with the free amino groups of the polypeptide or antibody molecule) and which are formed with inorganic acids such as, for example, hydrochloric or phosphoric acids, or such organic acids as acetic, oxalic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like. For oral administration, the compositions can be administered also in liposomal compositions or as microemulsions. Suitable forms include syrups, capsules, tablets, as is understood in the art.

The pharmaceutical compositions for use in a method of the present invention may also be administered locally to sites in subjects, both human and other vertebrates, such as domestic animals, rodents and livestock, using a variety of techniques known to those skilled in the art. For example, these may include sprays, lotions, gels or other vehicles such as alcohols, polyglycols, esters, oils and silicones.

The administration of the pharmaceutical compositions for use in a method of the present invention may be pharmacokinetically and pharmacodynamically controlled by calibrating various parameters of administration, including the frequency, dosage, duration mode and route of administration.

Variations in the dosage, duration and mode of administration may also be manipulated to produce the activity required.

The therapeutic compositions are conventionally administered in the form of a unit dose, for instance intravenously, as by injection of a unit dose, for example. The term "unit dose" when used in reference to a therapeutic composition of the present invention refers to physically discrete units suitable as unitary dosage for humans, each unit containing a predetermined quantity of active material calculated to produce the desired therapeutic effect in association with the required diluent; i.e., carrier, or vehicle.

The compositions are administered in a manner compatible with the agent selected for treating the subject, the dosage formulation, and in a therapeutically effective amount. If one desires to achieve the desired effect *in vitro,* the effective amounts may range from about 0.1 nM to about 10 µM, more preferably about 0.1 nM to about 5 µM, and most preferably from about 0.1 nM to about 1 nM. The desired effect may refer to the effect of the agent in reducing or inhibiting inflammation, reducing or inhibiting macrophage migration or accumulation, etc. Moreover, the quantity of the agent to be administered depends on the subject to be treated, and degree of inhibition or antagonism of the axon guidance protein desired. Precise amounts of active ingredient required to be administered depend on the judgment of the practitioner and are peculiar to each individual. However, suitable dosages to achieve the desired therapeutic effect *in vivo* may range from about 0.1 mg/kg body weight per day to about 200 mg/kg body weight per day, or from about 1.0 mg/kg body weight per day to about 100 mg/kg body weight per day, preferably about 25 mg/kg body weight per day to about 50 mg/kg body weight per day. In a particular instance, the term "about" means within 20%, preferably within 10%, and more preferably within 5%. The preferred dose will depend on the route of administration. However, dosage levels are highly dependent on the nature of the disease or situation, the condition of the subject, the judgment of the practitioner, and the frequency and mode of administration. If the oral route is employed, the absorption of the substance will be a factor effecting bioavailability. A low absorption will have the effect that in the gastro-intestinal tract higher concentrations, and thus higher dosages, will be necessary. Suitable regimes for initial administration and further administration are also variable, but are typified by an initial administration followed by repeated doses at one or more hour intervals by a subsequent injection or other administration.

Alternatively, continuous intravenous infusion sufficient to maintain desired concentrations, e.g. in the blood, are contemplated. The composition may be administered as a single dose multiple doses or over an established period of time in an infusion.

It will be understood that the appropriate dosage of the substance should suitably be assessed by performing animal model tests, where the effective dose level (e.g., ED₅₀) and the toxic dose level (e.g. TD₅₀) as well as the lethal dose level (e.g. LD₅₀ or LD₁₀) are established in suitable and acceptable animal models. Further, if a substance has proven efficient in such animal tests, controlled clinical trials should be performed.

The pharmaceutical compositions for use in a method of the present invention may be modified or formulated for administration at the site of pathology. Such modification may include, for instance, formulation which facilitate or prolong the half-life of the composition, particularly in the environment. Additionally, such modification may include the formulation of a composition to include a targeting protein or sequence which facilitates or enhances the desired result. In a particular instance, such modification results in the preferential targeting of the compound to the desired locations or cells.

Pharmaceutically acceptable carriers useful in these pharmaceutical compositions include, e.g., ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Sterile injectable forms of the compositions may be aqueous or oleaginous suspensions. The suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

Parenteral formulations may be a single bolus dose, an infusion or a loading bolus dose followed with a maintenance dose. These compositions may be administered once a day or on an "as needed" basis.

The pharmaceutical compositions may be orally administered in any orally acceptable dosage form including, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, the pharmaceutical compositions may be administered in the form of suppositories for rectal administration. These can be prepared by mixing the agent with a suitable non-irritating excipient which is solid at room temperature but liquid at rectal temperature and therefore will melt in the rectum to release the drug. Such materials include cocoa butter, beeswax and polyethylene glycols.

The pharmaceutical compositions for use in a method of this invention may also be administered topically. Topical application can be effected in a rectal suppository formulation (see above) or in a suitable enema formulation. Topically-transdermal patches may also be used.

For topical applications, the pharmaceutical compositions may be formulated in a suitable ointment containing the active component suspended or dissolved in one or more carriers. Carriers for topical administration of the pharmaceutical compositions for use in a methodof this invention include, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutical compositions may be formulated in an ointment such as petrolatum.

The pharmaceutical compositions for use in a method of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The disclosure also relates to a pharmaceutical pack or kit comprising one or more containers filled with one or more of the ingredients of the pharmaceutical compositions. Optionally associated with such container(s) can be a notice in the form prescribed by a governmental agency regulating the manufacture, use or sale of pharmaceuticals or biological products, which notice reflects (a) approval by the agency of manufacture, use or sale for human administration, (b) directions for use, or both.

### Effective Doses

Toxicity and therapeutic efficacy of compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds that exhibit large therapeutic indices are preferred. While compounds that exhibit toxic side effects can be used, care should be taken to design a delivery system that targets such compounds to the site of affected tissue in order to minimize potential damage to unaffected cells and, thereby, reduce side effects.

The data obtained from cell culture assays and animal studies can be used in formulating a dose range for use in humans. The dosage of such compounds lies preferably within a range of circulating concentrations that include the ED₅₀ with little or no toxicity. The dosage can vary within this range depending upon the dosage form employed and the route of administration utilized. For any pharmaceutical composition used in the method of the invention, the therapeutically effective dose can be estimated initially from cell culture assays. A dose can be formulated in animal models to achieve a circulating plasma concentration range that includes the IC₅₀ (i.e., the concentration of the test compound which achieves a half-maximal inhibition of symptoms) as determined in cell culture. Such information can be used to optimize efficacious doses for administration to humans. Plasma levels can be measured by any technique known in the art, for example, by high performance liquid chromatography.

In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each subject's circumstances. Normal dose ranges used for particular therapeutic agents employed for specific diseases can be found in the Physicians' Desk Reference 54th Edition (2000).

### EXAMPLES

The following examples are set forth to provide those of ordinary skill in the art with a description of how to make and use the pharmaceutical compositions for use in a method of the invention, and are not intended to limit the scope thereof. Efforts have been made to insure accuracy of numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### EXAMPLE 1

### Background

Given its role in inhibiting leukocyte migration, it is desirable to determine whether netrin-1 contributes to the retention of macrophages in the chronic inflammatory milieu of the atherosclerotic plaque. The present data shows that netrin-1 is abundantly expressed by macrophage foam cells formed *in vitro* and *in vivo,* and in human atherosclerotic lesions. In functional studies netrin-1 expressed by foam cells differentially regulates cellular constituents of atheroma: netrin-1 inactivates macrophage migration and supports chemoattraction of coronary artery smooth muscle cells. Thus, expression of netrin-1 in plaques likely simultaneously prevents inflammatory cell egress, and induces smooth muscle cell recruitment into the intima, thereby promoting lesion progression. In support of this, deletion of netrin-1 in myeloid cells dramatically reduces atherosclerosis lesion size and complexity in *Ldlr*^{-/-} mice, and is associated with macrophage emigration from plaques.

### Materials and Methods

Mice. C57BL/6J, *Ldlr*^{*-*/*-*} and *Apoe*^{-/-} mice were from Charles River Laboratories or Jackson Laboratories. *Ntn1*^{*+*/*-*} mice were obtained from Dr. Marc Tessier-Lavigne (Stanford University) and were backcrossed 8 generations onto a C57BL/6 background. *Cd36*^{-/-} mice were generated in our laboratory (Moore, et al., J Biol Chem, 2002, 277: 47373-47379). All mice were maintained in a pathogen-free facility. Experimental procedures were performed in accordance with the USDA Animal Welfare Act and the PHS Policy for the Human Care and Use of Laboratory Animals and the Massachusetts General Hospital's and New York University School of Medicine's Subcommittees on Research Animal Care and Use.

Immunohistochemical staining. Human left anterior descending coronary artery segments were obtained from hearts excised at the time of cardiac transplantation, then fixed in neutral buffered formalin and embedded in paraffin. Atherosclerotic plaque morphology was determined from 6-µm sections stained with Movat's pentachrome stains. Macrophages were identified using monoclonal antibody HAM-56 (titer: 1:10, DakoCytomation). Rabbit polyclonal antisera were used to identify netrin-1 (titer: 1:500, Calbiochem) or UNC5b (generated by Dr. T. Bernard Kinane against an extracellular domain: SQAGTDSGSEVLPDS) and normal rabbit serum was used as a negative control. Nova red (Vector Laboratories), which yields a red reaction product, was used as the peroxidase substrate and cell nuclei were counterstained with hematoxylin. Macrophages in mouse atherosclerotic lesion were immunostained using rat anti-macrophage antibody (MOMA-2, AbD Serotec), anti-rat HRP conjugated antibody (AbD Serotec) or control rat IgG2b (AbD Serotec) and DAB Substrate Kit (BD Pharmingen). To detect smooth muscle cells, anti-smooth muscle actin (1A4, AbD Serotec) or mouse IgG2a (DakoCytomation) as a negative control were used together with ARK® animal research kit peroxidase (DakoCytomation) according to manufacturer's instructions. Cell nuclei were counterstained with hematoxylin (Sigma-Aldrich).

Immunofluorescence staining. Mouse atherosclerotic lesions of the aortic sinus and cultured human CaSMC were fixed and staining was performed using anti-netrin-1 (ab26162, Abcam), anti-CD68 (FA-11, AbD Serotec), anti-macrophage (MOMA-2, AbD Serotec), anti-neogenin (C-20, Santa Cruz Biotechnology or ab26299, Abcam), anti-DCC (A-20, Santa Cruz Biotechnology), and anti-smooth muscle actin (ab21027, Abcam or 1A4, AbD Serotec) as described in Supplementary Methods.

Cell culture. RAW 264.7 and HEK293T cells were obtained from ATCC. Peritoneal macrophages were collected from mice by peritoneal lavage 4 days after intraperitoneal injection with 3% thioglycollate as previously described (Moore, et al., J Biol Chem, 2002, 277: 47373-47379). Cells were washed in PBS and used for migration or cultured overnight in DMEM (10% FBS) and stimulated for 6-48 h in reduced serum medium (2% FBS) with 50 µg/ml LDL (Biomedical Technologies Inc) or LDL oxidized as previously described (Kunjathoor, et al., J Biol Chem, 2002, 277: 49982-49988). RNA or protein was isolated as described (Kunjathoor, et al., J Biol Chem, 2002, 277: 49982-49988). Conditioned medium from 4x10⁶ cells were collected in 1 ml and concentrated to 250 µl using a centricon YM-30 (Millipore). Human coronary artery smooth muscle cells (Cambrex Bio Sciences) were cultured in growth factor supplemented SmBM.

RT-qPCR. RNA (0.5-1 µg) was reverse transcribed using a iScript™ cDNA Synthesis Kit (Biorad) according to the manufacturer's instruction. DNA from mouse blood was isolated using ArchivePure™ DNA Kit (5Prime) according to manufacturer's protocol. RT-PCR analysis was conducted using iQ SYBR green Supermix (Biorad) or iTaq Supermix (Biorad) on the iCycler Real-Time Detection System (Biorad). Fold change in mRNA expression was calculated using the comparative cycle method (ΔΔCt). Sequences of primers used are listed in Table 1.

**Table 1. PCR Primer sequences.**

| | **Forward** | **Reverse** |
|---|---|---|
| **Human β-actin** | TGCTATCCCTGTACGCCTCT | CCATCTCTTGCTCGAAGTCC |
| **Human DCC** | CGACCGAGGAGTTCCAGTGATCAAG | GGTTCTTCTGCCAGTGGATTGTTGG |
| **Human Neogenin** | ACCCCAGCCTGTGATTAGTG | TGTGATGGTTCAGAGCTTGC |
| **Human UNC5b** | CAGCCTTAAGGTCAAGGTCTACAGCTC | GTGACTGGATCTTTCAGCTCAAGACC |
| **Mouse ABCA1** | GGTTTGGAGATGGTTATACAATAGTTGT | CCCGGAAACGCAAGTCC |
| **Mouse CD3** | ATCGCCTGGAACACTTTCTGG | GCACGTCAACTCTACACTGGT |
| **Mouse CD11c** | GCAGGAGTGTCCAAAGCAAGA | CGTGTGCTAGGTCTCTGAAGC |
| **Mouse CD68** | TGTCTGATCTTGCTAGGACCG | GAGAGTAACGGCCTTTTTGTGA |
| **Mouse GAPDH** | TGTGAGGGAGATGCTCAGTG | TGTTCCTACGCCCAATGTGT |
| **Mouse LDLR (KO)** | CCATATGCATCCCCAGTCTT | AATCCATCTTGTTCAATGGCCATC |
| **Mouse LDLR (WT)** | CCATATGCATCCCCAGTCTT | GCGATGGATACACTCACTGC |
| **Mouse Netrin-1** | GCGGGTTATTGAGGTCGGTG | CAGCCTGATCCTTGCTCGG |
| **Mouse Neutrophil Elastase** | TTGCCAGGAATTTCGTCATGT | TGTGAGGGAGATGCTCAGTG |
| **Mouse UNC5b** | TGGATCTTTCAGCTCAAGACCCAG | AAGATGGCCAGCTGGAGCCG |
| **Mouse Netrin-1 (Taqman)** | GCAAGCCCTTCCACTACGAC | CGCGAGCTCCATGTTGAATCTGC |
| probe GAGGCCAACGAGTGCG | | |

Analysis of netrin-1 protein. Western blot analysis of netrin-1 was performed using and anti-netrin-1 antibody (158936, R&D Systems) and netrin-1 in cell culture supernatants was detected by ELISA.

Netrin-1 Promoter-Luciferase assay. HEK293T were transfected with a human Netrin-1 promoter luciferase reporter construct (SwitchGear Genomics) using lipofectamine™ 2000 (Invitrogen). After transfection, media was changed and cells were pretreated with the NFκB inhibitor BAY 11-7082 (20 µM) for 1 hour prior stimulation with 100 µg/ml oxLDL or PBS as a control for 24 hours. Luciferase activity was assessed using the Dual-Glo Luciferase Assay System (Promega) and normalized to a constitutively expressed Renilla reporter.

F-actin staining. Freshly isolated peritoneal macrophages were seeded on 8 well LabTek slides (Thermo Scientigfic) for 24 hours or used directly. The cells were pretreated with mouse recombinant netrin-1 (250 ng/ml, R&D Systems) or PBS for 30 minutes and stimulated with either CCL19 (500 ng/ml, R&D Systems) or MCP-1 (10 ng/ml R&D systems) for the indicated times. Macrophages were fixed (4% paraformaldehyde), permeabilized (0.1% Triton X-100) and actin filaments stained with Alexa Fluor 568 or 647 phalloidin (Molecular probes, Invitrogen) at a dilution of 1/40. Cells in suspension were analyzed by flow cytometry (C6 Flow Cytometer, Accuri Cytometers, BD Biosciences). The cells on coverslips were mounted after DAPI staining and vizualized with a Nikon Eclipse microscope and images were captured using the NIS-elements software. Images were background corrected followed by measurement of the average cell area of at least 10 cells per field using the Ivision software.

Migration assays. Chemotaxis of macrophages (pMø, RAW264.7) and CaSMC was measured in 96-well Boyden chamber with a 5 µm (macrophages) or 8 µm pore size filter (CaSMC) (Neuro Probe) as previously described (Ly, et al., Proc Natl Acad Sci USA, 2005, 102: 14729-14734). Macrophage chemotaxis was also quantified using a Real-time Cell Invasion and Migration (RT-CIM) xCelligence assay system with monitoring every 5 min (Roche Applied Science). Cell migration to MCP-1 (100 ng/ml; R&D Systems) or CCL19 (500 ng/ml; R&D Systems) was measured in the absence or presence of recombinant netrin-1 (mouse or chicken from R&D Systems), recombinant rat UNC5b-Fc chimera (cat# 1006-UN, R&D Systems) or whole IgG (Jackson Lab) or conditioned medium from LDL- or oxLDL-stimulated pMø. In some assays, cells were pretreated with netrin-1 or UNC5b blocking antibody (AF1006, R&D Systems) or Neogenin blocking antibody (Santa Cruz, sc-6537) or control goat IgG (Santa Cruz) for 1 hour prior to migration. Following cell migration for 3 or 16 hours (CaSMC and macrophages, respectively), four random high power fields from each well were counted to determine the number of cells that had migrated into the lower chamber. Migration was expressed as chemotactic index, which was calculated by dividing the number of migrating cells in the treated groups by the number of migrating cells in the lowest control well. Results of chemotaxis assays are representative of at least three independent experiments performed on triplicate samples.

*In vivo* studies of inflammatory leukocyte emigration from the peritoneum. As previously described, mice (n=6/group) were injected intraperitoneally with 1 ml of 3% thioglycollate to incite a sterile peritonitis with macrophage numbers peaking on day 4. After 4 days, the mice were pre-treated with netrin-1 (500 ng i.p.) or PBS for 45 min, and then injected with an inflammatory stimulus (400 ng lypopolysaccharide) to induce efflux of leukocytes from the peritoneum to the draining lymph nodes. After 3 hours the peritoneal cells were collected by lavage and counted. The percentage of macrophages in the lavage was quantified by flow cytometry using PE-conjugated anti-F4/80 antibody (CI:A3-1, AbD Serotec).

Fetal liver cell transplantation. Female and male *Ntn1*^{+/-} mice were mated. On day 14 of gestation, embryos were dissected free from the placenta and yolk sac, and a single-cell suspension of fetal liver cell (FLC) was prepared by flushing through graded sizes of needles. Part of the fetal tissues was used for genotyping by X-gal staining intensity, the other part to provide independent confirmation of the genotype by PCR analysis. FLC (2x10⁶) were i.v. injected in lethally irradiated *Ldlr*^{-/-} mice, by 2 exposures of 600 cGy. Four weeks after transplantation, mice were put on a western diet (Teklad Adjusted Calories 88137; 21% (w/w) fat; 0.15% (w/w) cholesterol; 19.5% (w/w) casein, no sodium cholate) for 12 weeks prior to atherosclerosis analysis.

Lipoprotein profile analysis. Pooled plasma from 3 mice per genotype was used to obtain lipoprotein profiles. Profiles were obtained using FLPC gel filtration and Superose 6 column (Amersham Pharmacia), fractions were analyzed for total cholesterol.

Atherosclerotic analysis. After 12 weeks of WD feeding, mice were anesthetized with Tribomoethanol (0.4mg/g i.p.) and exsanguinated by cardiac puncture. Plasma was collected from fasted mice, and total plasma cholesterol and triglyceride levels were measured as described (Moore, et al., J Clin Invest, 2005, 115: 2192-2201). Aortas were flushed with PBS and perfused with 10% neutral buffered formalin. Aortic roots were embedded in OCT medium, and the aortic arch and descending aorta were placed in formalin overnight. En face lesion analysis was performed on dissected aortas pinned open and imaged with a dissecting microscope. Lesion area was quantified using IP Lab Spectrum software version 3.9 (Scanalytics) and expressed as a percentage of the total aortic area or defined regions. For cross-sectional analysis of lesion area in the aortic root, every second section (5 µm thick) throughout the aortic sinus (400 µm) was taken for analysis and quantified using IP Lab Spectrum software. Lesional necrotic areas were analyzed as we previously described (Manning-Tobin, et al., Arterioscler Thromb Vasc Biol, 2009, 29: 19-26), from the average of 6 hematoxylin and eosin stained sections (5 µm thick) per mouse, spaced 25 µm apart. The necrotic area was defined as acellular and anuclear white area and quantified using IP Lab Spectrum software (Manning-Tobin, et al., Arterioscler Thromb Vasc Biol, 2009, 29: 19-26). Apoptotic cells were labeled after proteinase K treatment by TUNEL (TdT-mediated dUTP nick-end labeling) using the *in situ* cell death detection kit TMR-red (Roche Diagnostics). TUNEL and DAPI staining was viewed using a Zeiss Axioplan Inverted fluorescent microscope and only TUNEL-positive cells that colocalized with DAPI-stained nuclei were counted as being positive.

Labeling and tracking of blood monocytes. Circulating blood monocytes of *WT→Ldlr*^{*-*/*-*} and *Ntn1*^{*-*/*-*}→*Ldlr*^{-/-} chimeric mice were labeled in vivo by retro-orbital i.v. injection of 1 µm Fluoresbrite green fluorescent (YG) plain microspheres (Polysciences) diluted 1:4 in sterile PBS as previously described by us and others (Feig, et al., Circulation, 2011, 123: 989-998; Feig, et al., J Clin Invest, 2010, 120: 4415-4424; Feig, et al., Proc Natl Acad Sci U S A, 2011, 108: 7166-7171, and Tacke, et al., The Journal of Clinical Investigation, 2007, 117: 185-194). This method predominantly labels Ly6c^{lo} monocytes and and labeling efficiency (% beads positive blood monocytes) was measured by flow cytometry one day after injection of beads. One group of mice was harvested after 72 hours for baseline measurements, as this time point has previously been shown to have optimal recruitment of labeled monocytes to atherosclerotic plaques and clearance of the labeled monocytes from the blood. A second group of mice was harvested 14 days later to measure the number of labeled macrophages remaining in plaques. The number of beads per section was normalized for the monocyte labeling efficiency by multiplying the mean number of beads per section of mouse A by the % bead positive blood monocytes of all mice/mouse A as previously described. Over time, the bead content of the plaque decreases if the beads left in the same cells that brought them in or if they were transferred to another monocyte-derived cell that then left. In other words, a decrease in bead number indicates that there was emigration of monocyte-derived cells from the plaque.

Statistics. The difference between two groups was analyzed by Student's t-test or by one-way ANOVA followed by the Newman-Keuls multiple comparison test. A P-value of <0.05 was considered significant.

### Supplemental methods.

Western blot analysis. Cells were washed in ice-cold PBS and lysed in ice cold RIPA buffer supplemented with ImM sodium orthovanadate, ImM phenylmethanesulfonylfluoride and 1mg/ml protease inhibitor cocktail (Roche). Fifty micrograms of protein was separated on a SDS-PAGE gel, transferred to a nitrocellulose or PVDF membrane and blocked in 5% BSA in Tris-buffered saline containing 0.1% Tween-20 (TBS-T) for 2 hours at room temperature. The membranes were incubated with 1 µg/ml polyclonal rat anti-mouse netrin-1 (158936, R&D Systems) or 0.1 µg/ml phospho-FAK (Tyr576/577, Cell Signaling) overnight at 4°C. Blots were incubated with goat anti-rat horseradish peroxidase-conjugated secondary antibody (1:10,000 dilution, Sigma-Aldrich) or goat anti-rabbit horseradish peroxidase-conjugated secondary antibody (1:2000 dilution, Sigma-Aldrich) for 1 hour at room temperature and exposed to ECL reagent (Amersham Biosciences). Signal was recorded using Hyperfilm (Amersham Biosciences). After chemiluminescence detection, memberanes were stripped with 0.2 M sodium hydroxide and reprobed with antibodies against α-tubulin (B-5-1-2, Sigma-Aldrich) or FAK (77/FAK, BD Biosciences) for normalization. Densitometry analysis of the gels was carried out using ImageJ software from the NIH.

Netrin-1 ELISA. Detection of netrin-1 by ELISA was performed as previously described with minor modifications. Briefly, 96-well plates (EIA/RIA plate, Corning Incorporated) were coated with 500 ng/well recombinant UNC5b-Fc protein (R&D Systems). Before adding sample, each well was incubated with 350 µl/well of blocking solution, containing 5% (w/v) BSA (Fisher Scientific) in PBS. Standards made with recombinant netrin-1 protein or conditioned medium (250 µl) were added to the coated plates and incubated for 16 hours at 4°C plus 2 hours at 37°C. After three washes with 0.5% BSA in PBS, 100 µl/well rat anti-netrin-1 antibody (1µg/ml in 0.5%BSA in PBS, 158936, R&D Systems) was added to each well and incubated for 30 minutes at 37°C. After extensive washing, each well was incubated with 100 µl/well HRP-conjugated rabbit anti-rat antibody (1:2000 in blocking solution, Sigma-Aldrich) for 30 minutes at 37°C. After very extensive washing the plates were incubated for up to 20 minutes at room temperature with 200 µl/well peroxidase substrate solution, o-Phenylenediamine dihydrochloride (SIGMAFASTTM OPD tablets, Sigma-Aldrich). The reaction was stopped with 50 µl/well 3M HCl and plate read at 492 nm using a multi-well plate reader (SPECTRAmax M5, Molecular Devices).

Rac1 activation assays. Peritoneal macrophages were seeded in a 6-well plate overnight in regular growth media. The cells were stimulated with MCP-1 (10 ng/ml R&D systems) for 5 minutes with or without a pretreatment with mouse recombinant netrin-1 (250 ng/ml, R&D Systems) for 30 minutes. At the end of stimulation the cells were lysed and Rac1-GTP levels were measured using Rac1 Activation Assay (Cell Biolabs) according manufacturer's instructions.

Immunofluorescence. Human CaSMC cultured on glass coverslips were fixed with ice-cold methanol for 4 minutes at -20°C and blocked with 5% FBS in PBS for 20 minutes at room temperature. Thereafter, cells were incubated with the indicated primary antibodies for 1 hour at room temperature goat-anti-human neogenin (1 µg/ml, C-20, Santa Cruz Biotechnology), goat-anti-human DCC (4 µg/ml, A-20, Santa Cruz Biotechnology), or normal goat IgG (Santa Cruz Biotechnology)], followed by incubation with a donkey-anti-goat-IgG conjugated to Alexa 488 (Invitrogen). Cells were mounted in Vectashield® mounting medium with DAPI (Vector Laboratories). Images were taken on a Diaphot TMD inverted microscope (Nikon) with a 63x objective and appropriate filters. Cross-sections of the aortic roots from *Ldlr-*/*-* mice (5µm) were fixed with 100% acetone for 10 minutes and blocked with 4% BSA in PBS. Next, the sections were incubated with the indicated primary antibodies for 2 hours at room temperature or overnight at 4°C [rabbit-anti netrin-1 (0.3 µg/ml, ab26162, Abcam), rat-anti-mouse CD68 nonconjugated or conjugated to Alexa 488 (1:300, FA-11, AbD Serotec), rabbit-anti neogenin (1.6 µg/ml, ab26299, Abcam), goat-anti-smooth muscle actin (2.5 µg/ml, ab21027, Abcam)]. Sections were washed and when necessary incubated with biotinylated anti-rabbit-IgG (BA-1000, Vector Laboratories) for 30 minutes followed by incubation with strepavadin conjugated to Texas Red (Sa-5006, Vector Laboratories), strepavadin conjugated to Fluorescein (Sa-5001, Vector Laboratories), donkey-anti-goat IgG conjugated to Alexa 488 (Invitrogen) or goat-anti-rat IgG conjugated to Alexa 568 (Invitrogen) for 15 minutes. Sections without primary antibody served as negative controls. The sections were mounted using Vectashield medium with DAPI and visualized using either TCS-SP confocal laser scanning microscope (Leica) or Axioscope 2 Plus fluorescence microscope (Carl Zeiss).

### Results

### Macrophage foam cells express the neuroimmune guidance cue netrin-1

Given its role in attenuating leukocyte migration (Ly, et al., Proc Natl Acad Sci USA, 2005, 102: 14729-14734), whether netrin-1 is expressed in atherosclerotic plaques was determined. Immunostaining of serial sections of human coronary artery atherosclerotic plaques showed expression of netrin-1 and its chemorepulsive receptor UNC5H2 (UNC5b) in lesional cells that express the macrophage marker HAM56 (Fig. 1a; Fig 7a). By contrast, DCC, the chemoattractive netrin-1 receptor, was not detected in these plaques (data not shown). A similar pattern of lesional netrin-1 staining was seen in the *Ldlr*^{-/-} mouse model of atherosclerosis (Fig. 1b). In aortic sinus plaques of *Ldlr*^{*-*/*-*} mice fed a western diet (WD) for 12 weeks, double staining for netrin-1 and the macrophage marker CD68 showed netrin-1 expression by lesional macrophages. In addition, there appeared to be extracellular netrin-1 staining in macrophage-rich regions of the plaque, consistent with netrin-1 being a secreted protein that can bind to extracellular matrix components (Brankatschk, et al., Nature Neuroscience, 2006, 9: 188-194 and Yurchenco, et al., Current Opinion in Cell Biology, 2004, 16: 572-579). Analysis of the aortic arch, a second site of lesion predilection in these mice, showed that netrin-1 mRNA is increased in hyperlipidemic *Ldlr*^{*-*/*-*} mice compared to wild type C57BL6 mice, and netrin-1 expression is further upregulated by feeding these mice a WD (Fig. 1c). Similar results were obtained in the *Apoe*^{*-*/*-*} mouse model of atherosclerosis (Fig. 7b), indicating that netrin-1 expression by lesional macrophages is a common characteristic of mouse and human atheroma.

To understand the molecular mechanisms regulating netrin-1 and UNC5b expression, isolated peritoneal macrophages (pMø) from *Ldlr*^{-/-} mice were fed either a chow or WD. In this commonly used model of *in vivo* foam cell formation, WD-feeding markedly increased mRNA expression of *Ntn1* and *Unc5b,* as well as the cholesterol responsive gene *Abca1,* whereas expression of the macrophage marker CD68 was unchanged (Fig. 1d). Similar results were observed in 6 month old *Apoe*^{*-*/*-*} mice (Fig. 1c), which develop hypercholesterolemia and atherosclerotic lesions on a chow diet, suggesting that increased cellular lipid accumulation may upregulate expression of netrin-1 and UNC5b. To test this, mouse peritoneal macrophages (pMø) were treated *in vitro* with native LDL, or LDL that had been oxidized (oxLDL), a modification that promotes cholesterol loading of macrophages. Consistent with *in vivo* data, oxLDL increased macrophage expression of *Ntn1* and *Unc5b* mRNA 5-fold (Fig. 1e), whereas native LDL did not alter expression of these genes (Fig. 7d). Immunoblot analysis confirmed increased cell-associated netrin-1 protein in oxLDL-treated macrophages (Fig. 1f), which was paralleled by an increase in netrin-1 in cell culture supernatants as detected by ELISA (Fig. 1g). Notably, the induction of netrin-1 mRNA (Fig. 1h) and protein (Fig. 7e) by oxLDL required CD36, a scavenger receptor previously implicated in macrophage retention in atherosclerotic plaques (Park, et al., J Clin Invest, 2009, 119: 136-145). Because oxLDL binding to CD36 induces NFkB activation (Janabi, et al., Arterioscler Thromb Vasc Biol, 2000, 20: 1953-1960 and Stewart, et al., Nat Immunol, 2010, 11:155-161) and the netrin-1 promoter contains an NFkB binding site (Rosenberger, et al., Nat Immunol, 2009, 10: 195-202), whether oxLDL-induced NFkB contributes to the upregulation of netrin-1 transcription was determined. Using a netrin1 promoter-luciferase reporter gene, netrin-1 promoter activity is induced by oxLDL and this induction is inhibited by BAY 11-7082, an inhibitor of NFkB (Fig. 1i). Collectively, these data demonstrate that loading of macrophages with cholesterol under physiologic conditions, or by oxidized lipids via CD36 *in vitro,* increases expression of netrin-1 and its receptor UNC5b.

### Netrin-1 blocks the directed migration of macrophages

The impact of netrin-1 on macrophage chemotaxis was determined using both transwell Boyden chambers and a real-time detection method (xCelligence). Using both methods, recombinant netrin-1 potently inhibited the chemotaxis of the macrophage cell line RAW264.7 to MCP-1, but had little effect on macrophage migration in the absence of chemokine (Fig. 2a-b). The inhibitory effects of netrin-1 on chemotaxis were dose dependent (Fig. 2a), with 250 ng/ml netrin-1 inhibiting MCP-1 induced migration by >90% (Fig. 2b). Furthermore, pre-treating RAW264.7 cells with netrin-1 for 1 hour rendered the cells refractory to MCP-1 (Fig 2c). Similar to its effects on RAW264.7 macrophages, netrin-1 potently inhibited chemotaxis of pMø to MCP-1 (Fig 2d). This effect was not chemokine-specific, as netrin-1 also blocked migration of RAW264.7 and pMø to CCL19 and CCL21 (Fig 2e, Fig. 8a-g), chemokines implicated as noted, along with their receptor CCR7, in the egress of CD68+ cells from plaques (Feig, et al., J Clin Invest, 2010, 120: 4415-4424 and Trogan, et al., Proc Natl Acad Sci USA, 2006, 103: 3781-3786). To gain insight into the mechanisms by which netrin-1 inhibits directed chemotaxis of macrophages to multiple chemokines, its effect on organization of the actin cytoskeleton was determined. Stimulation of pMø with MCP-1 or CCL19 induced a marked reorganization of actin, characterized by the appearance of membrane ruffles, lamellipodia and filapodia (Fig. 2f (arrows), Fig. 9a), and rapid cell spreading (Fig. 2g, Fig. 9b). By contrast, pMø pre-treated with Netrin-1 prior to stimulation with MCP-1 or CCL19 maintained a rounded morphology (Fig. 2f (arrowheads), Fig. 9a) and showed no increase in mean cell area (Fig. 2g, Fig. 9b), consistent with a non-motile status. Quantification of phalloidin stained actin filaments by flow cytometry confirmed that pretreating macrophages with netrin-1 blocked the increase in polymerized actin-1 associated with MCP-1 stimulation (Fig. 2h). As the Rho GTPase Rac1 plays a key role in the reorganization of actin in macrophages, whether Netrin-1 altered Rac1 activation following chemotactic stimulation was determined. To measure activated Rac in macrophages treated with MCP-1, we employed GST beads conjugated to the PAK1-PBD to detect the activated GTP-bound form of Rac1. Upon stimulation of macrophages with MCP-1, levels of activated Rac1 were increased approximately 3-fold within 5 minutes (Fig. 2i). Whereas netrin-1 pretreatment of macrophages modestly increased basal levels of activated Rac1, it inhibited further MCP-1-induced Rac1 activation. Netrin-1 also inhibited MCP-1 induced phosphorylation of the focal adhesion kinase FAK (Fig. 2j), which cooperates with Rac1 to link the actin cytoskeleton to the extracellular matrix during cell spreading and migration (Chang, et al., Molecular Biology of the Cell, 2007, 18: 253-264). Collectively, these data indicate that netrin-1 inhibits the directional migration of macrophages by disrupting the Rac1 signaling cascade, re-organization of the actin cytoskeleton and cell polarization.

As both netrin-1 and Unc5b are upregulated in cholesterol-loaded macrophages, netrin-1 secreted by macrophage foam cells in atherosclerotic plaques may act in an autocrine/paracrine manner via Unc5b to immobilize macrophages in the artery wall. To test the role of Unc5b in the response to netrin-1, macrophages were pre-incubated with a recombinant UNC5b-Fc fusion protein or an antibody that binds to the extracellular domain of UNC5b, and their response to CCL19 was measured in the presence and absence of netrin-1. Both the recombinant UNC5b-Fc (Fig. 3a) and anti-Unc5b antibody (Fig. 3b), but not control IgG, reversed the inhibitory effect of netrin-1 on CCL19-induced migration. By contrast, no change was seen with an inhibitor of the A2B adenosine receptor, another netrin-1 receptor implicated in the attenuation of neutrophil migration (Fig. 3c). Furthermore, consistent with the secretion of netrin-1 by macrophage foam cells, conditioned medium from oxLDL-, but not LDL-treated macrophages, inhibited cell migration to CCL19 (Fig. 3d), and this effect was reversed by the addition of a recombinant UNC5b-Fc fusion protein (Fig. 3e). To confirm that netrin-1 is the active component secreted by oxLDL-treated Mø, pMø isolated from *Ntn1*^{*-*/*-*} bone marrow chimeric mice which do not express netrin-1 in response to oxLDL (Fig. 3f) were used. Whereas conditioned medium from oxLDL-treated *Ntn1*^{+/+} pMø inhibits migration of naive macrophages to CCL19 by 80%, conditioned medium from similarly treated *Ntn1*^{*-*/*-*} macrophages reduces migration by only 25% (Fig. 3f). Furthermore, in the presence of recombinant UNC5b-Fc to block the effects of netrin-1, *Ntn1*^{+/+} conditioned medium inhibited migration to a similar extent as *Ntn1*^{*-*/*-*} conditioned medium (≈25%). By contrast, the effects of *Ntn1*^{*-*/*-*} conditioned medium were unchanged by the addition of recombinant UNC5b (Fig. 3f). Together, these data suggest that netrin-1 secreted by cholesterol-laden macrophages promotes their accumulation in atherosclerotic plaques by inhibiting their emigration from this site of inflammation.

Studies in a transplant mouse model of atherosclerosis regression have demonstrated that macrophages in plaques exit via nearby lymphatics upon aggressive cholesterol lowering (Llodra, et al., Proc Natl Acad Sci USA, 2004, 101: 11779-11784). A similar pathway for macrophage clearance has been reported during resolving peritonitis where inflammatory macrophages emigrate to the draining lymphatics of the omentum (Bellingan, et al., J Immunol 1996, 157: 2577-2585 and Bellingan, et al., The Journal of Experimental Medicine, 2002, 196: 1515-1521). Thus, to test the effect of netrin-1 on macrophage emigration *in vivo* a well-characterized thioglycollate-induced peritonitis model was used in which administration of LPS induces the rapid egress of recruited leukocytes from the peritoneum (Park, et al., J Clin Invest, 2009, 119: 136-145). As previously reported, *i.p.* injection of LPS reduced the number of leukocytes in the peritoneum by 75% in control mice (Fig. 3g). By contrast, mice pretreated with netrin-1 (i.p.) 45 min prior to this inflammatory stimulus showed no significant change in peritoneal leukocytes. Flow cytometric analysis of the macrophage marker F4/80 confirmed that macrophages were retained in the peritoneum of netrin-1 pre-treated mice compared to control mice (0.4x10⁶ ± 0.1 x10⁶ control vs. 1.1x10⁶ ±0.3 x10⁶ netrin-1). Collectively, these data demonstrate that netrin-1 blocks macrophage migration both *in vitro* and *in vivo,* and can inhibit the emigration of macrophages from a site of active inflammation. These data demonstrate that netrin-1 status affects the emigration of macrophages from atherosclerotic lesions.

### Netrin-1 is a chemoattractant for smooth muscle cells via the neogenin receptor.

During the progression of atherosclerosis, smooth muscle cells from the underlying medial layer are recruited to the plaque and participate with macrophage foam cells in promoting plaque growth. To investigate whether netrin-1 expression in plaque affects other cellular components of the atherosclerotic lesion, its effect on migration of human coronary artery smooth muscle cells (CaSMC) was measured. Unlike its inhibitory effect on macrophages, netrin-1 dose-dependently induces migration of CaSMC (Fig. 4a). Furthermore, conditioned medium from oxLDL-, but not LDL-, treated macrophages induces migration of CaSMC (Fig. 4b) and consistent with the accumulation of secreted netrin-1 (Fig. 1g), the chemotactic component of the conditioned medium increased with time of oxLDL incubation (Fig. 4b). To understand how Netrin-1 mediates chemoattraction of CaSMC, expression of its known receptors was measured. Whereas CaSMC express low levels of *Dcc* and *Unc5b,* these cells abundantly express the DCC-related receptor neogenin (Fig. 4c-d), as has been reported for vascular smooth muscle cells. Notably, pre-treatment of CaSMC with a blocking antibody to neogenin abrogated the chemoattractive effect of conditioned medium from oxLDL-treated macrophages (Fig. 4e), supporting a role for foam cell derived netrin-1 in inducing SMC migration. Immunohistochemical staining of atherosclerotic plaques of *Ldlr*^{-/-} mice showed colocalization of neogenin with smooth muscle actin (Fig. 4f), but not the macrophage marker CD68 (Fig. 10a). Furthermore, neogenin was expressed in medial smooth muscle cells (arrows), as well as in smooth muscle cells that had migrated into the intima (arrowheads) (Fig. 4f). Together these data indicate that through mechanisms that are distinct from those in macrophages, netrin-1 modulates CaSMC migration and thus may induce accumulation of these cells in the plaque.

### Netrin-1 deficiency in hematopoietic cells reduces atherosclerosis.

Based on the above data, expression of netrin-1 by macrophage foam cells may promote atherosclerotic plaque growth by both blocking macrophage egress and inciting smooth muscle cell recruitment in the intima. Fetal liver cells from day E14 *Ntn1*^{*-*/*-*} or *Ntn1*^{+/+} (WT) pups were used to reconstitute the bone marrow of lethally irradiated *Ldlr*^{*-*/*-*} mice (Babaev, et al., J Clin Invest, 1999, 103: 1697-1705), thereby generating *Ldlr*^{*-*/*-*} mice with either *Ntn1*^{*-*/*-*} or WT macrophages. Four weeks post-transplantation, the chimeric *Ntn1*^{*-*/*-*}→*Ldlr*^{*-*/*-*} and *WT*→*Ldlr*^{*-*/*-*} mice were challenged with a WD for 12 weeks. Analysis *of Ntnl* and *Ldlr* gene expression in both circulating leukocytes and pMø of recipient mice confirmed a complete change in the genotype to the donor types (Table 1, Fig. 10b). There were no differences in serum total cholesterol and triglyceride levels in chimeric *Ntn1*^{*-*/}*⁻→Ldlr*^{*-*/*-*} and *WT→Ldlr*^{*-*/*-*} mice (Table 1), and size-exclusion chromatography showed similar accumulation of cholesterol in VLDL, LDL, and HDL fractions in these mice (Fig. 10c). Despite these similar serum cholesterol profiles, *Ldlr*^{*-*/*-*} mice lacking expression *of Ntnl* in bone marrow derived cells show a dramatic reduction in atherosclerosis (Fig. 5).

**Table 2. Metabolic parameters of WT→Ldlr^{-l-} and Ntn1^{-l-} →7Ldlr^{-l-} chimeric mice fed a Western diet for 12 weeks.**

| | **WT → *Ldlr^{-l-}*** | ***Ntn1^{-l-}* → *Ldlr^{-l-}*** |
|---|---|---|
| **Weight (g)** | 20.7 (±0.9) (n=9) | 20.2 (±1.5) (n=14) |
| **Total cholesterol (mg/dl)** | 716(±195) (n=10) | 738 (±138) (n=14) |
| **Triglycerides (mg/dl)** | 869 (±365) (n=9) | 936 (±588) (n=12) |
| **Reconstitution of leukocytes (%)*** | 99.7 (±0.2) (n=10) | 97.9 (±3.5) (n=10) |

| | | |
|---|---|---|
| * Analyzed by *Ldlr* WT and KO gene expression in blood leukocytes. | | |

Analysis of atherosclerotic lesions in the aorta en face showed that *Ntn1*^{*-*/*-*}→*Ldlr*^{*-*/*-*} mice had a 55% reduction in mean lesion area compared to *WT→Ldlr*^{*-*/*-*} mice (Fig 5a). Regional analysis of lesion distribution in the aorta demonstrated that hematopoietic deficiency of netrin-1 reduced atherosclerosis in both the aortic arch and the descending aorta equally (Fig. 10d). Isolation of mRNA from the aortic arch of the *Ntn1*^{*-*/*-*}→*Ldlr*^{*-*/*-*} mice showed reduced expression of the macrophage marker CD68 compared to *WT→Ldlr*^{*-*/*-*} mice (Fig. 5b), and correspondingly, lower expression of *Ntn1* (Fig. 5c). In addition to reduced macrophage accumulation, *Ntn1*^{*-*/}*⁻ →Ldlr*^{*-*/*-*} mice also had reduced expression of markers for other leukocyte subsets in the aortic arch, including CD3 (T cell) and C11c (dendritic cell) (Fig. 5b). Furthermore, analysis of atherosclerotic plaques in a second anatomical site, the aortic root, showed markedly smaller lesion area in *Ntn1*^{*-*/*-*}→*Ldlr*^{*-*/*-*} mice compared to *WT→Ldlr*^{*-*/*-*} mice (Fig. 5d-f). Quantification of lesion burden by cross-sectional analysis of the aortic root established that this decrease in lesion area in *Ntn1*^{*-*/}*⁻→Ldlr*^{*-*/*-*} mice was consistently present throughout the 400 µm of the aortic root (Fig. 5e).

Histological characterization of the aortic sinus plaques using the Stary method (Stary, et al., Circulation, 1994, 89: 2462-2478) revealed that *WT→Ldlr*^{*-*/*-*} mice had more advanced atherosclerosis, typified by a greater proportion of complex lesions (stage 3-4), whereas *Ntn1*^{*-*/*-*} →*Ldlr*^{*-*/*-*} mice tended to have less progressed (stage 1-3) lesions (Fig. 6a). To further examine lesion composition, immunohistochemical staining of aortic sinus plaques for macrophage and smooth muscle cell markers was performed. This analysis confirmed a reduction in accumulation of both macrophage (MOMA-2, Fig. 6b) and smooth muscle cells (alpha smooth muscle actin, Fig. 6c) in *Ntn1*^{*-*/}*⁻→Ldlr*^{*-*/*-*} compared to *WT→Ldlr*^{*-*/*-*} mice. During the progression of atherosclerosis, macrophage persistence contributes to changes in plaque morphology, particularly the formation of the necrotic core. This critical feature of dangerous plaques arises from the combination of apoptosis of cholesterol-laden macrophages and defective phagocytic clearance of the apoptotic cells by the surrounding macrophages (efferocytosis). Notably, TUNEL staining revealed that plaques in *Ntn1*^{*-*/}*⁻→Ldlr*^{*-*/*-*} mice contained significantly fewer apoptotic cells compared to *WT→Ldlr*^{*-*/*-*} mice (Fig. 6d) and this correlated with a 46% decrease in necrotic area in *Ntn1*^{*-*/*-*}→*Ldlr*^{*-*/*-*} plaques (Fig. 6e). Together these data suggest that netrin-1 expression by lesional macrophages promotes atherosclerotic plaque growth and complexity by enhancing macrophage retention in the intima.

To test whether netrin-1 expression in plaques inhibits macrophage emigration, a macrophage tracking technique was used in which monocytes can be selectively labeled *in vivo* with fluorescent beads so that their movement into (Tacke, et al., The Journal of Clinical Investigation, 2007, 117: 185-194) and out of (Feig, et al., Proc Natl Acad Sci USA, 2011, 108: 7166-7171) plaques can be tracked in mice. *WT→Ldlr*^{*-*/*-*} and *Ntn1*^{*-*/}*⁻→Ldlr*^{*-*/*-*} mice were harvested 72 hours after labeled bead injection, a time point previously shown to have peak recruitment of labeled cells to atherosclerotic plaque ("baseline") (Tacke, et al., The Journal of Clinical Investigation, 2007, 117: 185-194), and after 14 days to measure the number of labeled macrophages remaining in plaques. As previously shown in progressing plaques (Tacke, et al., The Journal of Clinical Investigation, 2007, 117: 185-194), the number of bead-labeled macrophages in *WT→Ldlr*^{*-*/*-*} plaques was similar at baseline and 14 days, indicating that macrophages do not emigrate from plaques in which netrin-1 is expressed (Fig. 6f). By contrast, in *Ntn1*^{*-*/}*⁻→Ldlr*^{*-*/*-*} plaques there is a 40% decrease in beads after 14 days compared to baseline, indicating that fewer macrophages are retained in the plaque in the absence of netrin-1. As the beads cannot be degraded, and any labeled macrophages undergoing apoptosis are phagocytosed by surrounding macrophages thereby transferring the bead label, the reduction in beads in the *Ntn1*^{*-*/}*⁻→Ldlr*^{*-*/*-*} mice is indicative of macrophage movement out of the plaque. Collectively, these data support the retention of macrophages in plaques by netrin-1 and demonstrate that upon the removal of this retention signal, macrophages emigrate from this site of chronic inflammation.

### Discussion

Plaques that cause clinical events, so called "vulnerable plaques", have a high macrophage content. Thus, strategies targeted at reducing macrophage accumulation and/or directing emigration of these cells from the plaque offer promise as a complement to standard lipid lowering therapies. However to date, the signals responsible for mediating macrophage retention in the artery wall have been poorly understood. These data establish that netrin-1, a neuronal guidance molecule with recently ascribed immunomodulatory functions, and its receptor UNC5b, are expressed by macrophage foam cells of human and mouse atherosclerotic plaques. Notably, recombinant netrin-1 and netrin-1 secreted by *in vitro* formed foam cells potently block the directed migration of macrophages to CCL19, a chemokine implicated in the emigration of monocyte-derived cells from plaques in a transplant model of regression (Feig, et al., J Clin Invest, 2010, 120: 4415-4424 and Trogan, et al., Proc Natl Acad Sci U S A, 2006, 103: 3781-3786). Moreover, netrin-1 also blocked migration of macrophages to MCP-1, which along with its receptor CCR2, has been shown to play roles in myeloid cell trafficking to the lymph node during inflammation (Sato, et al., The Journal of Experimental Medicine, 2000, 192: 205-218 and Jimenez, et al., Journal of Immunology, 2010, 184: 5571-5581). Thus, netrin-1 in plaques may act to immobilize macrophage foam cells and prevent their egress to the blood stream or lymphatic system. The selective deletion of netrin-1 in bone marrow derived cells markedly reduces atherosclerotic lesion size and complexity in *Ldlr*^{*-*/*-*} mice, and is associated with macrophage emigration from plaques. These data establish a causative role for netrin-1 in the persistence of inflammation in atherosclerosis, and highlight the importance of such negative regulators of leukocyte migration in chronic inflammation.

There have been an increasing number of reports that guidance molecules characterized in the developing nervous system can modulate leukocyte migration in inflammatory states (Ly, et al., Proc Natl Acad Sci USA, 2005, 102: 14729-14734; Delaire, et al., J Immunol, 2001, 166: 4348-4354; Munoz, et al., J Immunol, 2002, 169: 177-184 and Wu, et al., Nature, 2001, 410: 948-952). Such studies have shown that members of the netrin, slit, semaphorin and ephrin families of guidance cues can have both chemoattractive and chemorepulsive effects on leukocyte trafficking. Recent studies have shown that netrin-1 is expressed on endothelial and epithelial cells, where it appears to function in limiting leukocyte transmigration into tissues (Ly, et al., Proc Natl Acad Sci USA, 2005, 102: 14729-14734; Mirakaj, et al., Am J Respir Crit Care Med, 2010, 181: 815-824; Rosenberger, et al., Nat Immunol, 2009, 10: 195-202 and Wang, et al., Am J Pathol, 2009, 175: 1010-1018). For example, endothelial expression of netrin-1 is downregulated in the lung during acute *Staphylococcus aureus* induced inflammation, coincident with recruitment of neutrophils to the tissue (Ly, et al., Proc Natl Acad Sci USA, 2005, 102: 14729-14734). By contrast, netrin-1 is upregulated on gut epithelial cells during transient ischemia and attenuates neutrophil recruitment to protect the tissue from hypoxia-induced inflammation (Rosenberger, et al., Nat Immunol, 2009, 10: 195-202). These studies showing that netrin-1 inhibits migration of circulating leukocytes into tissues, and thus is anti-inflammatory in this capacity, have provided insight into the homeostatic barrier functions performed by netrin-1. In this regard, a recent study reported that intravenous viral delivery of human netrin-1 to *Ldlr*^{*-*/*-*} mice reduced atherosclerosis (Khan, et al., Gene Ther, 2010), presumably by increasing its expression on endothelial cells although no determination of the cell types expressing netrin-1 were made. These data indicate that the expression of netrin-1 by macrophage foam cells within plaques is pro-atherosclerotic, and its inactivation of macrophage emigration from this inflamed site likely inhibits the resolution of inflammation. Thus, as in the nervous system where netrin-1 can have both positive and negative effects on axonal migration, it may play multifunctional roles in regulating inflammation depending on the site of its expression (i.e. plaque vs. endothelium).

While expressed at low levels in monocytes and most tissue macrophages, netrin-1 is highly expressed by macrophage foam cells in human and mouse atheroma. The mechanisms of netrin-1 upregulation in this context may be similar to that seen in hypoxia, where HIF1-α and NFκB regulate netrin-1 transcription (Mirakaj, et al., Am J Respir Crit Care Med, 2010, 181: 815-824; Rosenberger, et al., Nat Immunol, 2009, 10: 195-202 and Paradisi, et al., Gastroenterology, 2008, 135: 1248-1257). Hypoxic stress is intimately linked to atherosclerosis (Parathath, et al., Circulation Research, 2011, 109: 1141-1152) and these transcription factors are activated in lesional macrophage (Brand, et al., The Journal of Clinical Investigation, 1996, 97: 1715-1722 and Sluimer, et al., Journal of the American College of Cardiology, 2008, 51:1258-1265), and in macrophages incubated with oxLDL *in vitro.* Induction of netrin-1 by oxLDL involves activation of NF-κB via CD36 (Janabi, et al., Arterioscler Thromb Vasc Biol, 2000, 20: 1953-1960 and Stewart, et al., Nat Immunol, 2010, 11:155-161), a scavenger receptor previously implicated in the retention of macrophages in atherosclerosis (Park, et al., J Clin Invest, 2009, 119: 136-145 and Curtiss, N Engl J Med, 2009, 360:1144-1146). Notably, macrophage expression of UNC5b is also increased by oxLDL, and thus netrin-1 secreted by macrophage foam cells in atherosclerotic plaques would be predicted to act in an autocrine/paracrine fashion via this receptor to immobilize these cells in the artery wall. Macrophages that infiltrate the arterial intima are thought to be cleared by at least three mechanisms: (1) exit via lymphatic vessels, (2) egress into the bloodstream and (3) death in situ and efferocytotic clearance by phagocytes (Gerrity, Am J Pathol, 1981, 103: 191-200; Kling, et al., Atherosclerosis, 1993, 101: 79-96 and Landers, et al., Virchows Arch, 1994, 425: 49-54). All of these mechanisms for macrophage clearance appear to be impaired in atherosclerosis, leading to the progressive accumulation of macrophages in plaques. However, recent studies in mouse models of atherosclerosis suggest that these processes can be restored with aggressive lipid management, providing hope that atherosclerosis regression can be achieved in humans (Williams, et al., Cardiovascular Medicine, 2008, 5: 91-102). Genetic manipulations that reduced plasma non-HDL cholesterol and/or increased the levels of HDL cholesterol, promoted macrophage emigration from plaques to regional and systemic lymph nodes (Rong, et al., Circulation, 2001, 104: 2447-2452; Llodra, et al., Proc Natl Acad Sci USA, 2004, 101: 11779-11784; Feig, et al., Circulation, 2011, 123: 989-998; Feig, et al., J Clin Invest, 2010, 120: 4415-4424 and Trogan, et al., Proc Natl Acad Sci USA, 2006, 103: 3781-3786). This process was characterized by lipid unloading of the macrophages, and required expression of CCR7, the receptor for CCL19/CCL21 (Feig, et al., J Clin Invest, 2010, 120: 4415-4424 and Trogan, et al., Proc Natl Acad Sci USA, 2006, 103: 3781-3786). Notably, both recombinant and foam cell secreted netrin-1 block macrophage chemotaxis to CCL19 and CCL21, and would therefore be expected to inhibit the normal migratory processes that bring about resolution of inflammation, resulting in macrophage trapping. Consistent with this, deletion of netrin-1 in lesional macrophages in *Ldlr*^{*-*/*-*} mice results in markedly smaller lesions and evidence of macrophage emigration from the plaques. Although the exact means by which netrin-1 inhibits migration remains to be elucidated, these data indicate that netrin-1 inhibits Rac-mediated re-organization of the actin cyctoskeleton, preventing cell spreading and impairing the migration of trapped macrophages out of plaques.

In addition to its effects on macrophages, netrin-1 acts as a chemo-attractant for coronary artery smooth muscle cells. Both recombinant netrin-1 and conditioned medium cholesterol loaded macrophages formed *in vitro* are potent chemoattractants for CaSMC, a process dependent on the neogenin receptor. These findings are in agreement with recent studies that showed that netrin-1 promotes angiogenesis by coordinate regulation of endothelial and vascular smooth muscle cells (Park, et al., PNAS USA, 2004, 101: 16210-16215). During plaque progression, the internal elastic lamina can become breached and smooth muscle cells migrate into the intima to participate in lesion growth. Thus, the recruitment of smooth cells by netrin-1 secreted by macrophage foam cells represents a second mechanism by which this guidance molecule could promote atherosclerosis. In plaques of *Ntn1*^{*-*/}*⁻→Ldlr* ^{*-*/*-*} chimeric mice, there is a reduction in the accumulation of both macrophages and smooth muscle cells, consistent with the notion that loss of netrin-1 allows macrophages to emigrate *from* the plaque, and curtails the recruitment of SMC *into* it. Furthermore, *Ntn1*^{*-*/}*⁻→Ldlr* ^{*-*/*-*} plaques show fewer apoptotic cells and reduced necrotic area indicating that the mechanisms regulating tissue homeostasis are enhanced in the absence of netrin-1. Together these data underscore the important role that negative guidance cues play in the persistence of inflammation, and indicate that local inhibition of such factors provides therapeutic value for the resolution of inflammation in atherosclerosis, and other chronic inflammatory diseases.

### EXAMPLE 2

### Materials and Methods

### Reagents

OxLDL was prepared as described by first dializing LDL (0.25 mg/ml) in PBS for 24 hours at 4°C and then incubating with 5bµM CuSO₄ for 6 hours at 37°C (Kunjathoor et al., J Biol Chem. 2002; 277:49982-49988). To stop the reaction, 0.25bmM EDTA and 50µM BHT were added. Cobalt chloride (CoCl₂) and dimethyloxalylglycine (DMOG) were from Sigma Aldrich (15862; D3695). The NFκB inhibitor Bay11-7082 and HIF-1α inhibitor (400083) were from Calbiochem.

### Cell culture

Primary bone marrow derived macrophage (BMDM) were prepared by flushing the bone marrow of the tibia and femur of 6-8 week old C57/B6 mice as described (The cells were grown in DMEM supplemented with 15% L929-conditioned media, 10% FBS and 1% penicillin/streptomycin for 7 days and then treated with oxLDL or subjected to hypoxic conditions. Hypoxic incubations were carried out at 37°C in a Billups-Rothenberg modular incubator chamber at 1% O₂, 5% CO₂ and 94% N₂ for 24 hours. J774 control (J774con) and HIF-1α overexpressing (J774Hif) macrophages (Parathath et al., Circ. Res. 2011;109:1141-1152) were grown in 5.5 mmol/L glucose containing DMEM supplemented with 15% FBS and 1% penicillin/streptomycin.

### Quantitative RT-PCR

Total RNA was isolated using TRIZOL reagent and RNA (0.5-1 mg) was reverse transcribed with an iScript cDNA Synthesis kit according to the manufacturer's instructions (Bio-Rad). RT-PCR analysis was performed using iQ SYBR green Supermix (Biorad) and a Mastercycler Realplex (Eppendorf) as we described (van Gils et al., Nat Immunol., 2012; 13:136-143). The change in mRNA expression was calculated by the comparative change-in-cycle-method (DDCT) relative to GAPDH mRNA levels. The following primer sets were used; Netrin 5'CAGCCTGATCCTTGCTCGG3',3'GCGGGTTATTGAGGTCGGTG5', Unc5b 5'CTGGGGACCGGGAAAGAAC3', 3'CTGATGGGTAGGAGTCTGGG5', Vegf 5'CTCCGCTCTGAACAAGGCT3', 3'GCACATAGAGAGAATGAGCTTCC5' GAPDH 5'AGGTCGGTGTGAACGGATTTG3',
3'TGTAGACCATGTAGTTGAGGTCA5'
RT2 Custom Profiler PCR Arrays (Qiagen) were obtained for neuronal guidance molecules and their receptors. 1 µg of total RNA was reverse transcribed and quantitative RT-PCR analysis was performed according to manufacturer's protocol. Data analysis was performed using the manufacturer's integrated web-based software package of the PCR Array System using ΔΔCt based fold-change calculations.

### Immunoflurescent staining

J774con or J774Hif were plated on coverslips and fixed in 4% paraformaldehyde and permeabilized with 0.1% TritonX100. Non-specific binding was blocked with 5% BSA for 30 minutes and then coverslips were incubated with anti-HIF-1alpha (Novus Biologicla, NB100-479) and anti-netrin chicken polyclonal (Abcam, ab39370), or rabbit polyclonal anti-unc5b antibody (Abcam, ab23393). Secondary fluorescent antibodies used were AlexaFluor 568-anti-rabbit, Alexafluor 488-anti-chicken antibody, or Alexafluor 488-anti-rabbit antibody (Molecular Probes, A11011, A11039, A11008) respectively. Experimental negative controls were incubated in PBS containing isotype matched control antibodies (Santa Cruz Biotechnology). Cells were visualized under x40 using a Nikon Eclipse microscope

### Atherosclerosis

C57BL/6 and *Ldlr*^{*-*/*-*} mice were from Charles River Laboratories and maintained in a pathogen-free facility. Experimental procedures were performed in accordance with the USDA Animal Welfare Act and the PHS Policy for the Human Care and Use of Laboratory Animals and New York University School of Medicine's Subcommittees on Research Animal Care and Use. Eight-week old *Ldlr*^{*-*/*-*} mice were fed a western diet for 12 weeks. Mice were injected prior to sacrifice with a hypoxia probe (hpi, Hypoxiaprobe, Inc) at a dose of 60mg/kg *i.p.* Mice were anesthetized with tribomoethanol (0.4 mg/g *i.p.*) and ex-sanguinated by cardiac puncture. Aortas were flushed with PBS and perfused with 10% sucrose. Aortic roots were embedded in OCT medium, snap frozen and cross-sections (7 µm thick) were taken for analysis. The use of human endarectomy carotid tissue was approved by the University of New York Human subjects review committee. For immunofluorescent staining, sections were fixed with ice cold acetone for 10 minutes and non-specific binding sites were blocked with 5% BSA for 30 minutes at room temperature. Thereafter, either anti-pimonidazole antibody (clone 4.3.11.3, MAb1) or goat anti-netrin1 antibody (R&D, AF 1109), or rabbit polyclonal anti-Unc5b antibody (Abcam, ab23393) or anti-CD68 (lifespan biosciences, LS-C33253) were applied to the sections. After PBS washes, sections were incubated with anti-mouse biotin followed by texas red-conjugated streptavidin to detect the hypoxia probe. Alexafluor 568-coupled to either anti-goat or anti-rabbit or anti-rat antibody (Molecular Probes, A11079, A11011, A11006), were applied for one hour each at room temperature. The segments were stained with DAPI to detect nuclei and mounted with Dako fluorescent mounting medium (Dako, S3023). The slides were visualized under Nikon Eclipse microscope and images captured under x20 objective.

### Promoter luciferase assays

Human NTN1 or UNC5B promoter-luciferase reporter plasmids (Switchgear Genomics) were transfected into HEK293T using lipofectamineTM 2000 (11668-019, Invitrogen). After transfection, media was changed and cells were treated with oxLDL (50 µg/ml) or CoCl₂ (0.1 mM), or DMOG (1 mmol/l) for 24 hours, in the presence or absence (PBS control) of inhibitors of NFκB (10 µmol/L Bay11-7082) or HIF1α (100 µmol/L HIF-1α inhibitor). Luciferase activity was measured using the DUAL-Glo Luciferase Assay system (E1910, Promega) and normalized to a constitutively expressed renilla reporter or to protein concentration.

### Migration assays

Macrophage chemotaxis was measured using the Real-Time Cell Invasion and Migration xCelligence Assay System with monitoring every 5 min (Roche Applied Science). BMDM migration towards MCP-1 (100 ng/ml; R&D Systems) was measured in the presence or absence of oxLDL (50µg/ml) and/or recombinant rat UNC5b-Fc chimera (1006-UN; R&D Systems) to block the effects of netrin-1. In a subset of experiments, cells were pre-treated with 0.1 mmol/L CoCl₂ for 1 h prior to migration. J774 macrophage migration was carried out in the presence or absence of UNC5b-Fc chimera. Results of chemotaxis assays are representative of at least three independent experiments performed on triplicate samples.

### Apoptotic assays

To induce apoptosis, BMDMs were grown in the absence of L929 conditioned media (CM) for 3 days under normoxic or hypoxic conditions. In some assays, cells were treated with 100 µmol/L HIF-1α inhibitor or Unc5b-FC fragment or control IgG. After treatment, apoptotic cells were labeled with PE-active caspase-3 (557091, BD pharmingen) or by the TUNEL method using an *in situ* cell detection kit (Roche Diagnostics). Active Caspase-3, TUNEL and DAPI staining were analyzed with a Nikon Eclipse fluorescent microscope. Only TUNEL positive cells that colocalized with DAPI stained nuclei were considered apoptotic.

### Statistical analysis

The difference between two groups was analyzed by Student's *t*-test. Data are mean ± sd. of triplicate samples in a single experiment and are representative of an experimental n of 3. *P* values of less than <0.05 were considered significant.

### Results

### Hypoxia correlates with Ntnl expression in atherosclerotic plaques

As netrin-1 expression has been shown to be upregulated in models of transient ischemia, whether netrin-1 is expressed in hypoxic regions of atherosclerotic plaques was determined. Immunofluorescence staining of aortic sinus plaques from *Ldlr*^{*-*/*-*} mice fed a western diet (WD) for 12 weeks and injected with a hypoxyprobe (pimonidazole hydrochloride) was performed. Using double immunostaining on serial sections, the macrophage marker cluster of differentiation 68 (CD68) co-localized with the hypoxia probe (Fig. 11a) was noted. Furthermore, netrin-1 was prominently expressed in these regions of the plaque and showed significant co-localization with the hypoxyprobe. To determine whether netrin-1 is also expressed in hypoxic regions of human atheroma, human carotid endarteroctomy sections were stained for netrin-1 and hypoxia inducible factor 1a (HIF-1α), a transcription factor that is stabilized under hypoxic conditions. As previously reported, HIF-1α was prominently expressed in the central region of human plaques, in areas maximally distant from the blood supply (Fig. 11b). Notably, staining for Netrin-1 co-localized with HIF-1α-positive cells in the plaque (Fig. 11b).

### HIF-1α-mediated induction of Ntnl in macrophages

To examine the role of hypoxia in the induction of the netrin-1 gene, *Ntnl,* BMDMs were incubated at a level of oxygen of 1%, which has previously been demonstrated in intraplaque regions (Parathath, et al., Circulation Research, 2011, 109:1141-1152). Macrophages incubated under these conditions showed a 4-fold increase in *Ntnl* mRNA, as well as the hypoxia-inducible gene *Vegf* (Fig. 8a). Similar results were seen under normoxic conditions in macrophages treated with cobalt chloride (CoCl₂), which mimics hypoxia by stabilizing HIF-1α (Piret, et al., Annals of the New York Academy of Sciences, 2002, 973:443-447) and with dimethyloxalylglycine (DMOG) (Groenman, et al., Lung Cellular and Molecular Physiology, 2007, 293:L557-567) which increases endogenous levels of this transcription factor by inhibiting the prolyl hydroxylases (PHDs) (Fig. 12b). Furthermore, as seen with the chemical inducers of hypoxia, oxLDL which is believed to promote oxidative stress in the artery wall (Shatrov, et al., Blood, 2003, 101:4847-4849), also increased both *Ntnl* and *Vegf* mRNA in macrophages (Fig. 12c). Notably, the increase in *Ntnl* mRNA under these various conditions was blocked by pretreating macrophages with an inhibitor of HIF-1α (Fig. 12d-f), implicating this transcription factor in the upregulation *of Ntnl.*

To confirm a role for HIF-1α in the transcriptional regulation of Ntn1 in macrophages, HEK293 cells were transfected with a netrin-1 promoter-luciferase reporter gene, and treated cells with oxLDL or DMOG in the presence or absence a HIF-1α inhibitor. Ntn1 promoter activity was potently induced by oxLDL and DMOG, and this increase was dependent on HIF-1α activity (Fig. 13a-b). Together, these data indicate a central role for this transcription factor in the upregulation of macrophage Ntn1 expression in hypoxic conditions relevant to atherosclerosis.

### HIF-1α induces the netrin-1 receptor Unc5b in macrophages

To investigate the role of HIF-1α in regulating macrophage expression of netrin-1 receptors and other neuronal guidance molecules, a mouse J774 macrophage cell line transduced with a retrovirus expressing HIF-1α engineered to be stable in normoxic conditions (Parathath et al. Circ Res. 2011;109:1141-1152) was used. Gene expression profiling of J774-Con and J774-HIF-1α macrophages using a custom microarray for the analysis of guidance cues of the netrin, slit, semaphorin and ephrin families and their receptors, showed that overexpression of HIF-1α induced expression of Ntn1 and its chemorepulsive receptor Unc5b, but decreased expression of its chemoattractant receptor *Dcc* (Fig. 14a, b, d). Notably, J774-HIF-1α cells also showed decreased expression of the adenosine A2a and A2b receptors (*Adora2a, Adora2b*), which have previously been implicated in the neutrophil response to netrin-1 during transient ischemia. To confirm these changes at the protein level, we performed immunofluorescent staining of J774-HIF-1α and control macrophages. Notably, J774-HIF-1α but not J774-Con cells showed abundant Netrin-1 and Unc5b staining (Fig. 14c), indicating that netrin-1 and unc5b are regulated by a common mechanism during hypoxia.

To further understand the molecular mechanisms regulating the Unc5b receptor in hypoxic conditions, macrophages were incubated with 1% oxygen, oxLDL or under normoxic conditions with chemical hypoxia mimics. As seen for *Ntnl* and *Vegf,* macrophages incubated under these conditions showed 3-6-fold increases in *Unc5b* mRNA (Fig. 15a-c). This increase in *Unc5b* mRNA was dependent on HIF-1α, as macrophages pre-treated with a HIF-1α inhibitor show no upregulation of *Unc5b* in response to low oxygen concentration, CoCl₂ or DMOG (Fig. 15e-f). Similarly, oxLDL induced expression of *Unc5b* mRNA in BMDM under normoxic conditions and this required HIF-1α activity (Fig. 15d). To confirm the role of HIF-1α in the transcriptional regulation of *Unc5b,* HEK293 cells were transfected with an *Unc5b* promoter-luciferase reporter gene. OxLDL dose-dependently increased luciferase activity in these cells (Fig. 15g), and treatment with a HIF-1α inhibitor abrogated this increase in Unc5b promoter activity (Fig. 15h). A similar HIF-1α-dependent increase in *Unc5b* promoter activity was also observed in cells treated with DMOG under normoxic conditions (Fig. 15i-j). Consistent with these *in vitro* studies, immunofluorescent staining of mouse atherosclerotic plaques showed co-localization of Unc5b with a hypoxia probe (Fig. 16a). Similarly, immunofluorescent staining for Unc5b co-localized with HIF-1α in human plaques (Fig. 16b). Collectively, these data demonstrate a key role for HIF-1α in the upregulation of both netrin-1 and its receptor Unc5b in plaque macrophages, which likely promotes an autocrine signaling loop.

### Netrin-1 reduces macrophage migration and promotes survival

As netrin-1 secreted during hypoxia may act in an autocrine/paracrine manner on Unc5b-expressing macrophages, the functional consequences of this regulation were examined. To understand the role of netrin-1 in regulating macrophage migration under hypoxic conditions, the chemotaxis of control or CoCl₂-treated BMDM to MCP-1 (CCL2) were measured in the presence and absence of recombinant Unc5b-FC to block the effects of netrin-1. Macrophages treated with CoCl₂ were strongly impaired in their ability to migrate to MCP-1 compared to control macrophages (Fig. 17a). Notably, treatment of macrophages with Unc5b-FC reversed this effect, restoring macrophage migration to MCP-1. Similarly, overexpression of stable HIF-1α, which induces netrin-1 expression, reduced the migratory capacity of J774 macrophages in the absence of exogenous chemokine (Fig. 17b), whereas J774-HIF cell treated with Unc5b-FC exhibited migration levels comparable to control J774 macrophages. Together these data indicate that upregulation of netrin-1 and Unc5b by hypoxic stress promotes macrophage chemostasis.

In addition to regulating migration, netrin-1 has also been reported to be a survival factor for neurons (Mazelin, et al., Nature, 2004, 431:80-84 and Castets, et al., Developmental Cell, 2009, 16:614-620). To test whether netrin-1 inhibits macrophage apoptosis under hypoxic conditions, apoptosis of growth-factor deprived BMDM was measured under normoxic and hypoxic conditions. Primary BMDM cultivated in the absence of L929 conditioned media undergo apoptosis under normoxic conditions, with a 5-fold increase in TUNEL staining observed after 3 days (Fig. 17c). However, these cells are largely protected from this increase in TUNEL staining when cultured under hypoxic conditions and this protection from apoptosis is reversed in the presence of a HIF-1α inhibitor. To determine whether HIF-1α induction of netrin-1 contributes to macrophage survival under hypoxic conditions, this experiment was repeated in the presence of recombinant Unc5b-FC to block the effects of netrin-1 or a control FC fragment. Under hypoxic conditions, growth factor deprived macrophages treated with control FC remained protected from apoptosis, whereas cells treated with Unc5b-FC showed increased TUNEL and active caspase-3 staining similar to normoxic controls (Fig. 17d-e). These results demonstrate that hypoxia- and HIF-1α-induced expression of netrin-1 promotes the survival of macrophages, which would be predicted to foster macrophage accumulation in plaques.

### Discussion

It is now widely accepted that hypoxia and inflammation are intertwined: hypoxia can induce inflammation, and inflamed tissue often become hypoxic, fueling a continual cycle of inflammation. Hypoxia and chronic inflammation are characteristic features of atherosclerosis, in which macrophages accumulate prominently in arterial plaques. These data provide a molecular mechanism for macrophage persistence during hypoxia through the upregulation of netrin-1, a neuroimmune guidance cue that inhibits chemokine-directed migration of macrophages. Netrin-1 and its receptor Unc5b are expressed in macrophages in hypoxic regions of mouse and human atherosclerotic lesions. Furthermore, HIF-1α, a transcription that mediates cellular adaptations to hypoxia, regulates the expression of both netrin-1 and Unc5b, leading to macrophage chemostasis and protection from apoptosis under hypoxic conditions. Thus, the regulation of the netrin-1-Unc5b axis under hypoxic conditions would be predicted to promote the accumulation of these highly metabolically active cells, further amplifying oxidative stress and chronic inflammation in atherosclerosis.

Recent studies indicate that hypoxia plays a key role in the progression of plaques to advanced stages by increasing lipid accumulation (Parathath, et al., Circulation Research, 2011, 109:1141-1152 and Shatrov, et al., Blood, 2003, 101:4847-4849), inflammation and angiogenesis. The transcription factor HIF-1α is a central regulator of these cellular programs, which help hypoxic cells adapt to their hostile environment. Previous studies reported that like human atheroma, hypoxic regions are present in mouse atherosclerotic plaques (Parathath, et al., Circulation Research, 2011, 109:1141-1152), and show increased accumulation of HIF-1α. *In vitro,* such hypoxic conditions alter macrophage lipid metabolism by increasing macrophage foam cell formation through both induction of sterol synthesis and suppression of cholesterol efflux through a mechanism involving HIF-1α. Lipid-laden macrophage foam cells are known to have a diminished capacity to migrate (Nagao et al., Arterioscler Thromb Vasc Biol., 2007, 27:1596-1602; Park, et al., J Clin Invest., 2009, 119:136-145; Qin et al., Arterioscler Thromb Vasc Biol., 2006, 26:372-378 and Randolph, Curr Opin Lipidol., 2008,19:462-468) thereby promoting macrophage retention in plaques. The present results demonstrate that hypoxia and stabilization of HIF-1α are factors that promote macrophage chemostasis and survival in atherosclerosis. Hypoxic stress induced by low oxygen concentration, or treatment with a key inflammatory component of plaques that increases oxidative stress, oxLDL, upregulated macrophage expression of netrin-1 and its receptor Unc5b. This neuroimmune guidance cue-receptor pair was recently shown to block macrophage migration to chemokines implicated in the egress of macrophages from plaques (e.g. CCL19, CCL2) (van Gils et al., Nat Immunol. 2012;13:136-143). Targeted deletion of netrin-1 in macrophages reduced atherosclerosis burden in *Ldlr*^{*-*/*-*} mice and restored macrophage emigration from plaques. Notably, macrophages overexpressing HIF-1α or treated with a hypoxia mimic have impaired migratory capacity, and this hypoxia-induced macrophage chemostasis is reversed by blocking netrin-1. These data demonstrate that strategies that target hypoxia, thereby reducing netrin-1 and Unc5b, may facilitate macrophage emigration from the plaque and the resolution of inflammation.

In addition to regulating cell migration, netrin-1 plays an important role in promoting macrophage survival during hypoxic stress. HIF-1α has been reported to promote the survival of neutrophils in hypoxic conditions (Tadagavadi, et al., J Immunol., 2010, 185:3750-3758), and a similar role exists for HIF-1α in protecting macrophages from apoptosis during hypoxia. Notably, inhibition of netrin-1 in macrophage cultures under these conditions resulted in a loss of protection from apoptosis. While the effect of netrin-1 on leukocyte survival has not previously been investigated, forced expression of DCC or UNC5 in neuronal and tumor cells in the absence of netrin-1 leads to cell apoptosis, which can be rescued by supplying recombinant netrin1. (Llambi, et al., Embo J., 2001, 20:2715-2722; Forcet, et al., Proc Natl Acad Sci U S A, 2001, 98:3416-3421; Williams, et al., J Biol Chem., 2003, 278:17483-17490 and Chen, et al., Oncogene, 1999, 18:2747-2754) Similarly, netrin-1 has been reported to suppress apoptosis in renal tubular epithelial cells in ischemia/reperfursion injury (Wang, et al., The American Journal of Pathology, 2009, 175:1010-1018). DCC and UNC5, are thought to initiate cell death in the absence of their ligand, as has been described for other dependence receptors including the neurotrophin receptor p75^{NTR} and the androgen receptor. (Rabizadeh, et al., Science, 1993, 261:345-348; Bredesen, et al., Cell Death Differ., 1998, 5:365-371; Ellerby, et al., J Neurochem., 1999, 72:185-195 and Bordeaux, et al., Embo J., 2000, 19:4056-4063)

Whereas the HIF-1α-induced upregulation of netrin-1 and Unc5b in macrophage foam cells in atherosclerotic plaques would likely foster inflammation by both blocking macrophage emigration and sustaining the survival of these trapped cells, other studies report protective roles for HIF-1α and netrin-1 during inflammation. For example, during transient ischemia in the gut, upregulation of netrin-1 on epithelial cells by HIF-1α attenuates neutrophil recruitment and protects the tissue from hypoxia-induced inflammation (Rosenberger, et al., Nature Immunology, 2009,10:195-202). In this model, neutrophil responses to netrin-1 required the adenosine A2B receptor to limit hypoxia induced inflammation, but not Unc5b which were found to be the active receptor in macrophages. In fact, using a custom microarray to measure expression of members of the netrin family and its receptors, in macrophages HIF-1α upregulates netrin-1, Unc5a and Unc5b, but highly downregulates other netrin-1 receptors, including the adenosine A2B receptor and DCC. Consistent with this, Unc5b was detected in macrophages in hypoxic regions of the plaque and co-localized with HIF-1α, where it would likely inactivate emigration and enhance survival of those macrophages, thereby promoting chronic inflammation. Thus, as in the nervous system where netrin-1 can have both positive and negative effects on axonal migration, netrin-1 may play multifunctional roles in regulating inflammation depending on the site of its expression (ie. plaque vs epithelium) and the receptors it engages.

Together, these studies establish that netrin-1 and its receptor, Unc5b, initially characterized in the specialized migration of neurons during development, also regulate macrophage trafficking and accumulation during hypoxia in atherosclerosis. These findings directly demonstrate HIF-1α regulation of netrin-1 in promoting macrophage accumulation and survival in atherosclerotic plaque and provide further evidence of a role for hypoxia in promoting inflammation.

### EXAMPLE 3

### Methods

### Experimental Animals and Diet

C57BL/6J, *Ldlr*^{*-*/*-*} and *Apoe*^{*-*/*-*} mice were from Charles River or Jackson Laboratories. All mice were maintained in a pathogen-free facility. Experimental procedures were performed in accordance with the New York University School of Medicine's Subcommittees on Research Animal Care and Use. All experiments were approved by the New York University School of Medicine Institutional Animal Care and Use Committee. Aortic arch transplantation studies were conducted as previously described (Chereshnev, et al., J Surg Res., 2003, 111:171-176). Briefly, *Apoe*^{*-*/*-*} mice were weaned at 4 weeks and placed on a Western diet (WD; 21% [wt/wt] fat, 0.3% cholesterol; Research Dyets) for 16 weeks. The mice were then divided into three groups (i) a pre-transplant group (n=5) for baseline analysis (ii) aortic transplants into *Apoe*^{*-*/*-*} recipient mice (n=5) (iii) aortic transplants into wild type (C57/BL/6) recipient mice (n=5). Recipient mice were 20 week old males kept on a standard chow diet. The donor arch was interpositioned with the abdominal aorta in the recipient mouse and blood flow was directed through the graft. Recipient mice were maintained on a standard chow diet and sacrificed at 3 days after transplantation. In a second model of regression, 4 week old *Ldlr*^{*-*/*-*} mice were placed on a WD diet for 14 weeks and were either sacrificed (baseline) or switched to chow diet for 4 weeks as previously described (Rayner, et al., J Clin Invest., 2011, 121:2921-2931).

### Histology and Immunostaining

Serial frozen sections of formalin fixed aortic arch or aortic sinus plaques were prepared from OCT embedded tissue using a cryostat. Sections were blocked with 10% normal serum and then incubated for 2 h at 22 °C or overnight at 4 °C with the following primary antibodies: rabbit antibody to Sema3E (10 µg/ml; AF3239; R&D), unconjugated or Alexa Fluor 488-conjugated rat antibody to mouse CD68 (1:250 dilution; FA-11; AbD Serotec), rabbit antibody to plexinD1 (gift from Jonathan Duke-Cohan, Dana-Farber Cancer Institute, Boston, MA) and goat antibody to smooth muscle actin (2.5 µg/ml; ab21027; Abcam). Sections were washed and, where necessary, incubated for 30 minutes with biotinylated antibody to rabbit IgG (BA-1000; Vector Laboratories), followed by incubation for 15 minutes with strepavadin-conjugated Texas Red (Sa-5006; Vector Laboratories) or fluorescein (Sa-5001, Vector Laboratories), or Alexa Fluor 488-conjugated donkey antibody to goat IgG (A11055; Invitrogen) or Alexa Fluor 568-conjugated goat antibody to rat IgG (A11077; Invitrogen). Sections without primary antibody served as negative controls. Sections were mounted with Vectashield medium with DAPI and were visualized with either a TCS-SP confocal laser-scanning microscope (Leica) or an Axioscope 2 Plus fluorescence microscope (Carl Zeiss).

### Laser Capture Microdissection (LCM) and RNA Extraction

Isolation and capture of CD68+ cells from aortic arch or root sections was performed as previously described. Briefly, a guide slide was prepared by staining sections for CD68 as described above. Cells corresponding to CD68+ area in serial sections were collected using a PixCell II instrument (Arcturus Bioscience), and RNA was extracted using the Arcturus Picopure RNA Isolation Kit. Total RNA was amplified using the Ovation WT Pico Amp Kit (NuGen), purified using Qiaquick PCR Purification Kit (Qiagen), and used for quantitative PCR.

### Quantitative RT-PCR

Total RNA was isolated from cells using TRIzol Reagent (Invitrogen). RNA (1µg) was reverse-transcribed with an iScript cDNA Synthesis kit according to the manufacturer's instructions (Bio-Rad). KAPA SYBR FAST qPCR Master Mix was used for RT-PCR analysis. Primers used are listed in supplemental Table I. The change in mRNA expression was calculated by the comparative change in cycle method (ΔΔC_{T}). All data were normalized to GAPDH or 28S and were expressed as fold change over the control.

### Cell Culture

RAW264.7 cells were obtained from American Type Culture Collection. Peritoneal macrophages (pMø) were collected from mice by peritoneal lavage 4 d after intraperitoneal injection of 3% (wt/vol) thioglycollate as previously described (Kunjathoor, et al., J Biol Chem., 2002, 277:49982-49988). Bone marrow derived macrophages (BMDMs) were prepared from mice as previously described (Kunjathoor, et al., J Biol Chem., 2002, 277:49982-49988). For gene expression experiments, macrophages were plated at 1 x10⁶ cells/well in 6 well plates. CoCl₂ (0.1M solution) was obtained from Sigma and used at the concentrations indicated to mimic hypoxia. Oxidation or acetylation of LDL (50 µg/mL; Biomedical Technologies) was performed as described (Kunjathoor, et al., J Biol Chem., 2002, 277:49982-49988). Recombinant IL-4 and IFN-γ were purchased from PeproTech. Ultrapure LPS from *E. coli* K12 was purchased from InvivoGen.

### F-actin staining

Freshly isolated peritoneal macrophages were seeded on 8 well Lab Tek slides (Thermo Scientific) for 24 hours or used directly. The cells were pretreated with mouse recombinant Semaphorin 3E (250 ng/ml; R&D Systems) or PBS for 30 minutes and stimulated with either CCL19 (500ng/ ml; R&D Systems) or CCL2 (10ng/ml; R&D Systems) for the indicated times. Macrophages were fixed (4% paraformaldehyde), permeabilized (0.1% Triton X-100) and actin filaments stained with Alexa Fluor 568 or 647 phalloidin (Molecular Probes, Invitrogen) at a dilution of 1/40. The cells on coverslips were mounted after DAPI staining and visualized with either a TCS-SP confocal laser-scanning microscope (Leica) or an Axioscope 2 Plus fluorescence microscope (Carl Zeiss). Images were background corrected followed by measurement of the average cell area of at least 10 cells per field using I-vision software.

### Migration Assays

Chemotaxis of macrophages (pMø and BMDMs) was measured in 96-well Boyden chamber with a 5 µm (BMDMs) or 8 µm (pMø) membrane (Neuro Probe) as previously described (van Gils, et al., Nat Immunol., 2012, 13:136-143). Macrophage chemotaxis was also quantified using a Real-time Cell Invasion and Migration (RT-CIM) assay system with monitoring every 5 min (xCELLigence, Roche Applied Science). Cell migration to CCL2 (100ng/ml; R&D Systems) or CCL19 (500 ng/ml; R&D Systems) was measured in the absence or presence of recombinant Sema3E (mouse or human). In some assays cells were pretreated Sema3E for 45 min prior to migration. Following cell migration for 3 or 16 hours, four random high power fields from each well were counted to determine the number of cells that had migrated through the membrane. Migration was expressed as the chemotactic index, which was calculated by dividing the number of migrating cells in the treated groups by the number of migrating cells in the lowest control well. Results of chemotaxis assays are representative of at least three independent experiments performed on triplicate samples.

### Western Blot

Cells were lysed in ice-cold buffer containing 50 mM Tris·HCl, pH 7.5, 125 mM NaCl, 1% Nonidet P-40, 5.3 mM NaF, 1.5 mM NaP, 1 mM orthovanadate, 175 mg/ml octylglucopyranoside, 1 mg/mL protease inhibitor cocktail (Roche), and 0.25 mg/m 4-(2-aminoethyl)-benzenesulfonyl fluoride hydrochloride (AEBSF) (Roche). Cell lysates were rotated at 4 °C for 1 h before the insoluble material was removed by centrifugation at 12,000 × g for 10 minutes. After normalizing for equal protein concentration, cell lysates were resuspended in SDS sample buffer before separation by SDS/PAGE. After overnight transfer of the proteins onto nitrocellulose membranes, the membranes were probed with the indicated antibodies, and protein bands were visualized using the Odyssey Infrared Imaging System (LI- COR). Densitometry analysis of the gels was carried out using ImageJ software from the National Institutes of Health.

### Results

### Sema3E is Expressed in Macrophages of Advanced Atherosclerotic Mouse Plaques and its Expression Decreases in Models of Plaque Regression.

To investigate the expression of Sema3E at the protein level in atherosclerosis, immunohistochemical staining was performed on aortic root plaques of *Apoe*^{*-*/*-*} mice fed a western diet for 12 weeks. In these progressing atherosclerotic plaques, double-staining for Sema3E and the macrophage marker CD68 showed Sema3E protein present in lesional macrophages (Fig. 18a, arrows). In addition, there appeared to be extracellular Sema3E staining in macrophage-rich regions of the plaque, consistent with Sema3E being a secreted protein that can bind to extracellular matrix. Furthermore, staining for the Sema3E receptor PlexinD1 also co-localized with CD68-positive macrophages in these advanced atherosclerotic plaques (Fig. 18b, arrows), demonstrating that these cells may be both the source and target of Sema3E secreted in the plaque.

To understand the dynamics of Sema3E expression in atherosclerosis, an established model of atherosclerosis regression was used in which the aortic arch from *Apoe*^{*-*/*-*} mice fed a western diet (WD) for 16 weeks is transplanted into either a hyperlipidemic Apoe^{*-*/*-*} (progressive environment) or normolipidemic wild type C57BL6 (regressive environment) recipient mouse for 3 days (Chereshnev, et al., J Surg Res., 2003, 111:171-176). Similar to its expression in aortic root plaques, Sema3E was abundantly expressed in aortic arch plaques in the progressive environment (*Apoe*^{*-*/*-*} → *Apoe*^{*-*/*-*}) in regions that stained positive for the macrophage marker CD68 (Fig. 19a). By contrast, staining for Sema3E was markedly reduced in plaques of aortic arch segments transplanted into the regressive environment (*Apoe*^{*-*/*-*}→C57BL6), which as in previous studies (eg. (Trogan, et al., Arterioscler Thromb Vasc Biol., 2004, 24:1714-1719)), also show a decrease in plaque size and macrophage content. To confirm that macrophages are a source of Sema3E expression in the plaque, mRNA was isolated from lesional CD68+ macrophages by laser capture microdissection and measured gene expression by quantitative RT-PCR (qPCR). Consistent with immunohistochemical analyses and the microarray studies (Feig, et al., PLoS One, 2012, 7:e39790), macrophages isolated from plaques in the progressive environment expressed abundant *Sema3e* mRNA (Fig. 19b), which was reduced by 90% in macrophages from plaques transplanted into a regressive environment for only 3 days. This decrease in macrophage *Sema3e* mRNA also correlated with a reduction in the inflammatory M1 macrophage marker *Nos2* and an increase in the anti-inflammatory M2 macrophage marker *Argl* (Fig. 19b), previously shown to characterize plaques undergoing regression (Feig, et al., Proc Natl Acad Sci USA, 2011, 108:7166 - 7171; Rayner, et al., J Clin Invest., 2011, 121:2921-2931; and Feig, et al., Circulation, 2011, 123:989-998). To investigate further whether *Sema3e* expression is associated with the M1 or M2 macrophage phenotype, its expression was measured in bone marrow derived macrophages (BMDM) polarized *in vitro. Sema3e* mRNA is highly increased in M1 macrophages polarized with LPS and IFNγ, but not M2 macrophages polarized with IL-4, compared to untreated macrophages (Fig. 19c), indicating that its expression is correlated with inflammatory macrophages.

As the atherosclerosis regression observed in the aortic arch transplant model is quite rapid, whether *Sema3e* was also regulated in plaques under conditions simulating less aggressive lipid-lowering management was determined. To do this, *Ldlr*^{*-*/*-*} mice were fed a WD for 14 weeks and either sacrificed for baseline plaque measurements or switched to a chow diet for an additional 4 weeks. This switch to chow is associated with reductions in total plasma cholesterol and plaque lipid content (Rayner, et al., J Clin Invest., 2011, 121:2921-2931). Consistent with findings in the transplant model of regression, macrophages isolated from aortic root plaques of *Ldlr*^{*-*/*-*} mice switched to chow diet showed a marked decrease in *Sema3e* mRNA compared to macrophages from baseline progressing atherosclerotic plaques (Fig. 19d). Collectively, these data indicate that *Sema3e* expression by lesional macrophages differentially responds to progressive and regressive atherosclerotic environments.

### Macrophage Sema3E expression is up-regulated by physiological drivers of plaque inflammation.

To further understand the molecular mechanisms of Sema3E expression in plaque macrophages and to extend the above observations in M1 and M2 polarized macrophages, whether Sema3E is induced by pro-inflammatory/atherogenic stimuli *in vitro* was determined. Macrophages were treated with acetylated or oxidized LDL (modifications that promote cholesterol loading of macrophages) and measured expression of *Sema3e* mRNA by qPCR. AcLDL or oxLDL induced a 3-4-fold increase in *Sema3e* mRNA (Fig. 20a-b), whereas native LDL did not alter expression of these genes (data not shown). As oxLDL promotes plaque oxidative stress (Feig, et al., Circulation, 2011, 123:989-998) and induces the hypoxia-inducible factor 1α (HIF1α) transcription factor, BMDM was treated with a chemical mimic of hypoxia, CoCl₂, which stabilizes HIF1α̃ Similar to oxLDL, CoCl₂ increased macrophage *Sema3e* mRNA 5-fold (Fig. 20c). Immunoblot analysis confirmed an increase in full-length Sema3E in the supernatant of oxLDL-treated macrophages, which was paralleled by an increase in the cleaved form of Sema3E (Fig. 20d). Notably, treatment with HDL, which promotes cholesterol efflux from the cell and blocks the inflammatory actions of oxLDL (Yvan-Charvet, et al., Arterioscler Thromb Vasc Biol., 2010, 30:139-143), reduced levels of Sema3E secreted by oxLDL-stimulated cells, whereas treatment of macrophages with HDL alone did not alter levels of full-length or cleaved Sema3E (Fig. 20d). Similarly, macrophages treated with oxLDL, but not HDL, show increased abundance of PlexinD1 protein, and the effects of oxLDL on PlexinD1 are partially reversed by co-incubation with HDL (Fig. 20e). Collectively, these data demonstrate that Sema3E and PlexinD1 expression in macrophages is dynamically regulated by pro- and anti-atherosclerotic stimuli.

### Sema3E Inhibits the Migration of Macrophages

Sema3E has previously been reported to act as a negative guidance cue for thymocytes, and inhibits the migration of these cells to CCL25 and CCL21 (Choi, et al., Immunity, 2008, 29:888-898). As recent studies have identified the importance of chemokine-mediated macrophage emigration from the plaque during atherosclerosis regression (Llodra, et al., Proc Natl Acad Sci USA, 2004, 101:11779-11784; Trogan, et al., Proc Natl Acad Sci USA, 2006, 103:3781-3786; Feig, et al., Proc Natl Acad Sci USA, 2011, 108:7166 - 7171; van Gils, et al., Nat Immunol., 2012, 13:136-143; Feig, et al., PLoS One, 2012, 7:e39790; and Trogan, et al., Arterioscler Thromb Vasc Biol., 2004, 24:1714-1719; and Feig, et al., Circulation, 2011, 123:989-998), the effect of Sema3E on macrophage chemotaxis was determined using both transwell Boyden chambers and a real-time detection method (xCelligence). Whereas Sema3E had little effect on macrophage migration in the absence of chemokine, it potently inhibited peritoneal macrophage migration towards the CCR7 ligand, CCL19, a chemokine receptor-ligand pair implicated in macrophage egress from atherosclerotic plaques_(Trogan, et al., Proc Natl Acad Sci USA, 2006, 103:3781-3786) (Fig 21a). Analogous results were obtained using real time detection of macrophage migration, where peritoneal macrophages showed an increase in migration to CCL19 in the lower chamber within 30 min, whereas for up to 4 hours, macrophages treated with 250 ng/ml Sema3E and CCL19 showed no increase migration above that in cells treated with media alone (Fig. 21b). Peritoneal macrophages pre-treated with Sema3E for 45 minutes, and then washed before exposure to chemokines, also showed impaired migration to CCL19 (Fig. 21c), indicating that Sema3E does not require direct interaction with the chemokine to mediate its inhibitory effect. This effect was not chemokine-specific, as Sema3E also blocked migration of peritoneal macrophages to CCL2 (Fig. 21d), a second chemokine implicated in the emigration of inflammatory macrophages to the lymph nodes (Jimenez, et al., Journal of immunology, 2010, 184:5571-5581 and Sato, et al., The Journal of Experimental Medicine, 2000, 192:205-218).

To gain insight into the mechanisms by which Sema3E inhibits directed chemotaxis of macrophages to multiple chemokines, its effect on the organization of the actin cytoskeleton was measured. Stimulation of pMø with CCL19 or CCL2 induced a marked reorganization of actin, characterized by the appearance of membrane ruffles, lamellipodia and filapodia (Fig. 22a-b). Pre-treatment with Sema3E prior to stimulation with CCL19 or CCL2 inhibited these effects, and cells maintained a rounded morphology consistent with their non-motile phenotype. Accordingly, whereas pMø exhibit rapid cell spreading in response to chemokine stimulation, cell pre-treated with Sema3E showed no increase in mean cell area (Fig. 22a-b).

As members of the Rho family of GTPases play key roles in the reorganization of actin in macrophages, whether Sema3E altered activation of Rac1 and CDC42 in response to chemotactic stimulation was determined. Using GST beads conjugated to PAK1-PBD to immunoprecipitate the GTP-bound forms of Rac1/CDC42, that levels of activated Rac1 and CDC42 were rapidly increased in BMDM stimulated with CCL2 (Fig. 23a-b). By contrast, pretreatment of macrophages with Sema3E inhibited CCL2-induced Rac1 and CDC42 activation. Thus, Sema3E blocks activation of the effectors of lamellipodia (Rac1) and filopodia (CDC42) formation, which are essential components of the migratory machinery. Collectively, these data indicate that Sema3E inhibits the directional migration of macrophages by disrupting the Rho GTPase signaling cascade, re-organization of the actin cytoskeleton and cell polarization.

### Discussion

Atherosclerosis is a chronic and progressive inflammatory condition, driven by the accumulation of lipids and immune cells in plaques. Current concepts (e.g. (Moore, et al., Cell, 2011, 145:341-355)) view the persistence of macrophages in plaques as a failure to resolve inflammation. Recent studies in mouse models of atherosclerosis have revived the hope that plaque macrophage lipid homeostasis and emigration can be clinically restored with aggressive lipid management, leading to the achievement of atherosclerosis regression in humans (Williams, et al., Cardiovascular Medicine, 2008, 5:91-102). These studies have indicated that macrophage egress from the plaque is likely to be actively inhibited during disease progression, such as with hypercholesterolemia, although the factors and their regulatory signals that impair egress remain largely unknown. The present studies demonstrate Sema3E to be one such factor by showing that: 1) it is expressed in mouse atherosclerotic plaques, where there is significant co-localization with monocyte-derived cells; 2) its expression *in vivo* is significantly lower in these cells in a plaque regression vs. progression environment; 3) its expression *in vitro* is higher in inflammatory M1 macrophages than in tissue remodeling, M2 ones, which are enriched in regressing plaques; and 4) it retards the migration of macrophages *in vitro* to a number of chemokines, including CCL19, previously shown to be a mediator of macrophage egress from regressing plaques (Trogan, et al., Proc Natl Acad Sci U S A, 2006, 103:3781-3786), apparently by altering cytoskeletal reorganization. Thus, Sema3E demonstrates macrophage-retention, anti inflammation-resolving effect in plaques similar to what we recently established for another neuronal guidance molecule, netrin-1 (van Gils, et al., Nat Immunol., 2012, 13:136-143).

Neuronal guidance cues are increasingly recognized as important players in immune function and chronic inflammatory diseases, and recent studies have implicated members of the Semaphorin family in the pathogenesis of rheumatoid arthritis (Sema3E, Sema3C) (Mangasser-Stephan, et al., Biochem Biophys Res Commun, 1997, 234:153-156 and Miller, et al., Arthritis Rheum., 2004, 50:1156-1163 and Miller, et al., Arthritis Rheum., 2004, 50:1156-1163) and atherosclerosis (Sema4D) (Yukawa, et al., Int J Mol Med., 2010, 26:39-44 and Zhu, et al., Arterioscler Thromb Vasc Biol., 2009, 29:1039-1045). This is the first time that Sema3E has been shown to be dynamically regulated in macrophages and identified in atherosclerotic plaques. Previous studies have shown the expression of Sema3E in the outer retina of embryonic chicks, in neurons throughout the ganglion cell layer of P4 mouse retinas, in thalamostriatal projection neurons and developing somites of mice, in mouse medullary thymocytes, and in mouse calvarial osteoblasts (Sakurai, et al., Mol Cell Biol., 2010, 30:3086-3098; Steinbach, et al., Exp Cell Res., 2002, 279:52-61; Choi, et al., Immunity, 2008, 29:888-898; Hughes, et al., Calcif Tissue Int., 2012, 90:151-162; Fukushima, et al., J Clin Invest., 2011, 121:1974-1985; Ding, et al., Nat Neurosci., 2012, 15:215-223; Gu, et al., Science, 2005, 307:265-268; and Klagsbrun, et al., J Clin Invest., 2010, 120:2658-2660). Although there is a lack of understanding of the factors that mediate Sema3E expression in the majority of these cell types, in addition to the factors identified herein (eg. hyperlipidemia, oxLDL, hypoxia), 1,25-dihydroxyvitamin D3 has been reported to increase Sema3E expression in mouse osteoblasts (Hughes, et al., Calcif Tissue Int., 2012, 90:151-162). PlexinD1, the canonical receptor for Sema3E, is highly expressed in many cell types, including macrophages (Janabi, et al., Arterioscler Thromb Vasc Biol., 2000, 20:1953-1960), as we also describe herein. In addition, we find that PlexinD1 is upregulated by oxLDL in vitro and is expressed by macrophages in atherosclerotic plaques.

Sema3E is unique from other Semaphorins in that it binds directly to PlexinD1, and does not require co-receptors to exert its repellant signal (Gu, et al., Science, 2005, 307:265-268). PlexinD1 is thought to exist at the plasma membrane in an inactive folded state, and upon binding with Sema3E undergoes a conformational change that activates its Guanosine triphosphates (GTP-ase)-Activating-Protein (GAP) domain, which may enable protein-protein interactions with other domains, such as its Rho GTPase-Binding Domain (RBD) (Gay, et al., Dev Biol., 2011, 349:1-19). The Sema3E activation of PlexinD1 can disrupt focal adhesions and integrin-mediated cell adhesion to the extracellular matrix, affect PI3K signaling and cause the RhoA dependant collapse of the cytoskeleton in neurons and endothelial cells (Perala, et al., Differentiation, 2012, 83:77-91; Gay, et al., Dev Biol., 2011, 349:1-19; and Wang, et al., Sci Signal, 2012, 5:ra6). Activation of PlexinD1 by Sema3E has also been shown to repress CCL25 signaling via its receptor CCR9 in thymocytes (Choi, et al., Immunity, 2008, 29:888-898), and to inhibit mouse osteoblast migration in wound healing assays (Hughes, et al., Calcif Tissue Int., 2012, 90:151-162).

Many of the properties of the Sema3E-PlexinD1 axis in other cell types, then, are consistent with what is observed in macrophages, namely the effects on the cytoskeleton and the inhibition of migration to a number of chemokines, including CCL19, previously implicated as an atherosclerosis regression factor (Trogan, et al., Proc Natl Acad Sci USA, 2006, 103:3781-3786). Perhaps more striking, then, was the dynamic regulation of Sema3E expression in macrophages by factors related to atherosclerosis. That Sema3E expression at the protein or mRNA level was higher in progressing than in regressing plaques, and increased *in vitro* by oxLDL and a hypoxia mimic (CoCl₂), strongly implies that it participates in the retention of macrophages and the failure to resolve inflammation under pro-atherogenic conditions. This is further supported in a model of *in vitro* macrophage polarization where it was shown that Sema3E is highly expressed in 'classically activated' inflammatory M1 macrophages, which are thought to be the predominant phenotype under conditions that are pro-atherogenic, but less expressed in the reparative "M2" macrophages, which are considered mediators of inflammatory resolution, and whose markers are enriched in regressing plaques (Feig, et al., Proc Natl Acad Sci USA, 2011, 108:7166 - 7171; Rayner, et al., J Clin Invest., 2011, 121:2921-2931; Feig, et al., PLoS One, 2012, 7:e39790; and Parathath, et al., Diabetes, 2011, 60:1759-1769) coincident with decreased *Sema3e* mRNA expression.

Monocyte and macrophage death are well-recognized factors that determine the content of macrophages in mouse models of atherosclerosis (Moore, et al., Cell, 2011, 145:341-355). An emerging picture (recently reviewed in Gerszten, et al., The New England Journal of Medicine, 2012, 366:1734-1736) based on the present and prior studies (Llodra, et al., Proc Natl Acad Sci U S A, 2004, 101:11779-11784; Trogan, et al., Proc Natl Acad Sci U S A, 2006, 103:3781-3786; van Gils, et al., Nat Immunol., 2012, 13:136-143; and Ly, et al., Proc Natl Acad Sci U S A, 2005, 102:14729-14734) is that macrophage egress is also a determinant of plaque macrophage content and that it is regulated by factors that can be broadly classified as gas pedals [emigration signals (e.g. CCR7)], and brakes [inhibitory guidance cues (e.g. Sema3E and netrin-1), adhesion molecules (e.g. cadherins, vinculin), cellular motility factors (e.g. actin and myosin)] (Feig, et al., PLoS One, 2012, 7:e39790). Based on the hundreds of differences in the transcriptomes of monocyte-derived cells in regressing vs. progressing plaques (Feig, et al., PLoS One, 2012, 7:e39790), it is likely that the list of gas pedals and brakes, as well as their modifiers, will grow. This will not only expand our fundamental understanding of atherosclerosis and other chronic inflammatory conditions, but will also suggest strategies to reduce the considerable residual risk of heart disease observed with the currently available therapies.

### EXAMPLE 4

### Materials and Methods

Mice. 6-8 week old C57BL/6J mice and 26 week old C57BL16J mice fed a HFD consisting of 60% Kcal from fat (012492, Research diets) or a control chow diet for 20 weeks were purchased from Jackson Laboratories. All mice were maintained in a pathogen-free facility. For bone marrow transplantation studies, female and male *Ntn1*^{*+*/*-*} mice (10 generations backcrossed onto C57BL/6 background) were mated to generate *Ntn1*^{*+*/*+*} and *Ntn1*^{*-*/*-*} donor mice. On day 14 of gestation, embryos were dissected free from the placenta and yolk sac, and single-cell suspensions of fetal liver cells were prepared by flushing through graded sizes of needles. A portion of the fetal tissue was used for genotyping by 5-bromo-4-chloro-3-indoyl-β-d-galactoside staining and PCR analysis. Fetal liver cells (2x 10⁶) were injected intravenously into 6-8 week old recipient C57BL/6 mice that were lethally irradiated (two exposures of 600 cGy), and mice were allowed to recover for 6 weeks. C57BL6 mice were fed a standard chow or HFD consisting of 60% Kcal from fat (012492, Research diets) for 20 weeks. At the termination of the study, mice were anesthetized with Tribomoethanol (0.4mg/g i.p.) and ex-sanguinated by cardiac puncture. Experimental procedures were performed in accordance with the USDA Animal Welfare Act and the PHS Policy for the Human Care and Use of Laboratory Animals and the New York University School of Medicine's Research Animal Care and Use.

Human subjects. Visceral adipose tissue (omental and mesenteric) was collected from patients who underwent laparotomy for neoplastic disease at a site remote from the collection of visceral adipose tissue. The lean patients had a body mass index (BMI) ≤ 25 kg/m². Obese patients had a BMI ≥ 28 kg/m². The NYU School of Medicine IRB approved tissue collection. Serum concentrations of netrin-1 were measured a second group of healthy age matched (mean 71±1.5 yr) lean (BM1=18.2±1.4 kg/m²;n=20;6M/14F) an d obese patients (BMI=38.6±2.4 kg/m²;n=20;9M/11F) recruited under an IRB approved protocol at the University of Ottawa Heart Institute.

Gene Expression Profiling. 1 mg of adipose tissue from either lean or obese animals were homogenized in TRIzol reagent (Invitrogen) using the Bullet BlenderTM (New England Bio Group) and total RNA isolated as we described (van Gils, et al., Nat Immunol, 2012; 13: 136-143). RNA quality was verified by pico chip (5067-1511; Agilent technologies). Adipose RNA (1µg) was reverse transcribed and quantitative RT-PCR analysis of the 4 families of the axonal guidance molecules was performed using RT2 Custom Profiler PCR Arrays (Qiagen) according to manufacturer's protocol. Data analysis was performed using the manufacturer's integrated web-based software package of the PCR Array System using ΔΔCt based fold- change calculations.

Real-time Quantitative RT-PCR Analysis. RNA (0.5-1µg) was reverse transcribed using iScriptTM cDNA Synthesis Kit (Biorad) and RT-PCR analysis was conducted using kapa SYBR green Supermix (kappa syber) and a Mastercycler Realplex (Eppendorf) the primers used are listed. Fold change in mRNA expression was calculated using the comparative cycle method (ΔΔCt).

Adipocyte and ATM purification. Fat pads of mice fed were excised and minced in HBSS. Tissue suspensions were centrifuged at 500 g for 5 minutes to remove erythrocytes and free leukocytes. Liberase (5401119001; Roche applied science) was added to 1 mg/ml and incubated at 37°C for 30 minutes with shaking. The cell suspension was filtered through a 100-µm filter and then spun at 300 g for 5 minutes to separate floating adipocytes from the SVF pellet. To ensure proper isolation, adipocyte fractions were examined by microscopy before and after plating on plastic to detect adherent cells. Samples were digested until adipocyte fractions were free of adherent cells by these 2 quality-control methods to ensure recovery of the majority of the SVF population. For cell culture, the adipocytes were resuspended in growth media for further experiments. Isolation of CD11b+ cells from SVF isolates was performed by magnetic immunoaffinity isolation with anti-CD11b antibodies conjugated to magnetic beads (120-000-300; MACS Miltenyi Biotec). Cells were isolated using positive selection columns prior to preparation of whole-cell lysates.

Cell culture. Primary bone marrow derived macrophage (BMDM) was prepared by flushing the bone marrow of the tibia and femur of 6-8 week old C57/B6 mice. The cells were grown in DMEM supplemented with 15% L929-conditioned media, 10% FBS and 1% penicillin/streptomycin for 7 days. BMDM were stimulated 250 pM palmitate conjugated with BSA or BSA as a control for 24 hours, or TNFα (10ng/ml, 410-MT-010; R&D systems), IL-6 (40 nglml, 406-ML-005; R&D systems) and IL-4 (1 Onglml, 404-ML-010; R&D systems) for 24 h. In some assays, differentiated BMDM were treated with conditioned media from 3T3-L1 adipocytes differentiated as described (Lin, et al., J Biol Chem, 2005; 280: 4617-4626) and treated with 250 pM palmitate conjugated with BSA or BSA for 24 hours. In certain experiments, anti-TNFa (0.4µg/ml, AF-410-NA; R&D systems) or anti-IL-6 (0.025µg/ml, MAB406; R&D systems) blocking antibody or IgG control (Normal Goat IgG: AB-108-C, Rat IgG1: MAB005; R&D systems) was added to conditioned media. Peritoneal macrophages were isolated as described (van Gils, et al., Nat Immunol, 2012; 13: 136-143). Mice were injected intraperitoneally with 1 ml of 3% (wt/vol) thioglycolate to elicit sterile sterile peritonitis with macrophage numbers peaking on day 4. Cells were collected by lavage and used for migration assays.

Palmitate-BSA preparation. Sodium palmitate (P9767; Sigma Aldrich) was dissolved in sterile water in to make a 100-mM solution by alternating heating and vortexing until the palmitate was dissolved. This occurred once the solution reached 70C. Immediately after the palmitate dissolved, it was conjugated to serum-free DMEM containing 5% NEFA-free BSA, creating a 5-mM palmitate solution as described (Yeop, et al., Diabetes, 2010; 59: 386-396). The 5-mM palmitate solution was shaken at 140 rpm at 40C for 1 hour and was then immediately used to treat the cells. Serum-free DMEM containing 5% NEFA-free BSA was used as the vehicle control.

Promoter-Luciferase assays. HEK293T cells were transfected with human NTNI or Unc5H2 promoter-luciferase reporter constructs (SwitchGear Genomics) using LipofectamineTM 2000 (11668-019, Invitrogen). After transfection, media was changed and cells were treated with palmitate or BSA for 24 hours, in the presence or absence of the NFkB inhibitor BAY 11-7082 (10 µmol/L). Luciferase activity was measured using the DUAL-Glo Luciferase Assay system (E1910, Promega).

Analytical procedures. Blood glucose values were determined from whole venous blood using an automatic glucose monitor (Freestyle Lite). The fat content in the mice was analyzed by dual x-ray absorptiometry (DXA) scanning. The mice were anesthetized and placed in the prone position on the specimen tray to allow scanning of the entire body.

Glucose-and insulin-tolerance tests. Glucose-tolerance tests were performed as previously described (Konner, et al., Cell Metab, 2007; 5: 438-449). After determination of fasted blood glucose levels, each animal received a glucose gavage of 1.5g/kg body weight of glucose (25% Dglucose, G7528; Sigma). Blood glucose levels were determined after 15, 30, 60 and 120 min. Insulin-tolerance tests were carried on unfasted animals by injecting an i.p injection of 1.5U/kg body weight of insulin (HumilinR 100U/ml). Blood glucose levels were detected after 15, 30, 60, 120 and 240 mins.

ELISA assays. Insulin (90080; crystal Chem Inc), adiponectin (ab108785; Abcam), FFA (SFA-1; Zen Bio), TNFa (88-7064-76; ebiosciences) and netrin-1 (E91827Mu; USCN life science Inc) levels were measured in the serum using mouse standards according to manufacturer's guidelines. Netrin-1 in human serum was measured using a human-specific netrin-1 ELISA from USCN Life Science Inc (700176; mybiosource).

Labeling and tracking of blood monocytes. Circulating blood monocytes were labeled in *WT*→*WT* and Ntn1^{*-*/*-*}→*WT* mice fed a HFD for 20 weeks by retro-orbital intravenous injection of 1µm Fluoresbite green fluorescent plain microspheres (Polysciences) diluted 1 :4 in sterile PBS as described (van Gils, et al., Nat Immunol, 2012; 13: 136-143; Sabio, et al., Science, 2008; 322: 1539-1543). Labeling efficiency was assessed 1 day after injection of beads. One group of mice was harvested after 3 days for baseline measurements, as this time point has previously been shown to have optimal recruitment of labeled monocytes into tissues and clearance of the labeled monocytes from the blood. A second group of mice was harvested 14 days later to measure the number of labeled macrophages remaining in adipose. Adipose tissue was sectioned (5 µM) and the number of beads per section (x20) was counted from 9 slides per mouse.

Immunohistochemistry. White adipose tissue was excised, fixed in formalin overnight, embedded in paraffin and sectioned. For adipocyte quantification, the area (µm2) of adipocytes was determined in the WAT using Image J software. The immunofluorescence analysis of netrin-1 (AF1109; R&D systems), caveolin-1 (610059; BD bioscience), Unc5b (ab54430; Abcam), F4/80 (MCA497GA; Abd serotec), CD68 (MCA1957; Abd serotec) was conducted after deparaffinization as described previously (Konner, et al., Cell Metab, 2007; 5: 438-449). Secondary antibodies were then applied (Alexa Fluor-568, A11011; Alexa Fluor-488, A11006; Invitrogen). Netrin-1 and Unc5b staining was amplified using the biotin conjugated antibodies (BA-2000 and BA-9500; Vector Laboratories) followed by streptavidin conjugated AMCA (Sa-5008; Vector Laboratories) staining. Sections were mounted and visualized using a Nikon Eclipse microscope.

Migration. The migration of ATM or peritoneal macrophages to CCL19 (500ng/ml; R&D Systems) was assessed by the xCelligence Real-time Cell Invasion and Migration system (ACEA biosciences Inc) that monitored chemotaxis every 5 minutes for over 6 h. The SVF fraction was isolated from mice fed a chow (lean) or HFD (obese) for 14-16 weeks as described above and the cells were sorted on magnetic columns to select for CDllb+/F4/80+ (NC97677703, 130-099-440, Miltenyi Biotec). In certain experiments, recombinant rat Unc5b-Fc or control IgG or recombinant mouse netrin-1 (R&D systems) was added to the Boyden chamber.

Western-blot analysis. The WAT, liver and muscle tissues were homogenized in lysis buffer as previously described (Konner, et al., CellMetab, 2007; 5: 438-449). Western-blot analyses were carried out according to standard protocols with antibodies raised against netrin-1 (MAB1109; R&D Systems), Unc5b (AF1006; R&D Systems), AKT (9272; Cell Signaling, 1:1,000), phosphoAKT Ser 473 (9271; Cell Signaling, 1:1,000), and a-tubulin (T6074; Sigma, 1:5,000) were used as loading controls.

Statistical methods. The difference between two groups was analyzed by Student's t-test or for multiple comparisons, by one-way analysis of variance, followed by Newman-Keus multiple comparison test. P values of less than 0.05 were considered significant.

### Results

Adipose tissue expression of netrin-1 and its receptor Unc5b is regulated by obesity. To identify neuronal guidance cues undergoing metabolic regulation and potentially contributing to the development of obesity-associated metabolic dysregulation in glucose and lipid metabolism, gene expression profiling of adipose tissue was performed from C57BLl6J mice fed either a chow or a high-fat diet (60% Kcal) for 20 weeks. Using a microarray representing a complete panel of netrin, semaphorin, slit and ephrin guidance cues and their receptors, expression of netrin (*Ntnl*) mRNA was highly expressed in visceral adipose tissue from HFD-fed obese mice, but not lean chow-fed mice (Fig. 24a). High fat diet-induced upregulation of netrin-1 in adipose tissue was confirmed by quantitative real-time PCR (qRT-PCR) analysis (Fig, 24a) and western blotting (Fig. 24b). As netrin-1 is a secreted molecule, its levels in the serum were measured by ELISA. There was no significant difference in chow- and HFD-fed mice (Suppl. Fig. 24a). In addition to the selective increase in netrin-1 expression in adipose tissue of obese mice, mRNA and protein levels of its receptor Unc5b, which mediates netrin-1 chemorepulsion, were increased (Fig. 24a, b). By contrast, expression of the netrin-1 chemoattractive receptors *Dcc* and *Neogenin* were unchanged in visceral adipose tissue of obese mice compared to lean mice (Fig. 24a). The increases in *Ntn1* and *Unc5b* mRNA correlated with increases in mRNA levels of the macrophage marker F4/80 (*Emr1*) and the pro-inflammatory cytokine genes *ll6* and *Tnfa* in obese adipose tissue (Fig. 24a). Increased expression of other select neuroimmune guidance molecules was also observed in obese adipose tissue, including *Slit2,* as well as the secreted semaphorin *Sema3e* (Fig. 24a), which was recently reported to promote monocyte recruitment into adipose tissue during HFD feeding (Schmidt, et al., Cell Metab, 2013; 18: 461-462; Shimizu, et al., Cell Metab, 2013; 18: 491-504).

To identify the cellular compartment expressing netrin-1 and Unc5b, mRNA was isolated from the adipocyte and stromal vascular fractions of adipose from chow- and HFD-fed mice and qRT-PCR analysis was performed. *Ntnl* and *Unc5b* mRNA were selectively increased in the stromal vascular fraction of adipose from HFD-fed mice (Fig. 24c). Consistent with this finding, immunofluorescence staining of adipose from HFD-fed mice showed netrin-1 and Unc5b reactivity in crown-like structures between adipocytes that co-localized with staining for the macrophage antigen F4/80 (Fig. 24d). Netrin-1 and Unc5b staining was not detected in adipose tissue from lean mice (data not shown), indicating that this guidance cue-receptor pair is specifically increased in macrophages that infiltrate obese adipose tissue.

To determine the translational relevance of the data in this mouse model of diet-induced obesity, the expression of netrin-1 and Unc5b in human adipose tissue collected from lean (BMI<25 kg/m²) and obese (BMI>28 kg/m²) individuals was measured. *NTNI* and *UNC5H2* (the homologue of *Unc5b),* but not *DCC* mRNA were elevated in adipose from obese compared to lean subjects, and correlated with increased expression of macrophage marker *CD68* and the inflammatory cytokine *TNFA. SEMA3E* was also upregulated in obese subjects, whereas other neuronal guidance cues with immunoregulatory functions such as *SEMA3A* and *EFNB2* were unchanged (Fig. 25a). Immunofluorescence staining showed netrin-1 reactivity in adipose tissue from obese, but not lean individuals that co-localized with CD68-positive macrophages in crown-like structures (Fig. 25b). Interestingly, serum levels of netrin-1 were modestly reduced in obese (mean BMI 38.6 kg/m²) subjects compared to lean (mean BMI 18.2 kg/m²) individuals (Suppl. Fig. 24b). Collectively, these data indicate a selective increase in netrin-1 in ATM of mice and humans during obesity.

### Palmitate regulates netrin-1 and Unc5b expression

The molecular mechanisms of netrin-1 and Unc5b upregulation in ATMs during obesity were investigated. Free fatty acids (FFA) are substantially elevated following HFD-feeding and are important adipocyte-derived mediators of macrophage inflammatory responses (Suganami, et al., Arterioscler Thromb Vasc Biol, 2005; 25: 2062-2068; Boden, Curr Opin Clin Nutr Metab Care, 2002; 5: 545-549). Whether palmitate, a saturated fatty acid that is abundant during obesity, could induce expression of netrin-1 and Unc5b in bone marrow derived macrophages (BMDM) was investigated. Palmitate conjugated to fatty acid-free bovine serum albumin (BSA), but not BSA alone, induced 5- and 3-fold increases in Ntn1 and Unc5b mRNA, respectively, in BMDMs (Fig. 26a). Use of peritoneal macrophages instead of BMDMs produced similar outcomes (not shown). As palmitate has been shown to induce signaling leading to NFkB activation, whether this contributes to the regulation of netrin-1 and Unc5b was investigated. Palmitate induced the activity of Ntn1- and Unc5b-promoter luciferase reporter genes in HEK293 cells, and this was inhibited, in part, by pretreating cells with the NFkB inhibitor, BAY 11-7085 (Fig. 26b-c). Furthermore, palmitate induced secretion of netrin-1 by BMDM, as measured by ELISA of cell supernatants, which was inhibited, in part, by blocking NFkB (Fig. 26d). As a number of studies have demonstrated paracrine interactions between adipocytes and macrophages in the WAT during inflammation (Suganami, et al., Arterioscler Thromb Vasc Biol, 2005; 25: 2062-2068), the role of adipocyte-secreted factors in inducing netrin-1 was investigated by treating BMDM with adipocyte-conditioned medium, derived from treating 3T3L1 adipocytes with palmitate or BSA. Conditioned medium from palmitate, but not BSA treated adipocytes potently induced Ntn1 and Unc5b mRNA in BMDM (Fig. 26e). As previously reported (Uysal, et al., Nature, 1997; 389: 610-614; Sabio, et al., Science, 2008; 322: 1539-1543), palmitate treatment of adipocytes induced expression of Tnfa and 116 mRNA (Fig. 26f), and the direct addition of TNFα or IL-6, but not IL-4, to BMDMs increased Ntn1 mRNA (Fig. 26g), implicating these adipocyte-secreted cytokines in the induction of macrophage netrin-1 expression. To test this, conditioned medium from palmitate- and BSA-stimulated adipocytes were treated with TNFα and IL-6 blocking antibodies, either alone or in combination. Antibodies to TNFα or IL-6, but not control IgG, partially blocked the induction of Ntn1 and Unc5b mRNA in BMDM by conditioned medium from palmitate-stimulated adipocytes (Fig. 26h, Suppl. Fig. 2), demonstrating a role for adipocyte-derived TNFα and IL-6 in the induction of netrin-1 and Unc5b in neighboring macrophages in obese adipose. Together, these data indicate that elevated levels of palmitate associated with obesity may induce macrophage expression of netrin-1 and Unc5b both directly and indirectly via induction of adipocyte-derived factors.

### Netrin-1 promotes defective ATM migration and accumulation in adipose.

As netrin-1 has recently been reported to inhibit macrophage migration, its secretion by ATMs may induce macrophage chemostasis, thereby blocking macrophage egress from the adipose tissue. To test this, CD11b+F4/80+ ATM were isolated from mice fed a chow or HFD for 16 weeks, and measured their migration to CCL19, a chemokine implicated in the emigration of tissue macrophages to draining lymph nodes. Notably, ATMs isolated from obese mice exhibit impaired migration to CCL19, as compared to ATMs from lean mice (Fig. 27a), despite equivalent expression of the CCL19 receptor *Ccr7* (Fig. 27b). A similar impairment of CCL19-induced migration was seen in ATMs from lean mice treated with netrin-1 (Fig. 27c). Indeed, treating ATMs from obese mice with a blocking antibody to Unc5b restored their ability to migrate to CCL19, to the levels observed in ATMs from lean mice (Fig. 27d), consistent with a role for netrin-1 in inducing ATM chemostasis in adipose tissue during obesity. Furthermore, treatment of peritoneal macrophages with palmitate, which induces netrin-1 secretion (Fig. 27e), blocked the migration of these cells to CCL19. Migration to CCL19 was restored in palmitate-treated macrophages by the addition of a chimeric Unc5b-FC peptide to block the effects of netrin-1, but not by a control FC (Fig. 27f).

Whether ATM expression of netrin-1 during obesity may promote the accumulation of these cells in adipose tissue, fostering chronic inflammation and metabolic dysfunction was investigated. Mice lacking netrin-1 in macrophages were generated by reconstituting lethally irradiated *C57BL*/*6* mice with fetal liver cells from *Ntnl^{-l-} or Ntn1*^{+/+} (WT) pups at embryonic day 14. Four weeks post-transplantation, the chimeric *Ntn1^{-l-}*→*3C57BL*/*6* and *WT→C57BL*/*6* mice were challenged with a HFD (60% Kcal) or chow diet for 20 weeks.

Analysis *of Ntnl* gene expression in both circulating leukocytes and peritoneal macrophages of recipient mice confirmed a complete change in the genotype to the donor types (data not shown). HFD diet-fed *Ntn1^{-l-}*→*3C57BL*/*6* and *WT→C57BL*/*6* mice gained significantly more weight than their chow-fed counterparts, and no differences in body weight or fat content as measured by dual-energy X-ray absorptiometry (DEXA) were observed between *WT* and *Ntnl^{-l-}* chimeric mice (Fig. 28a, b). Notably, despite similar numbers of macrophages in the adipose tissue when mice were fed a chow diet, there was a marked decrease of ATMs in *Ntnl^{-l-}* →*3C57BL*/*6* compared to *WT→C57BL*/*6* fed HFD, as assessed by reduced expression of *Emr1* (F4/80) mRNA (Fig 28c) and a decrease in the appearance of F4/80+ cells in crown-like structures (Fig 28d) in the visceral adipose. The isolation of ATMs from HFD-fed mice also revealed lower mRNA levels of pro-inflammatory cytokines and markers of MI macrophages *(Nos2, Tnfa and 116),* which are believed to promote insulin resistance, in *WT→C57BL*/*6* mice compared with *WT→C57BL*/*6* (Fig 28e). This was accompanied by an increase in markers of reparative M2 macrophages (*Cd206, Pparg and ll10*) characteristic of the ATMs of lean mice in HFD-fed *Ntn^{-l-}*→*3C57BL*/*6* mice compared with WT+C57BU6 (Fig 28e). Collectively, these data indicate that deletion of hematopoietic Ntn1 reduces ATM accumulation and restores the balance of MI and M2 macrophages in obese adipose tissue.

To test whether netrin-1 affects macrophage mobilization into and out of adipose tissue, a macrophage-tracking technique was used in which monocytes are selectively labeled *in vivo* with fluorescent beads and tracked over time (Tacke, et al., Journal of Clinical Investigation, 2007; 117: 185-194). To assess macrophage recruitment and retention in *WT→C57BL*/*6* and *Ntn1^{-l-}* →*3C57BL*/*6* mice, the number of bead-positive macrophages that accumulate in adipose tissue was compared at two time points after monocyte labeling: 3 days, which represents the time of peak recruitment of labeled cells to tissues (Tacke, et al., Journal of Clinical Investigation, 2007; 117: 185-194), and 14 days to assess the number of labeled macrophages remaining in the adipose. Using this technique, HFD-feeding was demonstrated to induce a 2.5-fold increase in the recruitment of labeled monocytes into visceral adipose tissue compared to chow-feeding (Fig 28f; day 3). As shown in Fig. 28g, the fluorescent beads carried in by these monocytes accumulate within F4/80-positive macrophages present in crown-like structures. Similar numbers of bead-labeled macrophages are detected after 3 days in the visceral adipose tissue of both chow and HFD-fed *WT→C57BL*/*6* and *Ntn1^{-l-}*→*3C57BL*/*6* mice, indicating that netrin-1 does not affect monocyte recruitment. Notably, the number of bead-labeled macrophages in visceral adipose tissue remains constant in HFD-fed *WT→C57BL*/*6* mice after 14 days, indicating that ATMs recruited to adipose of obese mice accrue in the tissue (Fig 29 a, b). By contrast, there was a 50% decrease in bead-labeled macrophages in *Ntn1^{-l-}*→*3C57BL*/*6* mice after 14 days compared to 3 days, indicating that fewer bead-labeled macrophages are retained in the adipose in the absence of netrin-1. As the reduction in bead-labeled cells in the *Ntn1^{-l-}*→*3C57BL*/*6* mice at day 14 is indicative of ATM movement out of the adipose, the draining lymph nodes were isolated from the mesentery and the number of bead-labeled cells in this tissue was analyzed. The mesenteric lymph nodes of HFD-fed *Ntn1^{-l-}*→*3C57BL*/*6* contained 40% more bead-labeled cells compared to *WT→C57BL*/*6* mice at day 14, which correlates well with the decrease in bead-labeled macrophages in the adipose tissue of *Ntn1^{-l-}*→*3C57BL*/*6* mice at this time point (Suppl. Fig. 3b). Together, these data demonstrate that netrin-1 secreted by ATMs acts locally as a macrophage retention signal to promotes ATM accumulation and sustain inflammation in the setting of obesity.

### Netrin-1 promotes insulin resistance in vivo.

The accumulation of macrophages within visceral adipose tissue has been shown to correlate with systemic inflammation and metabolic dysfunction. Consistent with reduced adipose inflammation in HFD-fed *Ntn1^{-l-}*→*3C57BL*/*6* mice, lower serum levels of TNFα compared to HFD-fed *WT→C57BL*/*6* mice were observed (Fig. 30a). Furthermore, glucose and insulin tolerance tests (GTT and ITT) revealed that the deletion of Netrin-1 in the hematopoietic fraction conferred an improvement in glucose homeostasis and protection from diet-induced insulin resistance (Fig. 30 b, c). These findings correlated with an improvement in metabolic parameters in *Ntn1^{-l-}*→*3C57BL*/*6* compared to *WT→C57BL*/*6 mice* fed HFD, including reductions in fasting blood glucose (Fig. 30 d), insulin (Fig. 30 e) and serum FFA levels (Fig. 30 f). Furthermore, plasma adiponectin levels, which correlates with insulin sensitivity, was similar in HFD-fed *Ntn1^{-l-}*→*3C57BL*/*6* and chow-fed mice, whereas it was reduced in HFD-fed *WT→C57BL*/*6* mice (Fig. 30 g). No difference in glucose metabolism, insulin sensitivity or serum levels of adiponectin and FFA was observed in chow-fed *Ntn1^{-l-}*→*3C57BL*/*6* and *WT→C57BL*/*6* mice. Finally, as the metabolic effects of insulin are dependent on phosphoinositol 3-kinase (Pl3K)-AKT signaling in target tissues, we measured the phosphorylation of AKT on Ser473 in adipose tissue, liver and muscle of HFD-fed mice. Consistent with the improved insulin sensitivity in the absence of netrin-1 expression in ATM, there was 2-4-fold more phospho-AKT in adipose tissue, liver and muscle of *Ntn1^{-l-}*→*3C57BL*/*6* mice compared to *WT→C57BL*/*6* controls (Fig. 30 h). Together, these data indicate that deletion of netrin-1 in adipose tissue macrophages leads to improved maintenance of glucose homeostasis and insulin sensitivity.

## Claims

1. A pharmaceutical composition comprising an antagonist anti-netrin-1 antibody or an antagonist anti-unc5b antibody effective to inhibit or reduce the biological activity of netrin-1 or its receptor unc5b, for use in a method for inhibiting, slowing or preventing atherosclerosis **characterized by** inflammation or for use in a method for treating a disease **characterised by** inflammation selected from the group consisting of atherosclerosis and obesity, wherein a therapeutically effective amount of the agent is to be administered.

## Patentansprüche

1. Pharmazeutische Zusammensetzung, umfassend einen antagonistischen Anti-Netrin-1-Antikörper oder einen antagonistischen Anti-unc5b-Antikörper, der die biologische Aktivität von Netrin-1 oder dessen Rezeptor unc5b inhibiert oder reduziert, zur Verwendung in einem Verfahren zum Inhibieren, Verlangsamen oder Verhindern von durch Entzündung gekennzeichneter Atherosklerose oder zur Verwendung in einem Verfahren zur Behandlung einer durch Entzündung gekennzeichneten Krankheit ausgewählt aus der Gruppe bestehend aus Atherosklerose und Obesitas, wobei eine therapeutisch wirksame Menge des Mittels verabreicht wird.

## Revendications

1. Composition pharmaceutique comprenant un anticorps antagoniste anti-nétrine-1 ou un anticorps antagoniste anti-unc5b actif dans l'inhibition ou la réduction de l'activité biologique de la nétrine-1 ou de son récepteur unc5b, pour utilisation dans une méthode d'inhibition, de ralentissement ou de prévention de l'athérosclérose **caractérisée par** une inflammation ou pour utilisation dans une méthode de traitement d'une maladie **caractérisée par** une inflammation choisie dans le groupe constitué par l'athérosclérose et l'obésité, dans laquelle une quantité thérapeutiquement active de l'agent doit être administrée.
